# EUROPÄISCHE PATENTSCHRIFT

(11) **EP 2 180 046 B1**
(45) Veröffentlichungstag und Bekanntmachung des Hinweises auf die Patenterteilung: **25.06.2014**
(21) Anmeldenummer: 09179432.1
(22) Anmeldetag: 20.12.2005
(51) Int. Cl.: C12N 9/10, C12N 15/54, A01K 67/027, C12N 5/10

(54) **Verfahren zur Herstellung von mehrfach ungesättigten langkettigen Fettsäuren in transgenen Organismen**
Method for producing polyunsaturated long-chain fatty acids in transgenic organisms
Procédé de production d'acides gras à longues chaînes multi-insaturés dans des organismes transgéniques

(30) Priorität: 23.12.2004 DE 102004062294
(43) Veröffentlichungstag der Anmeldung: 28.04.2010
(62) Teilanmeldung aus: 05821795.1
(73) Patentinhaber: BASF Plant Science GmbH, 67056 Ludwigshafen (DE)
(72) Erfinder: Cirpus, Petra, 67165 Waldsee (DE); Abbadi, Amine, 24214 Gettorf (DE); Kirsch, Jelena, 22547 Hamburg (DE); Bauer, Jörg, 67117 Limburgerhof (DE); Heinz, Ernst, 22609 Hamburg (DE)
(74) Vertreter: Sendrowski, Heiko

(56) Entgegenhaltungen:
- WO-A-00/18889
- WO-A-2004/076617
- WO-A-2004/087902
- FR-A- 2 785 911
- US-A1- 2004 034 888
- YAMASHITA ATSUSHI ET AL: "Acyltransferases and transacylases involved in fatty acid remodeling of phospholipids and metabolism of bioactive lipids in mammalian cells" JOURNAL OF BIOCHEMISTRY (TOKYO), Bd. 122, Nr. 1, 1997, Seiten 1-16, XP002387916 ISSN: 0021-924X
- DAHLQVIST A ET AL: "Phospholipid:diacylglycerol acyltransferase: an enzyme that catalyses the acyl-coA-independent formation of triacylglycerol in yeast and plants" PROCEEDINGS OF THE NATIONAL ACADEMY OF SCIENCES OF USA, NATIONAL ACADEMY OF SCIENCE, WASHINGTON, DC, US, Bd. 97, Nr. 12, 6. Juni 2000 (2000-06-06), Seiten 6487-6492, XP002235601 ISSN: 0027-8424
- YAMASHITA A ET AL: "ATP-independent fatty acyl-coenzyme A synthesis from phospholipid: coenzyme A-dependent transacylation activity toward lysophosphatidic acid catalyzed by acyl-coenzyme A:lysophosphatidic acid acyltransferase." THE JOURNAL OF BIOLOGICAL CHEMISTRY. 20 JUL 2001, Bd. 276, Nr. 29, 20. Juli 2001 (2001-07-20), Seiten 26745-26752, XP002387917 ISSN: 0021-9258
- DATABASE UniProt [Online] 6. Dezember 2005 (2005-12-06), "Lysophosphatidic acid acyltransferase, putative." XP002387919 gefunden im EBI Database accession no. Q337H3
- DOMERGUE F ET AL: "Relief for fish stocks: oceanic fatty acids in transgenic oilseeds" TRENDS IN PLANT SCIENCE, ELSEVIER SCIENCE, OXFORD, GB, Bd. 10, Nr. 3, März 2005 (2005-03), Seiten 112-116, XP004776171 ISSN: 1360-1385

### SEQUENCE LISTING

<110> BASF PI ant Sci ence GmbH
<120> Verfahren zur Verstellung von mehrfach ungesättigten langkettigen
   Fet t säur en i n
transgenen Organismen
<130> B 9440
<150> DE 10 2004 062 294.9
   <151> 2004-12-23
<160> 27
<170> Patent In version 3.3
<210> 1
   <211> 720
   <212> DNA
   <213> Ostreococcus tauri
<220>
   <221> CDS
   <222> (1)..(720)
<400> 1
   atg ctg gtc gcg cgc gtc cga gcg atc gcc ttc ttc gtc acc tcg ttc      48
   Met Leu Val Ala Arg Val Arg Ala Ile Ala Phe Phe Val Thr Ser Phe
   1               5                   10                  15
   acg ctc gcg gtg ccg ctc atc atg atc atg gcg atc ata ttc ccg ttt      96
   Thr Leu Ala Val Pro Leu Ile Met Ile Met Ala Ile Ile Phe Pro Phe
               20                  25                  30
   cag tac gcg ttc gat cga acg cga agg cgg gcg ctg agt ttc ttc aac     144
   Gln Tyr Ala Phe Asp Arg Thr Arg Arg Arg Ala Leu Ser Phe Phe Asn
           35                  40                  45
   gac gtc tgg gcg acg gtg tcc act ggg ttg ttc ttt ccg att gaa gtc     192
   Asp Val Trp Ala Thr Val Ser Thr Gly Leu Phe Phe Pro Ile Glu Val
       50                  55                  60
   gtc gga cgg gag aat ttg ccg agc gcg acg acg gcg gcg gtg tac gtc     240
   Val Gly Arg Glu Asn Leu Pro Ser Ala Thr Thr Ala Ala Val Tyr Val
   65                  70                  75                  80
   gcg aat cac gcg tcg ttc atg gat att tat tcc atg ttt cac ctg cga     288
   Ala Asn His Ala Ser Phe Met Asp Ile Tyr Ser Met Phe His Leu Arg
                   85                  90                  95
   cgc ccg ttc aag ttt gtg tcc aag acg agc aac ttt ttg atc ccc gtc     336
   Arg Pro Phe Lys Phe Val Ser Lys Thr Ser Asn Phe Leu Ile Pro Val
               100                 105                 110
   gtc ggg tgg tcg atg tat ctc acg gga cac atc cct ctg aag cgc atg     384
   Val Gly Trp Ser Met Tyr Leu Thr Gly His Ile Pro Leu Lys Arg Met
           115                 120                 125
   gag cgt cga tcg caa ttg gaa gat ttg aag acg tgc cgc gag atg ctc     432
   Glu Arg Arg Ser Gln Leu Glu Asp Leu Lys Thr Cys Arg Glu Met Leu
       130                 135                 140
   gcc gac ggt ggt tcg gtt ctt ttc ttc ccg gaa ggg acg aga agc gcc     480
   Ala Asp Gly Gly Ser Val Leu Phe Phe Pro Glu Gly Thr Arg Ser Ala
   145                 150                 155                 160
   gat ggg aag atg cag gcg ttc aag aag gga gcg ttt agc gtc gcg gct     528
   Asp Gly Lys Met Gln Ala Phe Lys Lys Gly Ala Phe Ser Val Ala Ala
                   165                 170                 175
   aag gaa aat gtc cca gtg gtg ccc gtc acc atc gtc gga gcg cac gag     576
   Lys Glu Asn Val Pro Val Val Pro Val Thr Ile Val Gly Ala His Glu
               180                 185                 190
   gcc atg gcg agc ggt aag gag tac gcg ctc aac gcg ggt ggg atc aag     624
   Ala Met Ala Ser Gly Lys Glu Tyr Ala Leu Asn Ala Gly Gly Ile Lys
           195                 200                 205
   gtg atc gtg cac cca ccg att caa tcc acc gac gcc gac gat ctc tgc     672
   Val Ile Val His Pro Pro Ile Gln Ser Thr Asp Ala Asp Asp Leu Cys
       210                 215                 220
   aag cgt tcc gag gct atc att aag gag agc ctc gtg aac aac tcg tga     720
   Lys Arg Ser Glu Ala Ile Ile Lys Glu Ser Leu Val Asn Asn Ser
   225                 230                 235
<210> 2
   <211> 239
   <212> PRT
   <213> Ostreococcus tauri
<400> 2
   Met Leu Val Ala Arg Val Arg Ala Ile Ala Phe Phe Val Thr Ser Phe
   1 5 10 15
   Thr Leu Ala Val Pro Leu Ile Met Ile Met Ala Ile Ile Phe Pro Phe
   20 25 30
   Gln Tyr Ala Phe Asp Arg Thr Arg Arg Arg Ala Leu Ser Phe Phe Asn
   35 40 45
   Asp Val Trp Ala Thr Val Ser Thr Gly Leu Phe Phe Pro Ile Glu Val
   50 55 60
   Val Gly Arg Glu Asn Leu Pro Ser Ala Thr Thr Ala Ala Val Tyr Val
   65 70 75 80
   Ala Asn His Ala Ser Phe Met Asp Ile Tyr Ser Met Phe His Leu Arg
   85 90 95
   Arg Pro Phe Lys Phe Val Ser Lys Thr Ser Asn Phe Leu Ile Pro Val
   100 105 110
   Val Gly Trp Ser Met Tyr Leu Thr Gly His Ile Pro Leu Lys Arg Met
   115 120 125
   Glu Arg Arg Ser Gln Leu Glu Asp Leu Lys Thr Cys Arg Glu Met Leu
   130 135 140
   Ala Asp Gly Gly Ser Val Leu Phe Phe Pro Glu Gly Thr Arg Ser Ala
   145 150 155 160
   Asp Gly Lys Met Gln Ala Phe Lys Lys Gly Ala Phe Ser Val Ala Ala
   165 170 175
   Lys Glu Asn Val Pro Val Val Pro Val Thr Ile Val Gly Ala His Glu
   180 185 190
   Ala Met Ala Ser Gly Lys Glu Tyr Ala Leu Asn Ala Gly Gly Ile Lys
   195 200 205
   Val Ile Val His Pro Pro Ile Gln Ser Thr Asp Ala Asp Asp Leu Cys
   210 215 220
   Lys Arg Ser Glu Ala Ile Ile Lys Glu Ser Leu Val Asn Asn Ser
   225 230 235
<210> 3
   <211> 702
   <212> DNA
   <213> Mantoni el la squarmt a
<220>
   <221> CDS
   <222> (1)..(702)
<400> 3
   atg tct ttt tac ctc gtc acc ttc acc ctc gcc tta ccc ctc ttc gcc 48
   Met Ser Phe Tyr Leu Val Thr Phe Thr Leu Ala Leu Pro Leu Phe Ala
   1 5 10 15
   gtc atg ctc att ttg ttc ccc ttc acc tac ctc acc gac aag tac cgc 96
   Val Met Leu Ile Leu Phe Pro Phe Thr Tyr Leu Thr Asp Lys Tyr Arg
   20 25 30
   cgc tac gcc ctc agc ttc gtc aac gac gtg tgg gca tgc gtc agc acg 144
   Arg Tyr Ala Leu Ser Phe Val Asn Asp Val Trp Ala Cys Val Ser Thr
   35 40 45
   tgg ttc ttc ttt cct gtc gag gtg att gga aaa gag aac atc cct ccg 192
   Trp Phe Phe Phe Pro Val Glu Val Ile Gly Lys Glu Asn Ile Pro Pro
   50 55 60
   gtc aca aca ccc gca gtg tac gtg gcc aac cac gcg agt tac ctg gac 240
   Val Thr Thr Pro Ala Val Tyr Val Ala Asn His Ala Ser Tyr Leu Asp
   65 70 75 80
   atc tac tcg ctg ttc cac ctc cgc agg ccc ttc aag ttc atc tcg aag 288
   Ile Tyr Ser Leu Phe His Leu Arg Arg Pro Phe Lys Phe Ile Ser Lys
   85 90 95
   gtg tcc aac ttc atc atc ccc atc atc ggg tgg tcc atg tac atg acg 336
   Val Ser Asn Phe Ile Ile Pro Ile Ile Gly Trp Ser Met Tyr Met Thr
   100 105 110
   ggg cac atc gcg ctc aag cgc acg gat cgc aag agc cag atg aaa acc 384
   Gly His Ile Ala Leu Lys Arg Thr Asp Arg Lys Ser Gln Met Lys Thr
   115 120 125
   ttg aag gat tgc cgc gag ttg ttg cag aag aac tgc tcg gtg ctt ttc 432
   Leu Lys Asp Cys Arg Glu Leu Leu Gln Lys Asn Cys Ser Val Leu Phe
   130 135 140
   ttc ccc gaa ggg acg cga agc gtg gac ggc acc atg gcg gag ttc aag 480
   Phe Pro Glu Gly Thr Arg Ser Val Asp Gly Thr Met Ala Glu Phe Lys
   145 150 155 160
   aag ggc gcc ttc agc gtc gcg gcc aag gag aag gcg ctg gtc gtg ccc 528
   Lys Gly Ala Phe Ser Val Ala Ala Lys Glu Lys Ala Leu Val Val Pro
   165 170 175
   atc acg ctc gtg ggc acg tcg gcg agg atg aag aac ggg aag gag tgg 576
   Ile Thr Leu Val Gly Thr Ser Ala Arg Met Lys Asn Gly Lys Glu Trp
   180 185 190
   atg ctg cgc agc ggg ggc atc aag gta gtg gtg cac ccg ccc att cag 624
   Met Leu Arg Ser Gly Gly Ile Lys Val Val Val His Pro Pro Ile Gln
   195 200 205
   agc aaa ggc gac gac gcc caa gag ctc tgc gat gag agc tac aaa acc 672
   Ser Lys Gly Asp Asp Ala Gln Glu Leu Cys Asp Glu Ser Tyr Lys Thr
   210 215 220
   atc aag gag agc ctt gtc aag tac tcg tga 702
   Ile Lys Glu Ser Leu Val Lys Tyr Ser
   225 230
<210> 4
   <211> 233
   <212> PRT
   <213> Mantoniella squamat a
<400> 4
   Met Ser Phe Tyr Leu Val Thr Phe Thr Leu Ala Leu Pro Leu Phe Ala
   1 5 10 15
   Val Met Leu Ile Leu Phe Pro Phe Thr Tyr Leu Thr Asp Lys Tyr Arg
   20 25 30
   Arg Tyr Ala Leu Ser Phe Val Asn Asp Val Trp Ala Cys Val Ser Thr
   35 40 45
   Trp Phe Phe Phe Pro Val Glu Val Ile Gly Lys Glu Asn Ile Pro Pro
   50 55 60
   Val Thr Thr Pro Ala Val Tyr Val Ala Asn His Ala Ser Tyr Leu Asp
   65 70 75 80
   Ile Tyr Ser Leu Phe His Leu Arg Arg Pro Phe Lys Phe Ile Ser Lys
   85 90 95
   Val Ser Asn Phe Ile Ile Pro Ile Ile Gly Trp Ser Met Tyr Met Thr
   100 105 110
   Gly His Ile Ala Leu Lys Arg Thr Asp Arg Lys Ser Gln Met Lys Thr
   115 120 125
   Leu Lys Asp Cys Arg Glu Leu Leu Gln Lys Asn Cys Ser Val Leu Phe
   130 135 140
   Phe Pro Glu Gly Thr Arg Ser Val Asp Gly Thr Met Ala Glu Phe Lys
   145 150 155 160
   Lys Gly Ala Phe Ser Val Ala Ala Lys Glu Lys Ala Leu Val Val Pro
   165 170 175
   Ile Thr Leu Val Gly Thr Ser Ala Arg Met Lys Asn Gly Lys Glu Trp
   180 185 190
   Met Leu Arg Ser Gly Gly Ile Lys Val Val Val His Pro Pro Ile Gln
   195 200 205
   Ser Lys Gly Asp Asp Ala Gln Glu Leu Cys Asp Glu Ser Tyr Lys Thr
   210 215 220
   Ile Lys Glu Ser Leu Val Lys Tyr Ser
   225 230
<210> 5
   <211> 819
   <212> DNA
   <213> Mantoniella squamat a
<220>
   <221> CDS
   <222> (1)..(819)
<400> 5
   atg tcg agg tcg acg gta tcg ata agc ttg att agg tgc cag agg acg 48
   Met Ser Arg Ser Thr Val Ser Ile Ser Leu Ile Arg Cys Gln Arg Thr
   1 5 10 15
   aga caa ggg gtg tcg tct cag ggc gag gag gat ggc gac aaa ttc tca 96
   Arg Gln Gly Val Ser Ser Gln Gly Glu Glu Asp Gly Asp Lys Phe Ser
   20 25 30
   ctc acc gcc gtc gtc cgc gcc atg tct ttt tac ctc gtc acc ttc acc 144
   Leu Thr Ala Val Val Arg Ala Met Ser Phe Tyr Leu Val Thr Phe Thr
   35 40 45
   ctc gcc tta ccc ctc ttc gcc gtc atg ctc att ttg ttc ccc ttc acc 192
   Leu Ala Leu Pro Leu Phe Ala Val Met Leu Ile Leu Phe Pro Phe Thr
   50 55 60
   tac ctc acc gac aag tac cgc cgc tac gcc ctc agc ttc gtc aac gac 240
   Tyr Leu Thr Asp Lys Tyr Arg Arg Tyr Ala Leu Ser Phe Val Asn Asp
   65 70 75 80
   gtg tgg gca tgc gtc agc acg tgg ttc ttc ttt cct gtc gag gtg att 288
   Val Trp Ala Cys Val Ser Thr Trp Phe Phe Phe Pro Val Glu Val Ile
   85 90 95
   gga aaa gag aac atc cct ccg gtc aca aca ccc gca gtg tac gtg gcc 336
   Gly Lys Glu Asn Ile Pro Pro Val Thr Thr Pro Ala Val Tyr Val Ala
   100 105 110
   aac cac gcg agt tac ctg gac atc tac tcg ctg ttc cac ctc cgc agg 384
   Asn His Ala Ser Tyr Leu Asp Ile Tyr Ser Leu Phe His Leu Arg Arg
   115 120 125
   ccc ttc aag ttc atc tcg aag gtg tcc aac ttc atc atc ccc atc atc 432
   Pro Phe Lys Phe Ile Ser Lys Val Ser Asn Phe Ile Ile Pro Ile Ile
           130                     135                     140
   ggg tgg tcc atg tac atg acg ggg cac atc gcg ctc aag cgc acg gat      480
   Gly Trp Ser Met Tyr Met Thr Gly His Ile Ala Leu Lys Arg Thr Asp
   145                 150                 155                 160
   cgc aag agc cag atg aaa acc ttg aag gat tgc cgc gag ttg ttg cag      528
   Arg Lys Ser Gln Met Lys Thr Leu Lys Asp Cys Arg Glu Leu Leu Gln
                   165                 170                 175
   aag aac tgc tcg gtg ctt ttc ttc ccc gag ggg acg cga agc gtg gac      576
   Lys Asn Cys Ser Val Leu Phe Phe Pro Glu Gly Thr Arg Ser Val Asp
               180                 185                 190
   ggc acc atg gcg gag ttc aag aag ggc gcc ttc agc gtc gcg gcc aag      624
   Gly Thr Met Ala Glu Phe Lys Lys Gly Ala Phe Ser Val Ala Ala Lys
           195                 200                 205
   gag aag gcg ctg gtc gtg ccc atc acg ctc gtg ggc acg tcg gcg agg      672
   Glu Lys Ala Leu Val Val Pro Ile Thr Leu Val Gly Thr Ser Ala Arg
       210                 215                 220
   atg aag aac ggg aag gag tgg atg ctg cgc agc ggg ggc atc aag gta      720
   Met Lys Asn Gly Lys Glu Trp Met Leu Arg Ser Gly Gly Ile Lys Val
   225                 230                 235                 240
   gtg gtg cac ccg ccc att cag agc aaa ggc gac gac gcc caa gag ctc      768
   Val Val His Pro Pro Ile Gln Ser Lys Gly Asp Asp Ala Gln Glu Leu
                   245                 250                 255
   tgc gat gag agc tac aaa acc atc aag gag agc ctt gtc aag tac tcg      816
   Cys Asp Glu Ser Tyr Lys Thr Ile Lys Glu Ser Leu Val Lys Tyr Ser
               260                 265                 270
   tga                                                                  819
<210> 6
   <211> 272
   <212> PRT
   <213> Mantoniella squamat a
<400> 6
   Met Ser Arg Ser Thr Val Ser Ile Ser Leu Ile Arg Cys Gln Arg Thr
   1               5                   10                  15
   Arg Gln Gly Val Ser Ser Gln Gly Glu Glu Asp Gly Asp Lys Phe Ser
               20                  25                  30
   Leu Thr Ala Val Val Arg Ala Met Ser Phe Tyr Leu Val Thr Phe Thr
           35                  40                  45
   Leu Ala Leu Pro Leu Phe Ala Val Met Leu Ile Leu Phe Pro Phe Thr
       50                  55                  60
   Tyr Leu Thr Asp Lys Tyr Arg Arg Tyr Ala Leu Ser Phe Val Asn Asp
   65                  70                  75                  80
   Val Trp Ala Cys Val Ser Thr Trp Phe Phe Phe Pro Val Glu Val Ile
                   85                  90                  95
   Gly Lys Glu Asn Ile Pro Pro Val Thr Thr Pro Ala Val Tyr Val Ala
   100 105 110
   Asn His Ala Ser Tyr Leu Asp Ile Tyr Ser Leu Phe His Leu Arg Arg
   115 120 125
   Pro Phe Lys Phe Ile Ser Lys Val Ser Asn Phe Ile Ile Pro Ile Ile
   130 135 140
   Gly Trp Ser Met Tyr Met Thr Gly His Ile Ala Leu Lys Arg Thr Asp
   145 150 155 160
   Arg Lys Ser Gln Met Lys Thr Leu Lys Asp Cys Arg Glu Leu Leu Gln
   165 170 175
   Lys Asn Cys Ser Val Leu Phe Phe Pro Glu Gly Thr Arg Ser Val Asp
   180 185 190
   Gly Thr Met Ala Glu Phe Lys Lys Gly Ala Phe Ser Val Ala Ala Lys
   195 200 205
   Glu Lys Ala Leu Val Val Pro Ile Thr Leu Val Gly Thr Ser Ala Arg
   210 215 220
   Met Lys Asn Gly Lys Glu Trp Met Leu Arg Ser Gly Gly Ile Lys Val
   225 230 235 240
   Val Val His Pro Pro Ile Gln Ser Lys Gly Asp Asp Ala Gln Glu Leu
   245 250 255
   Cys Asp Glu Ser Tyr Lys Thr Ile Lys Glu Ser Leu Val Lys Tyr Ser
   260 265 270
<210> 7
   <211> 6613
   <212> DNA
   <213> yeast expr essi on vector
<220>
   <221> m sc_feat ure
   <223> pYES2-Ot_ LPCAT
<400> 7
   acggattaga agccgccgag cgggtgacag ccctccgaag gaagactctc ctccgtgcgt 60
   cctcgtcttc accggtcgcg ttcctgaaac gcagatgtgc ctcgcgccgc actgctccga 120
   acaataaaga ttctacaata ctagctttta tggttatgaa gaggaaaaat tggcagtaac 180
   ctggccccac aaaccttcaa atgaacgaat caaattaaca accataggat gataatgcga 240
   ttagtttttt agccttattt ctggggtaat taatcagcga agcgatgatt tttgatctat 300
   taacagatat ataaatgcaa aaactgcata accactttaa ctaatacttt caacattttc 360
   ggtttgtatt acttcttatt caaatgtaat aaaagtatca acaaaaaatt gttaatatac 420
   ctctatactt taacgtcaag gagaaaaaac cccggatcgg actactagca gctgtaatac 480
   gactcactat agggaatatt aagctcgccc ttatgctggt cgcgcgcgtc cgagcgatcg 540
   ccttcttcgt cacctcgttc acgctcgcgg tgccgctcat catgatcatg gcgatcatat 600
   tcccgtttca gtacgcgttc gatcgaacgc gaaggcgggc gctgagtttc ttcaacgacg 660
   tctgggcgac ggtgtccact gggttgttct ttccgattga agtcgtcgga cgggagaatt 720
   tgccgagcgc gacgacggcg gcggtgtacg tcgcgaatca cgcgtcgttc atggatattt 780
   attccatgtt tcacctgcga cgcccgttca agtttgtgtc caagacgagc aactttttga 840
   tccccgtcgt cgggtggtcg atgtatctca cgggacacat ccctctgaag cgcatggagc 900
   gtcgatcgca attggaagat ttgaagacgt gccgcgagat gctcgccgac ggtggttcgg 960
   ttcttttctt cccggaaggg acgagaagcg ccgatgggaa gatgcaggcg ttcaagaagg 1020
   gagcgtttag cgtcgcggct aaggaaaatg tcccagtggt gcccgtcacc atcgtcggag 1080
   cgcacgaggc catggcgagc ggtaaggagt acgcgctcaa cgcgggtggg atcaaggtga 1140
   tcgtgcaccc accgattcaa tccaccgacg ccgacgatct ctgcaagcgt tccgaggcta 1200
   tcattaagga gagcctcgtg aacaactcgt gactcgagta agggcgagct tcgaggtcac 1260
   ccattcgaag gtaagcctat ccctaaccct ctcctcggtc tcgattctac gcgtaccggt 1320
   catcatcacc atcaccattg agtttctaga gggccgcatc atgtaattag ttatgtcacg 1380
   cttacattca cgccctcccc ccacatccgc tctaaccgaa aaggaaggag ttagacaacc 1440
   tgaagtctag gtccctattt atttttttat agttatgtta gtattaagaa cgttatttat 1500
   atttcaaatt tttctttttt ttctgtacag acgcgtgtac gcatgtaaca ttatactgaa 1560
   aaccttgctt gagaaggttt tgggacgctc gaaggcttta atttgcaagc tgcggccctg 1620
   cattaatgaa tcggccaacg cgcggggaga ggcggtttgc gtattgggcg ctcttccgct 1680
   tcctcgctca ctgactcgct gcgctcggtc gttcggctgc ggcgagcggt atcagctcac 1740
   tcaaaggcgg taatacggtt atccacagaa tcaggggata acgcaggaaa gaacatgtga 1800
   gcaaaaggcc agcaaaagcc caggaaccgt aaaaaggccg cgttgctggc gtttttccat 1860
   aggctccgcc cccctgacga gcatcacaaa aatcgacgct caagtcagag gtggcgaaac 1920
   ccgacaggac tataaagata ccaggcgttt ccccctggaa gctccctcgt gcgctctcct 1980
   gttccgaccc tgccgcttac cggatacctg tccgcctttc tcccttcggg aagcgtggcg 2040
   ctttctcata gctcacgctg taggtatctc agttcggtgt aggtcgttcg ctccaagctg 2100
   ggctgtgtgc acgaaccccc cgttcagccc gaccgctgcg ccttatccgg taactatcgt 2160
   cttgagtcca acccggtaag acacgactta tcgccactgg cagcagccac tggtaacagg 2220
   attagcagag cgaggtatgt aggcggtgct acagagttct tgaagtggtg gcctaactac 2280
   ggctacacta gaaggacagt atttggtatc tgcgctctgc tgaagccagt taccttcgga 2340
   aaaagagttg gtagctcttg atccggcaaa caaaccaccg ctggtagcgg tggttttttt 2400
   gtttgcaagc agcagattac gcgcagaaaa aaaggatctc aagaagatcc tttgatcttt  2460
   tctacggggt ctgacgctca gtggaacgaa aactcacgtt aagggatttt ggtcatgaga 2520
   ttatcaaaaa ggatcttcac ctagatcctt ttaaattaaa aatgaagttt taaatcaatc 2580
   taaagtatat atgagtaaac ttggtctgac agttaccaat gcttaatcag tgaggcacct 2640
   atctcagcga tctgtctatt tcgttcatcc atagttgcct gactccccgt cgtgtagata 2700
   actacgatac gggagcgctt accatctggc cccagtgctg caatgatacc gcgagaccca 2760
   cgctcaccgg ctccagattt atcagcaata aaccagccag ccggaagggc cgagcgcaga 2820
   agtggtcctg caactttatc cgcctccatt cagtctatta attgttgccg ggaagctaga 2880
   gtaagtagtt cgccagttaa tagtttgcgc aacgttgttg gcattgctac aggcatcgtg 2940
   gtgtcactct cgtcgtttgg tatggcttca ttcagctccg gttcccaacg atcaaggcga 3000
   gttacatgat cccccatgtt gtgcaaaaaa gcggttagct ccttcggtcc tccgatcgtt 3060
   gtcagaagta agttggccgc agtgttatca ctcatggtta tggcagcact gcataattct 3120
   cttactgtca tgccatccgt aagatgcttt tctgtgactg gtgagtactc aaccaagtca 3180
   ttctgagaat agtgtatgcg gcgaccgagt tgctcttgcc cggcgtcaat acgggataat 3240
   agtgtatcac atagcagaac tttaaaagtg ctcatcattg gaaaacgttc ttcggggcga 3300
   aaactctcaa ggatcttacc gctgttgaga tccagttcga tgtaacccac tcgtgcaccc 3360
   aactgatctt cagcatcttt tactttcacc agcgtttctg ggtgagcaaa aacaggaagg 3420
   caaaatgccg caaaaaaggg aataagggcg acacggaaat gttgaatact catactcttc 3480
   ctttttcaat gggtaataac tgatataatt aaattgaagc tctaatttgt gagtttagta 3540
   tacatgcatt tacttataat acagtttttt agttttgctg gccgcatctt ctcaaatatg 3600
   cttcccagcc tgcttttctg taacgttcac cctctacctt agcatccctt ccctttgcaa 3660
   atagtcctct tccaacaata ataatgtcag atcctgtaga gaccacatca tccacggttc 3720
   tatactgttg acccaatgcg tctcccttgt catctaaacc cacaccgggt gtcataatca 3780
   accaatcgta accttcatct cttccaccca tgtctctttg agcaataaag ccgataacaa 3840
   aatctttgtc gctcttcgca atgtcaacag tacccttagt atattctcca gtagataggg 3900
   agcccttgca tgacaattct gctaacatca aaaggcctct aggttccttt gttacttctt 3960
   ctgccgcctg cttcaaaccg ctaacaatac ctgggcccac cacaccgtgt gcattcgtaa 4020
   tgtctgccca ttctgctatt ctgtatacac ccgcagagta ctgcaatttg actgtattac 4080
   caatgtcagc aaattttctg tcttcgaaga gtaaaaaatt gtacttggcg gataatgcct 4140
   ttagcggctt aactgtgccc tccatggaaa aatcagtcaa gatatccaca tgtgttttta 4200
   gtaaacaaat tttgggacct aatgcttcaa ctaactccag taattccttg gtggtacgaa 4260
   catccaatga agcacacaag tttgtttgct tttcgtgcat gatattaaat agcttggcag 4320
   caacaggact aggatgagta gcagcacgtt ccttatatgt agctttcgac atgatttatc 4380
   ttcgtttcct gcaggttttt gttctgtgca gttgggttaa gaatactggg caatttcatg 4440
   tttcttcaac actacatatg cgtatatata ccaatctaag tctgtgctcc ttccttcgtt 4500
   cttccttctg ttcggagatt accgaatcaa aaaaatttca aagaaaccga aatcaaaaaa 4560
   aagaataaaa aaaaaatgat gaattgaatt gaaaagctag cttatcgatg ataagctgtc 4620
   aaagatgaga attaattcca cggactatag actatactag atactccgtc tactgtacga 4680
   tacacttccg ctcaggtcct tgtcctttaa cgaggcctta ccactctttt gttactctat 4740
   tgatccagct cagcaaaggc agtgtgatct aagattctat cttcgcgatg tagtaaaact 4800
   agctagaccg agaaagagac tagaaatgca aaaggcactt ctacaatggc tgccatcatt 4860
   attatccgat gtgacgctgc agcttctcaa tgatattcga atacgctttg aggagataca 4920
   gcctaatatc cgacaaactg ttttacagat ttacgatcgt acttgttacc catcattgaa 4980
   ttttgaacat ccgaacctgg gagttttccc tgaaacagat agtatatttg aacctgtata 5040
   ataatatata gtctagcgct ttacggaaga caatgtatgt atttcggttc ctggagaaac 5100
   tattgcatct attgcatagg taatcttgca cgtcgcatcc ccggttcatt ttctgcgttt 5160
   ccatcttgca cttcaatagc atatctttgt taacgaagca tctgtgcttc attttgtaga 5220
   acaaaaatgc aacgcgagag cgctaatttt tcaaacaaag aatctgagct gcatttttac 5280
   agaacagaaa tgcaacgcga aagcgctatt ttaccaacga agaatctgtg cttcattttt 5340
   gtaaaacaaa aatgcaacgc gacgagagcg ctaatttttc aaacaaagaa tctgagctgc 5400
   atttttacag aacagaaatg caacgcgaga gcgctatttt accaacaaag aatctatact 5460
   tcttttttgt tctacaaaaa tgcatcccga gagcgctatt tttctaacaa agcatcttag 5520
   attacttttt ttctcctttg tgcgctctat aatgcagtct cttgataact ttttgcactg 5580
   taggtccgtt aaggttagaa gaaggctact ttggtgtcta ttttctcttc cataaaaaaa 5640
   gcctgactcc acttcccgcg tttactgatt actagcgaag ctgcgggtgc attttttcaa 5700
   gataaaggca tccccgatta tattctatac cgatgtggat tgcgcatact ttgtgaacag 5760
   aaagtgatag cgttgatgat tcttcattgg tcagaaaatt atgaacggtt tcttctattt 5820
   tgtctctata tactacgtat aggaaatgtt tacattttcg tattgttttc gattcactct 5880
   atgaatagtt cttactacaa tttttttgtc taaagagtaa tactagagat aaacataaaa 5940
   aatgtagagg tcgagtttag atgcaagttc aaggagcgaa aggtggatgg gtaggttata 6000
   tagggatata gcacagagat atatagcaaa gagatacttt tgagcaatgt ttgtggaagc 6060
   ggtattcgca atgggaagct ccaccccggt tgataatcag aaaagcccca aaaacaggaa 6120
   gattgtataa gcaaatattt aaattgtaaa cgttaatatt ttgttaaaat tcgcgttaaa 6180
   tttttgttaa atcagctcat tttttaacga atagcccgaa atcggcaaaa tcccttataa 6240
   atcaaaagaa tagaccgaga tagggttgag tgttgttcca gtttccaaca agagtccact 6300
   attaaagaac gtggactcca acgtcaaagg gcgaaaaagg gtctatcagg gcgatggccc 6360
   actacgtgaa ccatcaccct aatcaagttt tttggggtcg aggtgccgta aagcagtaaa 6420
   tcggaagggt aaacggatgc ccccatttag agcttgacgg ggaaagccgg cgaacgtggc 6480
   gagaaaggaa gggaagaaag cgaaaggagc gggggctagg gcggtgggaa gtgtaggggt 6540
   cacgctgggc gtaaccacca cacccgccgc gcttaatggg gcgctacagg gcgcgtgggg 6600
   atgatccact agt 6613
<210> 8
   <211> 6602
   <212> DNA-
   <213> yeast expression vector
<220>
   <221 > misc_feature
   <223> pYES2- MA_112- LPCAT
<400> 8
   acggattaga agccgccgag cgggtgacag ccctccgaag gaagactctc ctccgtgcgt     60
   cctcgtcttc accggtcgcg ttcctgaaac gcagatgtgc ctcgcgccgc actgctccga    120
   acaataaaga ttctacaata ctagctttta tggttatgaa gaggaaaaat tggcagtaac    180
   ctggccccac aaaccttcaa atgaacgaat caaattaaca accataggat gataatgcga    240
   ttagtttttt agccttattt ctggggtaat taatcagcga agcgatgatt tttgatctat    300
   taacagatat ataaatgcaa aaactgcata accactttaa ctaatacttt caacattttc    360
   ggtttgtatt acttcttatt caaatgtaat aaaagtatca acaaaaaatt gttaatatac    420
   ctctatactt taacgtcaag gagaaaaaac cccggatcgg actactagca gctgtaatac    480
   gactcactat agggaatatt aagctcgccc ttaggatcca tgtcttttta cctcgtcacc    540
   ttcaccctcg ccttacccct cttcgccgtc atgctcattt tgttcccctt cacctacctc    600
   accgacaagt accgccgcta cgccctcagc ttcgtcaacg acgtgtgggc atgcgtcagc    660
   acgtggttct tctttcctgt cgaggtgatt ggaaaagaga acatccctcc ggtcacaaca    720
   cccgcagtgt acgtggccaa ccacgcgagt tacctggaca tctactcgct gttccacctc    780
   cgcaggccct tcaagttcat ctcgaaggtg tccaacttca tcatccccat catcgggtgg    840
   tccatgtaca tgacggggca catcgcgctc aagcgcacgg atcgcaagag ccagatgaaa    900
   accttgaagg attgccgcga gttgttgcag aagaactgct cggtgctttt cttccccgaa    960
   gggacgcgaa gcgtggacgg caccatggcg gagttcaaga agggcgcctt cagcgtcgcg   1020
   gccaaggaga aggcgctggt cgtgcccatc acgctcgtgg gcacgtcggc gaggatgaag   1080
   aacgggaagg agtggatgct gcgcagcggg ggcatcaagg tagtggtgca cccgcccatt   1140
   cagagcaaag gcgacgacgc ccaagagctc tgcgatgaga gctacaaaac catcaaggag   1200
   agccttgtca agtactcgtg actcgagtaa gggcgagctt cgaggtcacc cattcgaagg   1260
   taagcctatc cctaaccctc tcctcggtct cgattctacg cgtaccggtc atcatcacca   1320
   tcaccattga gtttctagag ggccgcatca tgtaattagt tatgtcacgc ttacattcac   1380
   gccctccccc cacatccgct ctaaccgaaa aggaaggagt tagacaacct gaagtctagg   1440
   tccctattta tttttttata gttatgttag tattaagaac gttatttata tttcaaattt   1500
   ttcttttttt tctgtacaga cgcgtgtacg catgtaacat tatactgaaa accttgcttg   1560
   agaaggtttt gggacgctcg aaggctttaa tttgcaagct gcggccctgc attaatgaat 1620
   cggccaacgc gcggggagag gcggtttgcg tattgggcgc tcttccgctt cctcgctcac 1680
   tgactcgctg cgctcggtcg ttcggctgcg gcgagcggta tcagctcact caaaggcggt 1740
   aatacggtta tccacagaat caggggataa cgcaggaaag aacatgtgag caaaaggcca 1800
   gcaaaagccc aggaaccgta aaaaggccgc gttgctggcg tttttccata ggctccgccc 1860
   ccctgacgag catcacaaaa atcgacgctc aagtcagagg tggcgaaacc cgacaggact 1920
   ataaagatac caggcgtttc cccctggaag ctccctcgtg cgctctcctg ttccgaccct 1980
   gccgcttacc ggatacctgt ccgcctttct cccttcggga agcgtggcgc tttctcatag 2040
   ctcacgctgt aggtatctca gttcggtgta ggtcgttcgc tccaagctgg gctgtgtgca 2100
   cgaacccccc gttcagcccg accgctgcgc cttatccggt aactatcgtc ttgagtccaa 2160
   cccggtaaga cacgacttat cgccactggc agcagccact ggtaacagga ttagcagagc 2220
   gaggtatgta ggcggtgcta cagagttctt gaagtggtgg cctaactacg gctacactag 2280
   aaggacagta tttggtatct gcgctctgct gaagccagtt accttcggaa aaagagttgg 2340
   tagctcttga tccggcaaac aaaccaccgc tggtagcggt ggtttttttg tttgcaagca 2400
   gcagattacg cgcagaaaaa aaggatctca agaagatcct ttgatctttt ctacggggtc 2460
   tgacgctcag tggaacgaaa actcacgtta agggattttg gtcatgagat tatcaaaaag 2520
   gatcttcacc tagatccttt taaattaaaa atgaagtttt aaatcaatct aaagtatata 2580
   tgagtaaact tggtctgaca gttaccaatg cttaatcagt gaggcaccta tctcagcgat 2640
   ctgtctattt cgttcatcca tagttgcctg actccccgtc gtgtagataa ctacgatacg 2700
   ggagcgctta ccatctggcc ccagtgctgc aatgataccg cgagacccac gctcaccggc 2760
   tccagattta tcagcaataa accagccagc cggaagggcc gagcgcagaa gtggtcctgc 2820
   aactttatcc gcctccattc agtctattaa ttgttgccgg gaagctagag taagtagttc 2880
   gccagttaat agtttgcgca acgttgttgg cattgctaca ggcatcgtgg tgtcactctc 2940
   gtcgtttggt atggcttcat tcagctccgg ttcccaacga tcaaggcgag ttacatgatc 3000
   ccccatgttg tgcaaaaaag cggttagctc cttcggtcct ccgatcgttg tcagaagtaa 3060
   gttggccgca gtgttatcac tcatggttat ggcagcactg cataattctc ttactgtcat 3120
   gccatccgta agatgctttt ctgtgactgg tgagtactca accaagtcat tctgagaata 3180
   gtgtatgcgg cgaccgagtt gctcttgccc ggcgtcaata cgggataata gtgtatcaca 3240
   tagcagaact ttaaaagtgc tcatcattgg aaaacgttct tcggggcgaa aactctcaag 3300
   gatcttaccg ctgttgagat ccagttcgat gtaacccact cgtgcaccca actgatcttc 3360
   agcatctttt actttcacca gcgtttctgg gtgagcaaaa acaggaaggc aaaatgccgc 3420
   aaaaaaggga ataagggcga cacggaaatg ttgaatactc atactcttcc tttttcaatg 3480
   ggtaataact gatataatta aattgaagct ctaatttgtg agtttagtat acatgcattt 3540
   acttataata cagtttttta gttttgctgg ccgcatcttc tcaaatatgc ttcccagcct 3600
   gcttttctgt aacgttcacc ctctacctta gcatcccttc cctttgcaaa tagtcctctt 3660
   ccaacaataa taatgtcaga tcctgtagag accacatcat ccacggttct atactgttga 3720
   cccaatgcgt ctcccttgtc atctaaaccc acaccgggtg tcataatcaa ccaatcgtaa 3780
   ccttcatctc ttccacccat gtctctttga gcaataaagc cgataacaaa atctttgtcg 3840
   ctcttcgcaa tgtcaacagt acccttagta tattctccag tagataggga gcccttgcat 3900
   gacaattctg ctaacatcaa aaggcctcta ggttcctttg ttacttcttc tgccgcctgc 3960
   ttcaaaccgc taacaatacc tgggcccacc acaccgtgtg cattcgtaat gtctgcccat 4020
   tctgctattc tgtatacacc cgcagagtac tgcaatttga ctgtattacc aatgtcagca 4080
   aattttctgt cttcgaagag taaaaaattg tacttggcgg ataatgcctt tagcggctta 4140
   actgtgccct ccatggaaaa atcagtcaag atatccacat gtgtttttag taaacaaatt 4200
   ttgggaccta atgcttcaac taactccagt aattccttgg tggtacgaac atccaatgaa 4260
   gcacacaagt ttgtttgctt ttcgtgcatg atattaaata gcttggcagc aacaggacta 4320
   ggatgagtag cagcacgttc cttatatgta gctttcgaca tgatttatct tcgtttcctg 4380
   caggtttttg ttctgtgcag ttgggttaag aatactgggc aatttcatgt ttcttcaaca 4440
   ctacatatgc gtatatatac caatctaagt ctgtgctcct tccttcgttc ttccttctgt 4500
   tcggagatta ccgaatcaaa aaaatttcaa agaaaccgaa atcaaaaaaa agaataaaaa 4560
   aaaaatgatg aattgaattg aaaagctagc ttatcgatga taagctgtca aagatgagaa 4620
   ttaattccac ggactataga ctatactaga tactccgtct actgtacgat acacttccgc 4680
   tcaggtcctt gtcctttaac gaggccttac cactcttttg ttactctatt gatccagctc 4740
   agcaaaggca gtgtgatcta agattctatc ttcgcgatgt agtaaaacta gctagaccga 4800
   gaaagagact agaaatgcaa aaggcacttc tacaatggct gccatcatta ttatccgatg 4860
   tgacgctgca gcttctcaat gatattcgaa tacgctttga ggagatacag cctaatatcc 4920
   gacaaactgt tttacagatt tacgatcgta cttgttaccc atcattgaat tttgaacatc 4980
   cgaacctggg agttttccct gaaacagata gtatatttga acctgtataa taatatatag 5040
   tctagcgctt tacggaagac aatgtatgta tttcggttcc tggagaaact attgcatcta 5100
   ttgcataggt aatcttgcac gtcgcatccc cggttcattt tctgcgtttc catcttgcac 5160
   ttcaatagca tatctttgtt aacgaagcat ctgtgcttca ttttgtagaa caaaaatgca 5220
   acgcgagagc gctaattttt caaacaaaga atctgagctg catttttaca gaacagaaat 5280
   gcaacgcgaa agcgctattt taccaacgaa gaatctgtgc ttcatttttg taaaacaaaa 5340
   atgcaacgcg acgagagcgc taatttttca aacaaagaat ctgagctgca tttttacaga 5400
   acagaaatgc aacgcgagag cgctatttta ccaacaaaga atctatactt cttttttgtt 5460
   ctacaaaaat gcatcccgag agcgctattt ttctaacaaa gcatcttaga ttactttttt 5520
   tctcctttgt gcgctctata atgcagtctc ttgataactt tttgcactgt aggtccgtta 5580
   aggttagaag aaggctactt tggtgtctat tttctcttcc ataaaaaaag cctgactcca 5640
   cttcccgcgt ttactgatta ctagcgaagc tgcgggtgca ttttttcaag ataaaggcat 5700
   ccccgattat attctatacc gatgtggatt gcgcatactt tgtgaacaga aagtgatagc 5760
   gttgatgatt cttcattggt cagaaaatta tgaacggttt cttctatttt gtctctatat 5820
   actacgtata ggaaatgttt acattttcgt attgttttcg attcactcta tgaatagttc 5880
   ttactacaat ttttttgtct aaagagtaat actagagata aacataaaaa atgtagaggt 5940
   cgagtttaga tgcaagttca aggagcgaaa ggtggatggg taggttatat agggatatag 6000
   cacagagata tatagcaaag agatactttt gagcaatgtt tgtggaagcg gtattcgcaa 6060
   tgggaagctc caccccggtt gataatcaga aaagccccaa aaacaggaag attgtataag 6120
   caaatattta aattgtaaac gttaatattt tgttaaaatt cgcgttaaat ttttgttaaa 6180
   tcagctcatt ttttaacgaa tagcccgaaa tcggcaaaat cccttataaa tcaaaagaat 6240
   agaccgagat agggttgagt gttgttccag tttccaacaa gagtccacta ttaaagaacg 6300
   tggactccaa cgtcaaaggg cgaaaaaggg tctatcaggg cgatggccca ctacgtgaac 6360
   catcacccta atcaagtttt ttggggtcga ggtgccgtaa agcagtaaat cggaagggta 6420
   aacggatgcc cccatttaga gcttgacggg gaaagccggc gaacgtggcg agaaaggaag 6480
   ggaagaaagc gaaaggagcg ggggctaggg cggtgggaag tgtaggggtc acgctgggcg 6540
   taaccaccac acccgccgcg cttaatgggg cgctacaggg cgcgtgggga tgatccacta 6600
   gt 6602
<210> 9
   <211> 6712
   <212> DNA
   <213> yeast expr essi on vector
<220>
   <221> misc_f eat ure
   <223> pYES2-MA_118-LPCAT
<400> 9
   acggattaga agccgccgag cgggtgacag ccctccgaag gaagactctc ctccgtgcgt 60
   cctcgtcttc accggtcgcg ttcctgaaac gcagatgtgc ctcgcgccgc actgctccga 120
   acaataaaga ttctacaata ctagctttta tggttatgaa gaggaaaaat tggcagtaac 180
   ctggccccac aaaccttcaa atgaacgaat caaattaaca accataggat gataatgcga 240
   ttagtttttt agccttattt ctggggtaat taatcagcga agcgatgatt tttgatctat 300
   taacagatat ataaatgcaa aaactgcata accactttaa ctaatacttt caacattttc 360
   ggtttgtatt acttcttatt caaatgtaat aaaagtatca acaaaaaatt gttaatatac 420
   ctctatactt taacgtcaag gagaaaaaac cccggatcgg actactagca gctgtaatac 480
   gactcactat agggaatatt aagctcgccc ttatgtcgag gtcgacggta tcgataagct 540
   tgattaggtg ccagaggacg agacaagggg tgtcgtctca gggcgaggag gatggcgaca 600
   aattctcact caccgccgtc gtccgcgcca tgtcttttta cctcgtcacc ttcaccctcg 660
   ccttacccct cttcgccgtc atgctcattt tgttcccctt cacctacctc accgacaagt    720
   accgccgcta cgccctcagc ttcgtcaacg acgtgtgggc atgcgtcagc acgtggttct    780
   tctttcctgt cgaggtgatt ggaaaagaga acatccctcc ggtcacaaca cccgcagtgt    840
   acgtggccaa ccacgcgagt tacctggaca tctactcgct gttccacctc cgcaggccct    900
   tcaagttcat ctcgaaggtg tccaacttca tcatccccat catcgggtgg tccatgtaca    960
   tgacggggca catcgcgctc aagcgcacgg atcgcaagag ccagatgaaa accttgaagg   1020
   attgccgcga gttgttgcag aagaactgct cggtgctttt cttccccgag gggacgcgaa   1080
   gcgtggacgg caccatggcg gagttcaaga agggcgcctt cagcgtcgcg gccaaggaga   1140
   aggcgctggt cgtgcccatc acgctcgtgg gcacgtcggc gaggatgaag aacgggaagg   1200
   agtggatgct gcgcagcggg ggcatcaagg tagtggtgca cccgcccatt cagagcaaag   1260
   gcgacgacgc ccaagagctc tgcgatgaga gctacaaaac catcaaggag agccttgtca   1320
   agtactcgtg aatcgagtaa gggcgagctt cgaggtcacc cattcgaagg taagcctatc   1380
   cctaaccctc tcctcggtct cgattctacg cgtaccggtc atcatcacca tcaccattga   1440
   gtttctagag ggccgcatca tgtaattagt tatgtcacgc ttacattcac gccctccccc   1500
   cacatccgct ctaaccgaaa aggaaggagt tagacaacct gaagtctagg tccctattta   1560
   tttttttata gttatgttag tattaagaac gttatttata tttcaaattt ttcttttttt   1620
   tctgtacaga cgcgtgtacg catgtaacat tatactgaaa accttgcttg agaaggtttt   1680
   gggacgctcg aaggctttaa tttgcaagct gcggccctgc attaatgaat cggccaacgc   1740
   gcggggagag gcggtttgcg tattgggcgc tcttccgctt cctcgctcac tgactcgctg   1800
   cgctcggtcg ttcggctgcg gcgagcggta tcagctcact caaaggcggt aatacggtta   1860
   tccacagaat caggggataa cgcaggaaag aacatgtgag caaaaggcca gcaaaagccc   1920
   aggaaccgta aaaaggccgc gttgctggcg tttttccata ggctccgccc ccctgacgag   1980
   catcacaaaa atcgacgctc aagtcagagg tggcgaaacc cgacaggact ataaagatac   2040
   caggcgtttc cccctggaag ctccctcgtg cgctctcctg ttccgaccct gccgcttacc   2100
   ggatacctgt ccgcctttct cccttcggga agcgtggcgc tttctcatag ctcacgctgt   2160
   aggtatctca gttcggtgta ggtcgttcgc tccaagctgg gctgtgtgca cgaacccccc   2220
   gttcagcccg accgctgcgc cttatccggt aactatcgtc ttgagtccaa cccggtaaga   2280
   cacgacttat cgccactggc agcagccact ggtaacagga ttagcagagc gaggtatgta   2340
   ggcggtgcta cagagttctt gaagtggtgg cctaactacg gctacactag aaggacagta   2400
   tttggtatct gcgctctgct gaagccagtt accttcggaa aaagagttgg tagctcttga   2460
   tccggcaaac aaaccaccgc tggtagcggt ggtttttttg tttgcaagca gcagattacg   2520
   cgcagaaaaa aaggatctca agaagatcct ttgatctttt ctacggggtc tgacgctcag   2580
   tggaacgaaa actcacgtta agggattttg gtcatgagat tatcaaaaag gatcttcacc   2640
   tagatccttt taaattaaaa atgaagtttt aaatcaatct aaagtatata tgagtaaact   2700
   tggtctgaca gttaccaatg cttaatcagt gaggcaccta tctcagcgat ctgtctattt 2760
   cgttcatcca tagttgcctg actccccgtc gtgtagataa ctacgatacg ggagcgctta 2820
   ccatctggcc ccagtgctgc aatgataccg cgagacccac gctcaccggc tccagattta 2880
   tcagcaataa accagccagc cggaagggcc gagcgcagaa gtggtcctgc aactttatcc 2940
   gcctccattc agtctattaa ttgttgccgg gaagctagag taagtagttc gccagttaat 3000
   agtttgcgca acgttgttgg cattgctaca ggcatcgtgg tgtcactctc gtcgtttggt 3060
   atggcttcat tcagctccgg ttcccaacga tcaaggcgag ttacatgatc ccccatgttg 3120
   tgcaaaaaag cggttagctc cttcggtcct ccgatcgttg tcagaagtaa gttggccgca 3180
   gtgttatcac tcatggttat ggcagcactg cataattctc ttactgtcat gccatccgta 3240
   agatgctttt ctgtgactgg tgagtactca accaagtcat tctgagaata gtgtatgcgg 3300
   cgaccgagtt gctcttgccc ggcgtcaata cgggataata gtgtatcaca tagcagaact 3360
   ttaaaagtgc tcatcattgg aaaacgttct tcggggcgaa aactctcaag gatcttaccg 3420
   ctgttgagat ccagttcgat gtaacccact cgtgcaccca actgatcttc agcatctttt 3480
   actttcacca gcgtttctgg gtgagcaaaa acaggaaggc aaaatgccgc aaaaaaggga 3540
   ataagggcga cacggaaatg ttgaatactc atactcttcc tttttcaatg ggtaataact 3600
   gatataatta aattgaagct ctaatttgtg agtttagtat acatgcattt acttataata 3660
   cagtttttta gttttgctgg ccgcatcttc tcaaatatgc ttcccagcct gcttttctgt 3720
   aacgttcacc ctctacctta gcatcccttc cctttgcaaa tagtcctctt ccaacaataa 3780
   taatgtcaga tcctgtagag accacatcat ccacggttct atactgttga cccaatgcgt 3840
   ctcccttgtc atctaaaccc acaccgggtg tcataatcaa ccaatcgtaa ccttcatctc 3900
   ttccacccat gtctctttga gcaataaagc cgataacaaa atctttgtcg ctcttcgcaa 3960
   tgtcaacagt acccttagta tattctccag tagataggga gcccttgcat gacaattctg 4020
   ctaacatcaa aaggcctcta ggttcctttg ttacttcttc tgccgcctgc ttcaaaccgc 4080
   taacaatacc tgggcccacc acaccgtgtg cattcgtaat gtctgcccat tctgctattc 4140
   tgtatacacc cgcagagtac tgcaatttga ctgtattacc aatgtcagca aattttctgt 4200
   cttcgaagag taaaaaattg tacttggcgg ataatgcctt tagcggctta actgtgccct 4260
   ccatggaaaa atcagtcaag atatccacat gtgtttttag taaacaaatt ttgggaccta 4320
   atgcttcaac taactccagt aattccttgg tggtacgaac atccaatgaa gcacacaagt 4380
   ttgtttgctt ttcgtgcatg atattaaata gcttggcagc aacaggacta ggatgagtag 4440
   cagcacgttc cttatatgta gctttcgaca tgatttatct tcgtttcctg caggtttttg 4500
   ttctgtgcag ttgggttaag aatactgggc aatttcatgt ttcttcaaca ctacatatgc 4560
   gtatatatac caatctaagt ctgtgctcct tccttcgttc ttccttctgt tcggagatta 4620
   ccgaatcaaa aaaatttcaa agaaaccgaa atcaaaaaaa agaataaaaa aaaaatgatg 4680
   aattgaattg aaaagctagc ttatcgatga taagctgtca aagatgagaa ttaattccac 4740
   ggactataga ctatactaga tactccgtct actgtacgat acacttccgc tcaggtcctt 4800
   gtcctttaac gaggccttac cactcttttg ttactctatt gatccagctc agcaaaggca 4860
   gtgtgatcta agattctatc ttcgcgatgt agtaaaacta gctagaccga gaaagagact 4920
   agaaatgcaa aaggcacttc tacaatggct gccatcatta ttatccgatg tgacgctgca 4980
   gcttctcaat gatattcgaa tacgctttga ggagatacag cctaatatcc gacaaactgt 5040
   tttacagatt tacgatcgta cttgttaccc atcattgaat tttgaacatc cgaacctggg 5100
   agttttccct gaaacagata gtatatttga acctgtataa taatatatag tctagcgctt 5160
   tacggaagac aatgtatgta tttcggttcc tggagaaact attgcatcta ttgcataggt 5220
   aatcttgcac gtcgcatccc cggttcattt tctgcgtttc catcttgcac ttcaatagca 5280
   tatctttgtt aacgaagcat ctgtgcttca ttttgtagaa caaaaatgca acgcgagagc 5340
   gctaattttt caaacaaaga atctgagctg catttttaca gaacagaaat gcaacgcgaa 5400
   agcgctattt taccaacgaa gaatctgtgc ttcatttttg taaaacaaaa atgcaacgcg 5460
   acgagagcgc taatttttca aacaaagaat ctgagctgca tttttacaga acagaaatgc 5520
   aacgcgagag cgctatttta ccaacaaaga atctatactt cttttttgtt ctacaaaaat 5580
   gcatcccgag agcgctattt ttctaacaaa gcatcttaga ttactttttt tctcctttgt 5640
   gcgctctata atgcagtctc ttgataactt tttgcactgt aggtccgtta aggttagaag 5700
   aaggctactt tggtgtctat tttctcttcc ataaaaaaag cctgactcca cttcccgcgt 5760
   ttactgatta ctagcgaagc tgcgggtgca ttttttcaag ataaaggcat ccccgattat 5820
   attctatacc gatgtggatt gcgcatactt tgtgaacaga aagtgatagc gttgatgatt 5880
   cttcattggt cagaaaatta tgaacggttt cttctatttt gtctctatat actacgtata 5940
   ggaaatgttt acattttcgt attgttttcg attcactcta tgaatagttc ttactacaat 6000
   ttttttgtct aaagagtaat actagagata aacataaaaa atgtagaggt cgagtttaga 6060
   tgcaagttca aggagcgaaa ggtggatggg taggttatat agggatatag cacagagata 6120
   tatagcaaag agatactttt gagcaatgtt tgtggaagcg gtattcgcaa tgggaagctc 6180
   caccccggtt gataatcaga aaagccccaa aaacaggaag attgtataag caaatattta 6240
   aattgtaaac gttaatattt tgttaaaatt cgcgttaaat ttttgttaaa tcagctcatt 6300
   ttttaacgaa tagcccgaaa tcggcaaaat cccttataaa tcaaaagaat agaccgagat 6360
   agggttgagt gttgttccag tttccaacaa gagtccacta ttaaagaacg tggactccaa 6420
   cgtcaaaggg cgaaaaaggg tctatcaggg cgatggccca ctacgtgaac catcacccta 6480
   atcaagtttt ttggggtcga ggtgccgtaa agcagtaaat cggaagggta aacggatgcc 6540
   cccatttaga gcttgacggg gaaagccggc gaacgtggcg agaaaggaag ggaagaaagc 6600
   gaaaggagcg ggggctaggg cggtgggaag tgtaggggtc acgctgggcg taaccaccac 6660
   acccgccgcg cttaatgggg cgctacaggg cgcgtgggga tgatccacta gt 6712
<210> 10
   <211> 23
   <212> DNA
   <213> Artificial
<220>
   <223> pri mer 5' - 119- Ot- LPCAT
<400> 10
   at gct ggt cg cgcgcgtccg agc 23
<210> 11
   <211> 27
   <212> DNA
   <213> Artificial
<220>
   <223> pr i mer 3' - 120- Ot- LPCAT- Xhol
<400> 11
   act cgagt ca cgagt t gt t c acgaggc 27
<210> 12
   <211> 34
   <212> DNA
   <213> Artificial
<220>
   <223> pr i mer 5' - 112- MA- LPCAT- BamH
<400> 12
   aggatccatg t ct t t t t acc t cgt cacct t cacc 34
<210> 13
   <211> 27
   <212> DNA
   <213> Artificial
<220>
   <223> pr i mer 3'-113- MA- LPCAT_XhoI
<400> 13
   actcgagtca cgagt act t g acaaggc 27
<210> 14
   <211> 23
   <212> DNA
   <213> Artificial
<220>
   <223> pr i mer 5' - 118- MA- LPCAT
<400> 14
   atgtcgaggt cgacggtatc gat 23
<210> 15
   <211> 23
   <212> DNA
   <213> Artificial
<220>
   <223> pr i mer Ot LPCAT- f or ward
<400> 15
   accat gct gg t cgcgcgcgt ccg 23
<210> 16
   <211> 20
   <212> DNA
   <213> Artificial
<220>
   <223> primer Ot LPCAT- r ever se
<400> 16
   tcacgagttg ttcacgaggc 20
<210> 17
   <211> 31
   <212> DNA
   <213> Artificial
<220>
   <223> pr imer PSUN-OtLPCAT-forward
<400> 17
   gcggccgcac catgctggtc gcgcgcgtcc g 31
<210> 18
   <211> 28
   <212> DNA
   <213> Artificial
<220>
   <223> pr imer PSUN-Ot LPCAT-reverse
<400> 18
   gcggccgctc acgagttgtt cacgaggc 28
<210> 19
   <211> 60
   <212> DNA
   <213> Artificial
<220>
   <223> T7 standard pr i mer
<400> 19
   gtcgacccgc ggact agt gg gccct ct aga cccgggggat ccggat ct gc t ggct at gaa 60
<210> 20
   <211> 23
   <212> DNA
   <213> Artificial
<220>
   <223> ol i gonucl eot i de primer
<400> 20
   accat gt ct tttacctcgt cac 23
<210> 21
   <211> 20
   <212> DNA
   <213> Artificial
<220>
   <223> ol i gonucl eot i de primer
<400> 21
   t cacgagt ac t t gacaaggc 20
<210> 22
   <211> 23
   <212> DNA
   <213> Artificial
<220>
   <223> oligonucleotide primer
<400> 22
   accat gt cga ggt cgacggt at c 23
<210> 23
   <211> 20
   <212> DNA
   <213> Artificial
<220>
   <223> ol i gonucl eot i de pr i mer
<400> 23
   t cacgagt ac t t gacaaggc 20
<210> 24
   <211> 31
   <212> DNA
   <213> Ar t i f i ci al
<220>
   <223> pr i mer PSUN-MsLPCAT112-forward
<400> 24
   gcggccgcac catgtctttt t t acct cgt ca c 31
<210> 25
   <211> 28
   <212> DNA
   <213> Arti f i ci al
<220>
   <223> primer PSUN MsLPCAT112-reverse
<400> 25
   gcggccgct c acgagt act t gacaaggc 28
<210> 26
   <211> 31
   <212> DNA
   <213> Ar t i f i ci al
<220>
   <223> pr i mer PSUN- MsLPCAT118- f or war d
<400> 26
   gcggccgcac cat gt cgagg t cgacggt at c 31
<210> 27
   <211> 28
   <212> DNA
   <213> Ar t i t i ci al
<220>
   <223> pr i mer PSUN-MsLPCAT118-reverse
<400> 27
   gcggccgctc acgagtactt gacaaggc 28

## Beschreibung

Die vorliegende Erfindung betrifft ein Verfahren zur Herstellung von mehrfach ungesättigten Fettsäuren in einem Organismus, indem Nukleinsäuren in den Organismus eingebracht werden, die für Polypeptide mit Acyl-CoA:Lysophospholipid-Acyltransferaseaktivität kodieren. Vorteilhaft können diese Nukleinsäuresequenzen gegebenenfalls zusammen mit weiteren Nukleinsäuresequenzen, die für Polypeptide des Fettsäure- oder Lipidstoffwechels kodieren, in dem transgenen Organismus exprimiert werden.

Die Erfindung betrifft weiterhin die erfindungsgemäßen Nukleinsäuresequenzen, Nukleinsäurekonstrukte enthaltend die erfindungsgemäßen Nukleinsäuresequenzen, Vektoren enthaltend die erfindungsgemäßen Nukleinsäuresequenzen und/oder die Nukleinsäurekonstrukte sowie transgene Organismen enthaltend die vorgenannten Nukleinsäuresequenzen, Nukleinsäurekonstrukte und/oder Vektoren.

Ein weiterer Teil der Offenbarung betrifft Öle, Lipide und/oder Fettsäuren hergestellt nach dem erfindungsgemäßen Verfahren und deren Verwendung.

Fettsäuren und Triglyceride finden eine Vielzahl von Anwendungen in der Lebensmittelindustrie, der Tierernährung, der Kosmetik und im Pharmabereich. Je nachdem, ob es sich um freie gesättigte oder ungesättigte Fettsäuren oder um Triglyceride mit einem erhöhten Gehalt an gesättigten oder ungesättigten Fettsäuren handelt, sind sie für die unterschiedlichsten Anwendungen geeignet, so werden beispielsweise mehrfach ungesättigte Fettsäuren der Babynahrung zur Erhöhung des Nährwertes zugesetzt. Mehrfach ungesättigte ω-3- und ω-6-Fettsäuren stellen dabei einen wichtigen Bestandteil der tierischen und menschlichen Nahrung dar. Aufgrund der heute üblichen Zusammensetzung der menschlichen Nahrung ist ein Zusatz von mehrfach ungesättigten ω-3-Fettsäuren, die vor allem in Fischölen vorkommen, zur Nahrung besonders wichtig. So werden beispielsweise der Babynahrung mehrfach ungesättigte ω-3-Fettsäuren wie Docosahexaensäure (= DHA, C22:6^{Δ4,7,10,13,16,19}) oder Eisosapentaensäure (= EPA, C20:5^{Δ5,8,11,14,17}) zur Erhöhung des Nährwertes zugesetzt. Der ungesättigten Fettsäure DHA wird dabei ein positiver Effekt auf die Entwicklung des Gehirns zugeschrieben.

Den mehrfach ungesättigten ω-3-Fettsäuren wird auch ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche, speziell chronisch entzündliche Prozesse im Rahmen immunologischer Erkrankungen wie rheumatoider Arthritis lassen sich durch ω-3-Fettsäuren positiv beeinflussen. Sie werden deshalb Lebensmitteln, speziell diätischen Lebensmitteln, zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, und den Thromboxanen und Leukotrienen, die sich von der Arachidonsäure und der Eicosapentaensäure ableiten. Eicosanoide, die aus ω-6-Fettsäuren gebildet werden (sog. PG₂-Serie), fördern in der Regel Entzündungsreaktionen, während Eicosanoide aus ω-3-Fettsäuren (sog. PG₃-Serie) geringe oder keine entzündungsfördernde Wirkung haben.

Im Folgenden werden mehrfach ungesättigte Fettsäuren als PUFA, PUFAs, LCPUFA oder LCPUFAs bezeichnet (**p**oly **u**nsaturated **f**atty **a**cid = **PUFA**; **l**ong **c**hain **p**oly **u**nsaturated **f**atty **a**cid = **LCPUFA**).

Die verschiedenen Fettsäuren und Triglyceride werden hauptsächlich aus Mikroorganismen wie Mortierella oder Schizochytrium oder aus Öl-produzierenden Pflanzen wie Soja oder Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Höhere mehrfach ungesättigte Fettsäuren wie DHA, EPA, Arachidonsäure (= ARA, C20:4^{Δ5,8,11,14}), Dihomo-γ-linolensäure (C20:3^{Δ8,11,14}) oder Docosapentaensäure (DPA, C22:5^{Δ7,10,13,16,19}) lassen sich nicht aus Ölfruchtpflanzen wie Raps, Soja, Sonnenblume, Färberdistel oder anderen isolieren. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch sowie Algen.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase und in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al. (1990) J. Biol. Chem. 265: 20144-20149, Wada et al. (1990) Nature 347: 200-203 oder Huang et al. (1999) Lipids 34: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und charakterisieren sind (McKeon et al. (1981) Methods in Enzymol. 71: 12141-12147, Wang et al. (1988) Plant Physiol. Biochem. 26: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen sind in WO 93/06712, US 5,614,393, WO 96/21022, WO00/21557 und WO 99/27111 beschrieben, während deren Anwendung zur Produktion von Fettsäuren in transgenen Organismen in WO98/46763, WO98/46764 und WO98/46765 beschrieben ist. Dabei wird auch die Expression verschiedener Desaturasen wie in WO99/64616 oder WO98/46776 und Bildung mehrfach ungesättigter Fettsäuren beschrieben und beansprucht. Bezüglich der Effektivität der Expression von Desaturasen und ihren Einfluss auf die Bildung mehrfach ungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus ω-3- und ω-6-Fettsäuren erhalten.

WO 2004/076617A betrifft ein Verfahren zum Herstellen mehrfach ungesättigter Fettsäuren in einem Organismus durch Einführen von Nukleinsäuren codierend für Polypeptide mit Acyl-CoA:Lysophospholipid-Acyltransferase-Aktivität in den Organismus.

Besonders geeignete Mikroorganismen zur Herstellung von PUFAs sind Mikroorganismen wie Thraustochytrien- oder Schizochytrien-Stämme, Algen wie *Phaeodactylum tricornutum* oder Crypthecodinium-Arten, Ciliaten wie *Stylonychia* oder *Colpidium* und Pilze wie *Mortierella, Entomophthora* oder *Mucor.* Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wann immer möglich, wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen, wobei diese in der Regel zudem je nach verwendetem Mikroorganismus als Fettsäuregemische aus beispielsweise EPA, DPA und DHA anfallen.

Alternativ kann die Produktion von Feinchemikalien im großen Maßstab vorteilhaft in Pflanzen durchgeführt werden, die so entwickelt werden, dass sie die vorstehend genannten PUFAs herstellen. Besonders gut für diesen Zweck geeignete Pflanzen sind Ölfruchtpflanzen, die große Mengen an Lipidverbindungen enthalten, wie Raps, Canola, Lein, Soja, Sonnenblumen, Borretsch und Nachtkerze. Aber auch andere Nutzpflanzen, die Öle oder Lipide und Fettsäuren enthalten, sind gut geeignet, wie in der eingehenden Beschreibung dieser Erfindung erwähnt. Mittels herkömmlicher Züchtung ist eine Reihe von Mutantenpflanzen entwickelt worden, die ein Spektrum an wünschenswerten Lipiden und Fettsäuren, Cofaktoren und Enzymen produzieren. Die Selektion neuer Pflanzensorten mit verbesserter Produktion eines bestimmten Moleküls ist jedoch ein zeitaufwändiges und schwieriges Verfahren oder sogar unmöglich, wenn die Verbindung in der entsprechenden Pflanze nicht natürlich vorkommt, wie im Fall von mehrfach ungesättigten C₁₈-, C₂₀-und C₂₂-Fettsäuren und Fettsäuren mit längeren Kohlenstoffketten.

In der Vergangenheit hat es daher nicht an Ansätzen gefehlt, Gene, die an der Synthese von Fettsäuren bzw. Triglyceriden beteiligt sind, für die Herstellung von Ölen in verschiedenen Pflanzen mit einem geändertem Gehalt an mehrfach ungesättigten Fettsäuren verfügbar zu machen. Bisher konnten jedoch längerkettige mehrfach ungesättigte C₂₀- und/oder C₂₂-Fettsäuren wie EPA oder ARA nicht in Pflanzen hergestellt werden.

Aber auch in anderen Organismen wie Mikroorganismen wie Algen oder Pilzen führten die gentechnischen Veränderungen des Fettsäurestoffwechselweges über das Einbringen und die Expression beispielsweise von Desaturasen nur zu relativ geringen Steigerungen der Produktivität in diesen Organismen. Ein Grund hierfür mag in dem sehr komplexen Fettsäurestoffwechsel liegen. So ist der Einbau von mehrfach ungesättigten Fettsäuren in Membranlipide und/oder in Triacylglyceride und deren Ab- und Umbau sehr komplex und bis heute biochemisch und speziell genetisch noch nicht vollständig aufgeklärt und verstanden.

Die Biosynthese von LCPUFAs und der Einbau von LCPUFAs in Membranen oder Triacylglyceride erfolgt über verschiedene Stoffwechselwege (Abbadi et al. (2001) European Journal of Lipid Science & Technology 103:106-113). In Bakterien wie *Vibrio* und Mikroalgen wie *Schizochytrium* wird Malonyl-CoA über eine LCPUFAproduzierende Polyketidsynthase zu LCPUFAs umgesetzt (Metz et al. (2001) Science 293: 290-293; WO 00/42195; WO 98/27203; WO 98/55625). In Mikroalgen wie *Phaeodactylum* und Moosen wie *Physcomitrella* werden ungesättigte Fettsäuren wie Linolsäure oder Linolensäure in Form ihrer Acyl-CoAs in mehreren Desaturierungs- und Elongationsschritten zu LCPUFAs umgesetzt (Zank et al. (2000) Biochemical Society Transactions 28: 654-658). Bei Säugetieren beinhaltet die Biosynthese von DHA zusätzlich zu Desaturierungs- und Elongationsschritten eine Kettenverkürzung über β-Oxidation.

LCPUFAs liegen in Mikroorganismen und niederen Pflanzen entweder ausschließlich in Form von Membranlipiden vor, wie bei *Physcomitrella* und *Phaeodactylum,* oder sie sind sowohl in Membranlipiden als auch in Triacylglyceriden vorhanden, wie bei *Schizochytrium* und *Mortierella.* Der Einbau von LCPUFAs in Lipide und Öle wird durch verschiedene Acyltransferasen und Transacylasen katalysiert, die bereits bekannt für den Einbau von gesättigten und ungesättigten Fettsäuren sind (Slabas (2001) J. Plant Physiology 158: 505-513; Frentzen (1998) Fett/Lipid 100: 161-166; Cases et al. (1998) Proc. Nat. Acad. Sci. USA 95: 13018-13023). Bei den Acyltransferasen handelt sich um Enzyme des sogenannten Kennedy-Pathways, die an der cytoplasmatischen Seite des Membransystems des Endoplasmatischen Reticulums, nachfolgend als 'ER' bezeichnet, lokalisiert sind. Experimentell können Membranen des ER als sogenannte ,mikrosomale Fraktionen' aus verschiedenen Organismen isoliert werden (Knutzon et al. (1995) Plant Physiology 109: 999-1006; Mishra & Kamisaka (2001) Biochemistry 355: 315-322; US 5968791). Diese ER-gebundenen Acyltransferasen in der mikrosomalen Fraktion verwenden Acyl-CoA als aktivierte Form der Fettsäuren. Glycerin-3-phosphat-Acyltransferase, im folgenden GPAT genannt, katalysiert den Einbau von Acylgruppen an der sn-1 Position von Glycerin-3-phosphat. 1-Acylglycerin-3-phosphat-Acyltransferase (E.C. 2.3.1.51), auch Lysophosphatidsäure-Acyltransferase, im Folgenden LPAAT genannt, katalysiert den Einbau von Acylgruppen an der sn-2 Position von Lysophosphatidsäure, nachfolgend als LPA abgekürzt. Nach Dephosphorylierung von Phosphatidsäure durch Phosphatidsäure-Phosphatase katalysiert Diacylglycerin-Acyltransferase, im Folgenden DAGAT genannt, den Einbau von Acylgruppen an der sn-3-Position des Diacylglycerins. Neben diesen Enzymen des Kennedy-Pathways sind weitere Enzyme am Einbau von Fettsäuren in Triacylglyceride beteiligt, die Acylgruppen aus Membranlipiden in Triacylglyceride einbauen können, wie etwa die Phospholipid-Diacylglycerin-Acyltransferase, nachfolgend PDAT genannt, und die Lysophosphatidylcholin-Acyltransferase, nachfolgend LPCAT genannt.

Die enzymatische Aktivität einer LPCAT wurde erstmals in Ratten beschrieben (Land (1960) J. Biol. Chem. 235: 2233-2237). In Pflanzen existiert eine plastidäre Isoform der LPCAT (Akermoun et al. (2000) Biochemical Society Transactions 28: 713-715) sowie eine ER-gebundene Isoform (Tumaney und Rajasekharan (1999) Biochimica et Biophysica Acta 1439: 47-56; Fraser und Stobart, Biochemical Society Transactions (2000) 28: 715-7718). LPCATs sind in Tieren wie auch in Pflanzen an der Biosynthese und der Transacylierung von mehrfach ungesättigten Fettsäuren beteiligt (Stymne und Stobart (1984) Biochem. J. 223: 305-314; Stymne und Stobart (1987) in 'The Biochemistry of Plants: a Comprehensive Treatise', Vol. 9 (Stumpf, P.K. ed.) pp. 175-214, Academic Press, New York). Eine wichtige Funktion der LPCAT oder allgemeiner gesagt einer Acyl-CoA:Lysophospholipid-Acyltransferase, nachfolgend LPLAT genannt, bei der ATP-unabhängigen Synthese von Acyl-CoA aus Phospholipiden wurde von Yamashita et al. (2001) J. Biol. Chem. 276: 26745-26752 beschrieben.

Höhere Pflanzen enthalten mehrfach ungesättigte Fettsäuren wie Linolsäure (C18:2) und Linolensäure (C 18:3). Arachidonsäure (ARA), Eicosapentaensäure (EPA) und Docosahexaensäure (DHA) kommen hingegen im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es ist vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu werden vorteilhaft über gentechnische Methoden Gene kodierend für Enzyme der Biosynthese von LCPUFAs in Ölsaaten eingeführt und exprimiert. Dies sind beispielsweise Gene kodierend für eine Δ-6-Desaturase, Δ-6-Elongase, Δ-5-Desaturase, Δ-5-Elongasen und Δ-4-Desaturase. Diese Gene können vorteilhaft aus Mikroorganismen, Tieren und niederen Pflanzen isoliert werden, die LCPUFAs herstellen und in die Membranen oder Triacylglyceride einbauen. So konnten bereits Δ-6-Desaturase-Gene aus dem Moos *Physcomitrella patens* und Δ-6-Elongase-Gene aus *P. patens* und dem Nematoden *C. elegans* isoliert werden.

Erste transgene Pflanzen, die Gene kodierend für Enzyme der LCPUFA-Biosynthese enthalten und exprimieren und LCPUFAs produzieren wurden in DE 102 19 203 (Verfahren zur Herstellung mehrfach ungesättigter Fettsäuren in Pflanzen) erstmals beschrieben. Diese Pflanzen produzieren allerdings LCPUFAs in Mengen, die für eine Aufarbeitung der in den Pflanzen enthaltenen Öle noch weiter optimiert werden müssen.

Um eine Anreicherung der Nahrung und des Futters mit diesen mehrfach ungesättigten Fettsäuren zu ermöglichen, besteht daher ein großer Bedarf an einem einfachen, kostengünstigen Verfahren zur Herstellung dieser mehrfach ungesättigten Fettsäuren speziell in eukaryontischen Systemen.

Es bestand daher die Aufgabe, ein Verfahren zur Herstellung von mehrfach ungesättigten Fettsäuren in einem eukaryontischen Organismus zu entwickeln. Diese Aufgabe wurde durch das erfindungsgemäße Verfahren zur Herstellung mehrfach ungesättigter Fettsäuren in einem Organismus gelöst, das dadurch gekennzeichnet ist, dass es folgende Schritte umfasst:
a) Einbringen mindestens einer Nukleinsäuresequenz in den Organismus ausgewählt aus der Gruppe bestehend aus:
   i) Nukleinsäuresequenzen mit der in SEQ ID NO: 3 dargestellten Sequenz, die für ein Polypeptid mit einer Acyl-CoA:Lysophospholipid-Acyltransferaseaktivität kodieren; oder
   ii) Nukleinsäuresequenzen, die sich als Ergebnis des degenerierten genetischen Codes von der in SEQ ID NO: 3 dargestellten Sequenz ableiten lassen; oder
   iii) Derivaten der in SEQ ID NO: 3 dargestellten Nukleinsäuresequenz, die für Polypeptide mit der in SEQ ID NO:4 dargestellten Aminosäuresequenz kodieren und mindestens 70% Identität auf Aminosäureebene mit SEQ ID NO: 4 zeigen und eine äquivalente Acyl-CoA:Lysophospholipid-Acyltransferaseaktivität aufweisen,
      und
b) Kultivieren und Ernten des Organismus.

Vorteilhaft enthalten die im erfindungsgemäßen Verfahren hergestellten mehrfach ungesättigten Fettsäuren mindestens zwei, vorteilhaft drei Doppelbindungen. Besonders vorteilhaft enthalten die Fettsäuren vier oder fünf Doppelbindungen. Im Verfahren hergestellte Fettsäuren haben vorteilhaft 16-, 18-, 20- oder 22 C-Atome in der Fettsäurekette. Diese Fettsäuren können als einziges Produkt im Verfahren hergestellt werden oder in einem Fettsäuregemisch vorliegen.

Bei den im erfindungsgemäßen Verfahren verwendeten Nukleinsäuresequenzen handelt es sich um isolierte Nukleinsäuresequenzen, die für Polypeptide mit Acyl-CoA:Lysophospholipid-Acyltransferaseaktivität kodieren.

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie wie gesagt als "Reinprodukte" oder aber vorteilhaft in Form von Mischungen verschiedener Fettsäuren oder Mischungen unterschiedlicher Glyceride vorliegen. Dabei lassen sich die in den Triacylglyceriden gebundenen verschiedenen Fettsäuren von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind langkettige Fettsäuren, besonders bevorzugt sind die langkettigen C₁₈-, C₂₀- und/oder C₂₂-Fettsäuren (LCPUFAs).

Im erfindungsgemäßen Verfahren werden vorteilhaft Fettsäureester mit mehrfach ungesättigten C₁₆-, C₁₈-, C₂₀- und/oder C₂₂-Fettsäuremolekülen mit mindestens zwei Doppelbindungen im Fettsäureester hergestellt. Bevorzugt enthalten diese Fettsäuremoleküle drei, vier oder fünf Doppelbindungen und führen vorteilhaft zur Synthese von Hexadecadiensäure (C16:2^{Δ9,12}), γ-Linolensäure (= GLA, C18:3^{Δ6,9,12}), Stearidonsäure (= SDA, C18:4 ^{Δ6,9,12,15}), Dihomo-γ-Linolensäure (= DGLA, 20:3 ^{Δ8,11,14}), Eicosatetraensäure (= ETA, C20:4^{Δ5,8,11,14}), Arachidonsäure (ARA), Eicosapentaensäure (EPA) oder deren Mischungen, bevorzugt zur Synthese von EPA und/oder ARA.

Die Fettsäureester mit mehrfach ungesättigten C₁₆-, C₁₈-, C₂₀- und/oder C₂₂-Fettsäuremolekülen können aus den Organismen, die für die Herstellung der Fettsäureester verwendet wurden, in Form eines Öls oder Lipids, beispielsweise in Form von Verbindungen wie Sphingolipiden, Phosphoglyceriden, Lipiden, Glykolipiden wie Glykosphingolipid, Phospholipiden wie Phosphatidylethanolamin, Phosphatidylcholin, Phosphatidylserin, Phosphatidylglycerol, Phosphatidylinositol oder Diphosphatidylglycerol, Monoacylglyceriden, Diacylglyceriden, Triacylglyceriden oder sonstigen Fettsäureestern wie den Acetyl-CoenzymA-Estern, die die mehrfach ungesättigten Fettsäuren mit mindestens zwei, bevorzugt drei, Doppelbindungen enthalten, isoliert werden. Neben diesen Estern sind die mehrfach ungesättigten Fettsäuren auch als freie Fettsäuren oder gebunden in anderen Verbindungen in den Organismen, vorteilhaft den Pflanzen, enthalten. In der Regel liegen die verschiedenen vorgenannten Verbindungen (Fettsäureester und freie Fettsäuren) in den Organismen in einer ungefähren Verteilung von 80 bis 90 Gew.-% Triglyceriden, 2 bis 5 Gew.-% Diglyceriden, 5 bis 10 Gew.-% Monoglyceriden, 1 bis 5 Gew.-% freien Fettsäuren, 2 bis 8 Gew.-% Phospholipiden vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-%, am meisten bevorzugt von mindestens 15 Gew.-%, bezogen auf die gesamten Fettsäuren in den transgenen Organismen, vorteilhaft einer transgenen Pflanze, hergestellt. Da im erfindungsgemäßen Verfahren ausgehend von den Verbindungen Hexadecadiensäure (C16:2), Linolsäure (C 18:2) bzw. Linolensäure (C 18:3) mehrere Reaktionsschritte durchlaufen werden, fallen die Endprodukte des Verfahrens wie beispielsweise Arachidonsäure (ARA) oder Eicosapentaensäure (EPA) nicht als absolute Reinprodukte an, sondern es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA und EPA als Mischungen vor. Die Vorstufen sollten vorteilhaft nicht mehr als 20 Gew.-%, bevorzugt nicht mehr als 15 Gew.-%, besonders bevorzugt nicht mehr als 10 Gew.-%, am meisten bevorzugt nicht mehr als 5, 4, 3, 2, 1 Gew.-%, bezogen auf die Menge des jeweilige Endprodukts, betragen. Vorteilhaft werden in einer transgenen Pflanze als Endprodukte nur ARA oder nur EPA im erfindungsgemäßen Verfahren gebunden oder als freie Fettsäuren hergestellt. Werden beide Verbindungen (ARA + EPA) gleichzeitig hergestellt, werden sie vorteilhaft in einem Verhältnis von mindestens 1:2 (EPA:ARA), vorteilhaft von mindestens 1:3, bevorzugt von mindestens 1:4, besonders bevorzugt von mindestens 1:5 hergestellt.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Organismus für die Herstellung im erfindungsgemäßen Verfahren kommen prinzipiell alle Organismen wie Pilze wie *Mortierella* oder *Traustochytrium,* Hefen wie *Saccharomyces* oder *Schizosaccharomyces,* Moose wie *Physcomitrella* oder *Ceratodon,* nicht-humane Tiere wie *Caenorhabditis,* Algen wie *Crypthecodinium* oder *Phaeodactylum* oder Pflanzen wie zweikeimblättrige oder einkeimblättrige Pflanzen in Frage. Vorteilhaft werden im erfindungsgemäßen Verfahren Organismen verwendet, die zu den Öl-produzierenden Organismen gehören, das heißt, die für die Herstellung von Ölen verwendet werden, wie Pilze wie *Mortierella* oder *Traustochytrium,* Algen wie *Crypthecodinium* oder *Phaeodactylum* oder Pflanzen, insbesondere Ölfruchtpflanzen, die große Mengen an Lipidverbindungen enthalten, wie Erdnuss, Raps, Canola, Sonnenblume, Safflor (Färberdistel), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte wie Mais, Weizen, Roggen, Hafer, Triticale, Reis, Gerste, Baumwolle, Maniok, Pfeffer, Tagetes, Solanaceen-Pflanzen wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Safflor (Färberdistel), Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure-reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Am meisten bevorzugt sind Pflanzen wie Färberdistel, Sonnenblume, Mohn, Nachtkerze, Walnuss, Lein oder Hanf.

Für das erfindungsgemäße beschriebene Verfahren ist es vorteilhaft, zusätzlich zu den unter Verfahrensschritt (a) eingebrachten Nukleinsäuren weitere Nukleinsäuren in den Organismus einzubringen, die für Enzyme des Fettsäure- oder Lipidstoffwechsels kodieren.

Im Prinzip können alle Gene des Fettsäure- oder Lipidstoffwechsels vorteilhaft in Kombination mit der erfinderischen Acyl-CoA:Lysophospholipid-Acyltransferase im Verfahren zur Herstellung mehrfach ungesättigter Fettsäuren verwendet werden, vorteilhaft werden Gene des Fettsäure- oder Lipidstoffwechsels ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-Acyl-Transferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Acyl-CoA:Lysophospholipid-Acyltransferase verwendet. Besonders bevorzugt werden Gene ausgewählt aus der Gruppe der Δ-4-Desaturasen, Δ-5-Desaturasen, Δ-6-Desaturasen, Δ-8-Desaturasen, Δ-9-Desaturasen, Δ-12-Desaturasen, Δ-5-Elongasen, Δ-6-Elongasen oder Δ-9-Elongasen in Kombination mit der erfinderischen Acyl-CoA:Lysophospholipid-Acyltransferase im erfindungsgemäßen Verfahren verwendet.

Durch die enzymatische Aktivität der im erfindungsgemäßen Verfahren verwendeten Nukleinsäuren, die für Polypeptide mit Acyl-CoA:Lysophospholipid-Acyltransferaseaktivität kodieren, vorteilhaft in Kombination mit Nukleinsäuresequenzen, die für Polypeptide des Fettsäure- oder Lipidstoffwechsels mit Δ-4-, Δ-5-, Δ-6-, Δ-8-Desaturase- oder Δ-5-, Δ-6-oder Δ-9-Elongaseaktivität kodieren, können im erfindungsgemäßen Verfahren unterschiedlichste mehrfach ungesättigte Fettsäuren hergestellt werden. Je nach Auswahl der für das erfindungsgemäße Verfahren verwendeten Organismen wie den vorteilhaften Pflanzen lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder solche, die sich von C18:3-Fettsäuren ableiten, wie SDA, ETA oder EPA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15}) vorhanden, beispielsweise wie in Lein, so können als Produkte des Verfahrens nur SDA, ETA und EPA entstehen, die wie oben beschrieben als freie Fettsäuren oder gebunden vorliegen können. Durch Modifikation der Aktivität der an der Synthese beteiligten Enzyme Acyl-CoA:Lysophospholipid-Acyltransferase vorteilhaft in Kombination mit der Δ-5- und Δ-6-Desaturase sowie der Δ-6-Elongase, oder der Δ-5- und Δ-8-Desaturase sowie der Δ-9-Elongase oder in Kombination mit nur den ersten beiden Genen der Synthesekette, Δ-6-Desaturase und Δ-6-Elongase oder Δ-8-Desaturase und Δ-9-Elongase, lassen sich in den vorgenannten Organismen, vorteilhaft in den vorgenannten Pflanzen, gezielt nur einzelne Produkte herstellen. Durch die Aktivität der Δ-6-Desaturase und Δ-6-Elongase entstehen je nach Ausgangspflanze und ungesättigter Fettsäure beispielsweise GLA und DGLA bzw. SDA und ETA. Bevorzugt entstehen DGLA bzw. ETA oder deren Mischungen. Wird zusätzlich die Δ-5-Desaturase in die Organismen, vorteilhaft in die Pflanze, eingebracht, so entstehen auch ARA oder EPA. Dies gilt auch für Organismen, in die vorher die Δ-8-Desaturase und Δ-9-Elongase eingebracht wurde.

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft, die Menge an Ausgangsprodukt für die Fettsäuresynthese zu steigern. Dies kann beispielsweise durch das Einbringen einer Nukleinsäure, die für ein Polypeptid mit Δ-12-Desaturaseaktivität kodiert, in den Organismus erreicht werden. Dies ist besonders vorteilhaft in Öl-produzierenden Organismen wie Raps, die einen hohen Ölsäuregehalt aufweisen. Da diese Organismen nur einen geringen Gehalt an Linolsäure aufweisen (Mikoklajczak et al., Journal of the American Oil Chemical Society 38: 1961, 678 - 681) ist die Verwendung einer Δ-12-Desaturase zur Herstellung des Ausgangsprodukts Linolsäure vorteilhaft.

Die im erfindungsgemäßen Verfahren verwendeten Nukleinsäuren stammen vorteilhaft aus Pflanzen wie Algen wie *Isochrysis* oder *Crypthecodinium,* Algen/Diatomeen wie *Phaeodactylum,* Moosen wie *Physcomitrella* oder *Ceratodon* oder höheren Pflanzen wie den Primulaceae wie *Aleuritia, Calendula stellata, Osteospermum spinescens* oder *Osteospermum hyoseroides,* Mikroorganismen wie Pilzen wie *Aspergillus, Thraustochytrium, Phytophtora, Entomophthora, Mucor* oder *Mortierella,* Hefen oder Tieren wie Nematoden wie *Caenorhabditis*, Insekten oder dem Menschen. Vorteilhaft stammen die Nukleinsäuren aus Pilzen, Tieren oder aus Pflanzen wie Algen oder Moosen, bevorzugt aus Nematoden wie Caenorhabditis.

Vorteilhaft werden im erfindungsgemäßen Verfahren die vorgenannten Nukleinsäuresequenzen oder deren Derivate oder Homologe, die für Polypeptide kodieren, die noch die enzymatische Aktivität der durch die Wildtyp-Nukleinsäuresequenzen kodierten Proteine besitzen, verwendet. Diese Sequenzen werden einzeln oder in Kombination mit der für die Acyl-CoA:Lysophospholipid-Acyltransferase kodierenden Nukleinsäuresequenz in Expressionskonstrukte kloniert und zum Einbringen und zur Expression in Organismen verwendet. Diese Expressionskonstrukte ermöglichen eine optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder eines ganzen Organismus, der die im Verfahren verwendeten Nukleinsäuresequenzen enthält, wobei die Zelle und/oder der Organismus mit der erfindungsgemäßen Nukleinsäuresequenz, die für die Acyl-CoA:Lysophospholipid-Acyltransferase kodiert, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels kodieren, transformiert wird. Bei einer weiteren bevorzugten Ausführungsform umfasst dieses Verfahren ferner den Schritt des Gewinnens der Feinchemikalie aus der Kultur. Bei der Kultur kann es sich beispielsweise um eine Fermentationskultur, beispielsweise im Falle der Kultivierung von Mikroorganismen wie z.B. *Mortierella, Saccharomyces* oder *Traustochytrium,* oder um eine Treibhaus- oder Feldkultur einer Pflanze handeln. Die so hergestellte Zelle oder der so hergestellte Organismus ist vorteilhaft eine Zelle eines Öl-produzierenden Organismus wie einer Ölfruchtpflanze wie beispielsweise Erdnuss, Raps, Canola, Lein, Hanf, Erdnuss, Soja, Safflower, Hanf, Sonnenblumen oder Borretsch.

Unter "Anzucht" ist beispielsweise im Falle von Pflanzenzellen, -geweben oder -organen die Kultivierung auf oder in einem Nährmedium oder im Falle der ganzen Pflanze die Kultivierung auf bzw. in einem Substrat beispielsweise in Hydrokultur, Blumentopferde oder auf einem Ackerboden zu verstehen.

"Transgen" bzw. "rekombinant" im Sinne der Erfindung bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassetten oder Vektoren alle durch gentechnische Methoden zustande gekommenen Konstruktionen, in denen sich entweder
a) die erfindungsgemäße Nukleinsäuresequenz oder
b) eine mit der erfindungsgemäßen Nukleinsäuresequenz funktionell verknüpfte genetische Kontrollsequenz, zum Beispiel ein Promotor oder
c) (a) und (b)
nicht in ihrer natürlichen, genetischen Umgebung befinden oder durch gentechnische Methoden modifiziert wurden, wobei es sich bei der Modifikation beispielhaft um eine Substitution, Addition, Deletion, Inversion oder Insertion eines oder mehrerer Nukleotidreste handeln kann.

"Natürliche genetische Umgebung" meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der erfindungsgemäßen Nukleinsäuresequenz mit dem entsprechenden Acyl-CoA:Lysophospholipid-Acyltransferase -Gen - wird zu einer transgenen Expressionskassette, wenn diese durch nicht-natürliche, synthetische ("künstliche") Verfahren wie beispielsweise eine Mutagenisierung geändert wird. Entsprechende Verfahren sind beispielsweise beschrieben in US 5,565,350 oder WO 00/15815.

Unter transgenem Organismus bzw. transgener Pflanze im Sinne der Erfindung ist wie vorgenannt zu verstehen, dass sich die im Verfahren verwendeten Nukleinsäuren nicht an ihrer natürlichen Stelle im Genom eines Organismus befinden, wobei die Nukleinsäuren homolog oder heterolog exprimiert werden können. Transgen bedeutet aber auch wie genannt, dass sich die erfindungsgemäßen Nukleinsäuren an ihrem natürlichen Platz im Genom eines Organismus befinden, dass jedoch die Sequenz gegenüber der natürlichen Sequenz verändert wurde und/oder dass die Regulationssequenzen der natürlichen Sequenzen verändert wurden. Bevorzugt ist unter transgen die Expression der erfindungsgemäßen Nukleinsäuren an nichtnatürlicher Stelle im Genom zu verstehen, das heißt eine homologe oder bevorzugt heterologe Expression der Nukleinsäuren liegt vor. Bevorzugte transgene Organismen sind Pilze wie *Mortierella* oder Pflanzen wie Ölfruchtpflanzen.

Als Organismen bzw. Wirtsorganismen für die im erfindungsgemäßen Verfahren verwendeten Nukleinsäuren, die Expressionskassetten oder Vektoren eignen sich prinzipiell vorteilhaft alle Organismen, die in der Lage sind, Fettsäuren, speziell ungesättigte Fettsäuren, zu synthetisieren bzw. die für die Expression rekombinanter Gene geeignet sind. Beispielhaft seien Pflanzen wie *Arabidopsis,* Asteraceae wie *Calendula* oder Kulturpflanzen wie Soja, Erdnuss, Rizinus, Sonnenblume, Mais, Baumwolle, Flachs, Raps, Kokosnuss, Ölpalme, Färbersafflor (*Carthamus tinctorius*) oder Kakaobohne, Mikroorganismen wie Pilze beispielsweise der Gattung *Mortierella, Saprolegnia* oder *Pythium,* Bakterien wie die Gattung *Escherichia,* Hefen wie die Gattung *Saccharomyces,* Cyanobakterien, Ciliaten, Algen oder Protozoen wie Dinoflagellaten wie *Crypthecodinium* genannt. Bevorzugt sind Organismen, die natürlicherweise Öle in größeren Mengen synthetisieren können, wie Pilze wie z.B. *Mortierella alpina, Pythium insidiosum* oder Pflanzen wie Soja, Raps, Kokosnuss, Ölpalme, Färbersafflor, Flachs, Hanf, Rizinus, Calendula, Erdnuss, Kakaobohne oder Sonnenblume oder Hefen wie *Saccharomyces cerevisiae,* besonders bevorzugt sind Soja, Flachs, Raps, Färbersafflor, Sonnenblume, Calendula, *Mortierella* oder *Saccharomyces cerevisiae.* Prinzipiell sind als Wirtsorganismen neben den vorgenannten transgenen Organismen auch transgene Tiere, vorteilhaft nicht-humane Tiere wie beispielsweise *C. elegans* geeignet.

Nutzbare Wirtszellen sind weiterhin genannt in: Goeddel, Gene Expression Technology: Methods in Enzymology 185, Academic Press, San Diego, CA (1990).

Verwendbare Expressionsstämme, z.B. solche, die eine geringere Proteaseaktivität aufweisen, sind beschrieben in: Gottesman, S., Gene Expression Technology: Methods in Enzymology 185, Academic Press, San Diego, California (1990) 119-128.

Transgene Pflanzen, die die im erfindungsgemäßen Verfahren synthetisierten mehrfach ungesättigten Fettsäuren enthalten, können vorteilhaft direkt vermarktet werden, ohne dass die synthetisierten Öle, Lipide oder Fettsäuren isoliert werden müssen. Unter Pflanzen im erfindungsgemäßen Verfahren sind ganze Pflanzen sowie alle Pflanzengewebe, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, zu verstehen, die sich von der transgenen Pflanze ableiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen, vorteilhaft Pflanzen, in Form ihrer Öle, Fette, Lipide und/oder freien Fettsäuren isoliert werden. Durch dieses Verfahren hergestellte mehrfach ungesättigte Fettsäuren lassen sich durch Ernten der Organismen entweder aus der Kultur, in der sie wachsen, oder vom Feld gewinnen. Dies kann über Pressen oder Extraktion der Pflanzenteile, bevorzugt der Pflanzensamen, erfolgen. Dabei können die Öle, Fette, Lipide und/oder freien Fettsäuren durch sogenanntes Kaltschlagen oder Kaltpressen ohne Zuführung von Wärme durch Pressen gewonnen werden. Damit sich die Pflanzenteile, speziell die Samen, leichter aufschließen lassen, werden sie vorher zerkleinert, gedämpft oder geröstet. Die so vorbehandelten Samen können anschließend gepresst werden oder mit Lösungsmittel wie warmem Hexan extrahiert werden. Anschließend wird das Lösungsmittel wieder entfernt. Im Falle von Mikroorganismen werden diese nach der Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base, beispielsweise Natronlauge, entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen, werden die Produkte einer Bleichung mit beispielsweise Bleicherde oder Aktivkohle unterzogen. Zum Schluss wird das Produkt noch beispielsweise mit Wasserdampf desodoriert.

Vorzugsweise sind die durch dieses Verfahren produzierten PUFAs bzw. LCPUFAs C₁₈-, C₂₀- oder C₂₂-Fettsäuremoleküle mit mindestens zwei Doppelbindungen im Fettsäuremolekül, vorzugsweise drei, vier, fünf oder sechs Doppelbindungen. Diese C₁₈-, C₂₀- oder C₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind transgene Pflanzen.

Eine hier beschriebene Ausführungsform sind deshalb Öle, Lipide oder Fettsäuren oder Fraktionen davon, die durch das oben beschriebene Verfahren hergestellt worden sind, besonders bevorzugt Öle, Lipide oder eine Fettsäurezusammensetzung, die PUFAs umfassen und von transgenen Pflanzen herrühren.

Eine weitere hier beschriebene Ausführungsform ist die Verwendung des Öls, Lipids, der Fettsäuren und/oder der Fettsäurezusammensetzung in Futtermitteln, Nahrungsmitteln, Kosmetika oder Pharmazeutika.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-linolensäure, Arachidonsäure, α-Linolensäure, Stearidonsäure, Eicosatetraensäure, Eicosapentaensäure, Docosapentaensäure oder Docosahexaensäure aufweist. Vorzugsweise beträgt der Anteil an ungesättigten veresterten Fettsäuren ungefähr 30 %, besonders bevorzugt ist ein Anteil von 50 %, am meisten bevorzugt ist ein Anteil von 60 %, 70 %, 80 % oder mehr. Der Anteil an einer Fettsäure kann z.B. nach Überführung der Fettsäuren in die Methylester durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen handelt es sich beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den im erfindungsgemäßen Verfahren hergestellten mehrfach ungesättigten Fettsäuren mit vorteilhaft mindestens zwei Doppelbindungen lassen sich die enthaltenen mehrfach ungesättigten Fettsäuren beispielsweise über eine Alkalibehandlung, beispielsweise mit wässriger KOH oder NaOH, oder saure Hydrolyse vorteilhaft in Gegenwart eines Alkohols wie Methanol oder Ethanol oder über eine enzymatische Abspaltung freisetzen und über beispielsweise Phasentrennung und anschließende Ansäuerung mit z.B. H₂SO₄ isolieren. Die Freisetzung der Fettsäuren kann auch direkt ohne die vorhergehend beschriebene Aufarbeitung erfolgen.

Die im Verfahren verwendeten Nukleinsäuren können nach Einbringung in einen Organismus, vorteilhaft eine Pflanzenzelle bzw. Pflanze, entweder auf einem separaten Plasmid liegen oder in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen, vorteilhaft zur multiparallelen samenspezifischen Expression von Genen in die Pflanzen, gebracht.

Moose und Algen sind die einzigen bekannten Pflanzensysteme, die erhebliche Mengen an mehrfach ungesättigten Fettsäuren wie Arachidonsäure (ARA) und/oder Eicosapentaensäure (EPA) und/oder Docosahexaensäure (DHA) herstellen. Moose enthalten PUFAs in Membranlipiden, während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Als Substrate der erfindungsgemäßen Acyl-CoA:Lysophospholipid-Acyltransferasen werden vorteilhaft C₁₆-, C₁₈-, C₂₀- oder C₂₂-Fettsäuren verwendet.

Zur Herstellung der erfindungsgemäßen langkettiger PUFAs müssen die mehrfach ungesättigten C₁₆- oder C₁₈-Fettsäuren zunächst durch die enzymatische Aktivität einer Desaturase desaturiert und anschließend über eine Elongase um mindestens zwei Kohlenstoffatome verlängert werden. Nach einer Elongationsrunde führt diese Enzymaktivität zu C₁₈- oder C₂₀-Fettsäuren, und nach zwei oder drei Elongationsrunden zu C₂₂- oder C₂₄-Fettsäuren. Die Aktivität der im erfindungsgemäßen Verfahren verwendeten Desaturasen und Elongasen führt vorzugsweise zu C₁₈-, C₂₀- und/oder C₂₂-Fettsäuren, vorteilhaft mit mindestens zwei Doppelbindungen im Fettsäuremolekül, vorzugsweise mit drei, vier oder fünf Doppelbindungen, besonders bevorzugt zu C₂₀- und/oder C₂₂-Fettsäuren mit mindestens zwei Doppelbindungen im Fettsäuremolekül, vorzugsweise mit drei, vier oder fünf Doppelbindungen im Molekül. Nachdem eine erste Desaturierung und die Verlängerung stattgefunden hat, können weitere Desaturierungsschritte wie z.B. eine solche in Δ-5-Position erfolgen. Besonders bevorzugt als Produkte des erfindungsgemäßen Verfahrens sind Dihomo-γ-linolensäure, Arachidonsäure, Eicosapentaensäure, Docosapentaensäure und/oder Docosahexaensäure. Die C₁₈-Fettsäuren mit mindestens zwei Doppelbindungen in der Fettsäure können durch die erfindungsgemäße enzymatische Aktivität in Form der freien Fettsäure oder in Form der Ester, wie Phospholipiden, Glykolipiden, Sphingolipiden, Phosphoglyceriden, Monoacylglycerin, Diacylglycerin oder Triacylglycerin, verlängert werden.

Der bevorzugte Biosyntheseort von Fettsäuren, Ölen, Lipiden oder Fetten in den vorteilhaft verwendeten Pflanzen ist beispielsweise der Samen im Allgemeinen oder Zellschichten des Samens, so dass eine samenspezifische Expression der im Verfahren verwendeten Nukleinsäuren sinnvoll ist. Es ist jedoch nahe liegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Durch die Verwendung der erfindungsgemäßen Nukleinsäuren, die für Acyl-CoA:Lysophospholipid-Acyltransferasen kodieren, kann im Verfahren die Menge der hergestellten mehrfach ungesättigten Fettsäuren mindestens um 10 % , bevorzugt mindestens um 15 %, besonders bevorzugt mindestens um 20 %, ganz besonders bevorzugt um mindestens 50 % und am meisten bevorzugt um mindestens 100% gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Organismen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Werden im erfindungsgemäßen Verfahren als Organismen Mikroorganismen wie Hefen wie *Saccharomyces* oder *Schizosaccharomyces,* Pilze wie *Mortierella, Aspergillus, Phytophtora, Entomophthora, Mucor* oder *Traustochytrium,* Algen wie *Isochrysis, Phaeodactylum* oder *Crypthecodinium* verwendet, so werden diese Organismen vorteilhaft fermentativ in einer dem Fachmann bekannten Weise angezogen bzw. gezüchtet.

Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH-Wert der Nährflüssigkeit auf einem festen Wert gehalten werden, das heißt während der Anzucht reguliert werden, oder nicht. Die Anzucht kann batchweise, semi-batchweise oder kontinuierlich erfolgen. Nährstoffe können zu Beginn der Fermentation vorgelegt oder semikontinuierlich oder kontinuierlich nachgefüttert werden. Die hergestellten mehrfach ungesättigten Fettsäuren können nach dem Fachmann bekannten Verfahren, beispielsweise über Extraktion, Destillation, Kristallisation, ggf. Salzfällung und/oder Chromatographie wie oben beschrieben aus den Organismen isoliert werden. Die Organismen können dazu vorher noch vorteilhaft aufgeschlossen werden.

Das erfindungsgemäße Verfahren wird, wenn es sich bei den Wirtsorganismen um Mikroorganismen handelt, vorteilhaft bei einer Temperatur zwischen 0 °C bis 95 °C, bevorzugt zwischen 10 °C bis 85 °C, besonders bevorzugt zwischen 15 °C bis 75 °C, und am meisten bevorzugt zwischen 15 °C bis 45 °C durchgeführt

Der pH-Wert wird dabei vorteilhaft zwischen pH 4 und 12, bevorzugt zwischen pH 6 und 9, besonders bevorzugt zwischen pH 7 und 8 gehalten.

Das erfindungsgemäße Verfahren kann batchweise, semi-batchweise oder kontinuierlich betrieben werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galaktose, Ribose, Sorbose, Ribulose, Lactose, Maltose, Saccharose, Raffinose, Stärke oder Zellulose. Man kann Zucker auch über komplexe Verbindungen, wie Melassen oder andere Nebenprodukte der Zucker-Raffinierung, zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl, Sonnenblumenöl, Erdnussöl und/oder Kokosfett, Fettsäuren wie z. B. Palmitinsäure, Stearinsäure und/oder Linolsäure, Alkohole und/oder Polyalkohole wie z. B. Glycerin, Methanol und/oder Ethanol und/oder organische Säuren wie z. B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger Form oder Gasform oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphat- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-Salze verwendet werden.

Dem Medium können Chelatbildner zugegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß zur Kultivierung von Mikroorganismen eingesetzten Fermentationsmedien enthalten üblicherweise auch andere Wachstumsfaktoren wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig aus komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Informationen über die Medienoptimierung sind erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden entweder durch Hitze (20 min bei 1,5 bar und 121°C) oder durch Sterilfiltration sterilisiert. Die Komponenten können entweder zusammen oder nötigenfalls getrennt sterilisiert werden. Sämtliche Medienkomponenten können zu Beginn der Anzucht zugegen sein oder wahlweise kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere mehrfach ungesättigte Fettsäuren enthaltenden, Fermentationsbrühen haben üblicherweise eine Trockenmasse von 7,5 bis 25 Gew.-%.

Die Fermentationsbrühe kann anschließend weiterverarbeitet werden. Je nach Anforderung kann die Biomasse ganz oder teilweise durch Separationsmethoden, wie z. B. Zentrifugation, Filtration, Dekantieren oder eine Kombination dieser Methoden aus der Fermentationsbrühe entfernt oder vollständig in ihr belassen werden. Vorteilhaft wird die Biomasse nach Abtrennung aufgearbeitet.

Die Fermentationsbrühe kann aber auch ohne Zellabtrennung mit bekannten Methoden, wie z. B. mit Hilfe eines Rotationsverdampfers, Dünnschichtverdampfers, Fallfilmverdampfers, durch Umkehrosmose oder durch Nanofiltration eingedickt beziehungsweise aufkonzentriert werden. Diese aufkonzentrierte Fermentationsbrühe kann schließlich zur Gewinnung der darin enthaltenen Fettsäuren aufgearbeitet werden.

Die Begriffe Produktion oder Produktivität sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohlenstoff-Ausbeute ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle dieser Verbindung in einer bestimmten Kulturmenge über einen festgelegten Zeitraum erhöht. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmazeutika, Nahrungsmitteln, Tierfutter oder Kosmetika verwendet werden.

Ein weiterer erfindungsgemäßer Gegenstand sind isolierte Nukleinsäuresequenzen, die für Polypeptide mit Acyl-CoA:Lysophospholipid-Acyltransferaseaktivität kodieren, wobei die durch die Nukleinsäuresequenz kodierte Acyl-COA: Lysophospholipid-Acyltransferase spezifisch C₁₆-, C₁₈-, C₂₀- oder C₂₂-Fettsäuren mit mindestens einer Doppelbindung im Fettsäuremolekül umsetzen, wobei die Nukleinsäuresequenz ausgewählt aus der Gruppe bestehend aus
a) einer Nukleinsäuresequenz mit der in SEQ ID NO: 3 dargestellten Sequenz,
b) Nukleinsäuresequenzen, die sich als Ergebnis des degenerierten genetischen Codes von der in SEQ ID NO: 3 enthaltenen kodierenden Sequenz ableiten lassen, und
c) Derivaten der in SEQ ID NO: 3 oder dargestellten Nukleinsäuresequenz, die für Polypeptide mit der in SEQ ID NO: 4 dargestellten Aminosäuresequenz kodieren und mindestens 70% Identität auf Aminosäureebene mit SEQ ID NO: 4 zeigen und eine Acyl-CoA:Lysophospholipid-Acyltransferaseaktivität aufweisen.

Vorteilhaft stammen die oben genannten Nukleinsäuresequenzen aus einem eukaryontischen Organismus, besonders bevorzugt aus *Ostreococcus tauri* oder *Mantoniella squamata.*

Die im Verfahren verwendeten Nukleinsäuresequenzen, die für Proteine mit Acyl-CoA:Lysophospholipid-Acyltransferase-Aktivität oder für Proteine des Fettsäure-oder Lipidstoffwechsels kodieren, werden vorteilhaft in einer Expressionskassette (= Nukleinsäurekonstrukt), die die Expression der Nukleinsäuren in einem Organismus, vorteilhaft einer Pflanze oder einem Mikroorganismus, ermöglicht, eingebracht.

Zum Einbringen werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten und die gleichzeitig die stabile Transformation von Pflanzen ermöglichen. Zu nennen sind insbesondere verschiedene für die T-DNAvermittelte Transformation geeignete, binäre und co-integrierte Vektorsysteme. Derartige Vektorsysteme sind in der Regel **dadurch gekennzeichnet, dass** sie zumindest die für die Agrobakterium-vermittelte Transformation benötigten vir-Gene sowie die T-DNA-begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in *E. coli* als auch in *Agrobacterium* zu replizieren. Zu diesen binären Vektoren gehören Vektoren der Serien pBIB-HYG, pPZP, pBecks, pGreen. Erfindungsgemäß bevorzugt verwendet werden pBin19, pBI101, pBinAR, pGPTV und pCAMBIA. Eine Übersicht über binäre Vektoren und ihre Verwendung gibt Hellens et al, Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittene und erforderlichenfalls gereinigte Amplifikat mit ähnlich präparierten Vektorfragmenten unter Einsatz von Ligase kloniert. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere *Escherichia coli* und *Agrobacterium tumefaciens,* unter selektiven Bedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren, die erfinderischen Nukleinsäuren und Nukleinsäurekonstrukte in Organismen wie Mikroorganismen oder vorteilhaft Pflanzen eingebracht werden und damit bei der Pflanzentransformation verwendet werden, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus (1991) Annu. Rev. Plant Physiol. Plant Molec. Biol. 42: 205-225). Die im Verfahren verwendeten Nukleinsäuren, die erfinderischen Nukleinsäuren und Nukleinsäurekonstrukte und/oder Vektoren lassen sich damit zur gentechnologischen Veränderung eines breiten Spektrums an Organismen, vorteilhaft an Pflanzen, verwenden, so dass diese bessere und/oder effizientere Produzenten von PUFAs werden.

Es gibt eine Reihe von Mechanismen, durch die die Veränderung eines erfindungsgemäßen Acyl-CoA:Lysophospholipid-Acyltransferase-Proteins die Ausbeute, Produktion und/oder Effizienz der Produktion einer Feinchemikalie in einer Ölfruchtpflanze oder einem Mikroorganismus direkt beeinflussen kann. Die Anzahl oder Aktivität des Acyl-CoA:Lysophospholipid-Acyltransferase-Proteins oder -Gens sowie von Genkombinationen von Acyl-CoA:Lysophospholipid-Acyltransferasen, Desaturasen und/oder Elongasen kann erhöht sein, so dass größere Mengen der produzierten Verbindungen de novo hergestellt werden, weil den Organismen diese Aktivität und Fähigkeit zur Biosynthese vor dem Einbringen des/der entsprechenden Gens/Gene fehlte. Entsprechendes gilt für die Kombination mit weiteren Desaturasen oder Elongasen oder weiteren Enzymen aus dem Fettsäure-und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglichen.

Durch das Einbringen eines Acyl-CoA:Lysophospholipid-Acyltransferase-, Desaturase- und/oder Elongase-Gens oder mehrerer Acyl-CoA:Lysophospholipid-Acyltransferase-, Desaturase- und/oder Elongase-Gene allein oder in Kombination mit anderen Genen in einen Organismus bzw. eine Zelle kann nicht nur der Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Feinchemikalien (z.B. Fettsäuren, polaren und neutralen Lipiden) benötigt werden, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Fettsäuren und Lipide sind selbst als Feinchemikalien wünschenswert; durch Optimierung der Aktivität oder Erhöhung der Anzahl einer oder mehrerer Acyl-CoA:Lysophospholipid-Acyltransferasen, Desaturasen und/oder Elongasen, die an der Biosynthese dieser Verbindungen beteiligt sind, oder durch Zerstören der Aktivität einer oder mehrerer Desaturasen, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten isolierten Nukleinsäuremoleküle kodieren für Proteine oder Teile von diesen, wobei die Proteine oder das einzelne Protein oder Teile davon eine Aminosäuresequenz enthalten, die ausreichend homolog zu einer Aminosäuresequenz der Sequenz SEQ ID NO: 4 ist, so dass das Protein oder der Teil davon eine Acyl-CoA:Lysophospholipid-Acyltransferase-Aktivität beibehält. Vorzugsweise hat das Protein oder der Teil davon, das/der von dem Nukleinsäuremolekül kodiert wird, noch seine wesentliche enzymatische Aktivität und besitzt die Fähigkeit, am Stoffwechsel von zum Aufbau von Zellmembranen oder Lipidkörperchen in Organismen, vorteilhaft in Pflanzen, notwendigen Verbindungen oder am Transport von Molekülen über diese Membranen teilzunehmen. Das von den Nukleinsäuremolekülen kodierte Protein ist zu mindestens 70 %, 80 % oder 90 % und am meisten bevorzugt zu mindestens etwa 95 %, 96 %, 97 %, 98 %, 99 % oder mehr Identisch zu einer Aminosäuresequenz der Sequenz SEQ ID NO: 4. Im Sinne der Erfindung ist unter Homologie oder homolog Identität oder identisch zu verstehen.

Unter wesentlicher enzymatischer Aktivität der verwendeten Acyl-CoA:Lysophospholipid-Acyltransferasen ist zu verstehen, dass sie gegenüber den durch die Sequenz mit SEQ ID NO: 3 und deren Derivate kodierten Proteinen/Enzymen im Vergleich noch eine enzymatische Aktivität von mindestens 10 %, bevorzugt mindestens 20 %, besonders bevorzugt mindestens 30 % und ganz besonders bevorzugt mindestens 40 % aufweisen und damit am Stoffwechsel von zum Aufbau von Fettsäuren in einem Organismus, vorteilhaft einer Pflanzenzelle, notwendigen Verbindungen oder am Transport von Molekülen über Membranen teilnehmen können, wobei desaturierte C16-, C18-, C20- oder C24-Kohlenstoffketten mit Doppelbindungen an mindestens zwei, vorteilhaft drei, vier oder fünf Stellen gemeint sind.

Vorteilhaft im Verfahren verwendbare Nukleinsäuren stammen aus Pilzen oder Pflanzen wie Algen oder Moosen wie den Gattungen *Physcomitrella, Thraustochytrium, Phytophtora, Ceratodon, Isochrysis, Aleurita, Muscarioides, Mortierella, Borago, Phaeodactylum, Crypthecodinium* oder aus Nematoden wie *Caenorhabditis,* speziell aus den Gattungen und Arten *Physcomitrella patens, Phytophtora infestans, Ceratodon purpureus, Isochrysis galbana, Aleurita farinosa, Muscarioides viallii, Mortierella alpina, Borago officinalis, Phaeodactylum tricornutum* oder besonders vorteilhaft aus *Ostreococcus tauri* oder *Mantoniella squamata.*

Alternativ können die hier beschriebenen isolierten Nukleotidsequenzen für Acyl-CoA:Lysophospholipid-Acyltransferasen kodieren, die an eine Nukleotidsequenz der SEQ ID NO: 1, SEQ ID NO: 3 oder SEQ ID NO: 5 hybridisieren, z.B. unter stringenten Bedingungen.

Die im Verfahren verwendeten Nukleinsäuresequenzen werden vorteilhaft in einer Expressionskassette, die die Expression der Nukleinsäuren in Organismen wie Mikroorganismen oder Pflanzen ermöglicht, in den jeweiligen Organismus eingebracht.

Dabei werden die für die erfinderischen Acyl-CoA:Lysophospholipid-Acyltransferasen, die verwendeten Desaturasen und/oder die Elongasen kodierenden Nukleinsäuresequenzen mit einem oder mehreren Regulationssignalen vorteilhafterweise zur Erhöhung der Genexpression funktionell verknüpft. Diese regulatorischen Sequenzen sollen die gezielte Expression der Gene und der Proteine ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen, an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt) kann aber auch einfacher aufgebaut sein, das heißt, es wurden keine zusätzlichen Regulationssignale vor die Nukleinsäuresequenz oder deren Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "Enhancer-Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Acyl-CoA:Lysophospholipid-Acyltransferase-Gene sowie die vorteilhaft verwendeten Δ-4-Desaturase-, Δ5-Desaturase-, Δ-6-Desaturase- und/oder Δ-8-Desaturase-Gene und/oder die Δ-5-Elongasen-, Δ-6-Elongasen- und/oder Δ-9-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte kann/können zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene in das Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Expression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Eine weitere Ausführungsform der Erfindung sind ein oder mehrere Genkonstrukte, die eine oder mehrere Sequenzen enthalten, die durch SEQ ID NO: 3 oder deren Derivate definiert sind und für Polypeptide gemäß SEQ ID NO: 4 kodieren. Die genannten Acyl-CoA:Lysophospholipid-Acyltransferasen führen dabei zu einem Austausch der Fettsäuren zwischen dem Mono-, Di- und/oder Triglyceridpool der Zelle und dem CoA-Fettsäureester-Pool, wobei das Substrat vorteilhaft ein, zwei, drei, vier oder fünf Doppelbindungen und vorteilhaft 16, 18, 20, 22 oder 24 Kohlenstoffatome im Fettsäuremolekül aufweist. Gleiches gilt für ihre Homologen, Derivate oder Analoga, die funktionsfähig mit einem oder mehreren Regulationssignalen, vorteilhafterweise zur Steigerung der Genexpression, verbunden sind.

Vorteilhafte Regulationssequenzen für das neue Verfahren liegen beispielsweise in Promotoren wie dem cos-, tac-, trp-, tet-, trp-tet-, lpp-, lac-, Ipp-lac-, lacIq-, T7-, T5-, T3-, gal-, trc-, ara-, SP6-, λ-PR- oder λ-PL-Promotor vor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S (Franck et al. (1980) Cell 21: 285-294), PRP1 (Ward et al. (1993) Plant. Mol. Biol. 22), SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Gatz et al. (1992) Plant J. 2: 397-404 (Tetracyclin-induzierbar), EP-A-0 335 528 (Abszisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al. (1989) EMBO J. 8: 2445), der Phosphoribosylpyrophosphatamidotransferase-Promotor aus *Glycine max* (Genbank-Zugangs-nr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren wie der ausführungsgemäße USP-Promotor, aber auch andere Promotoren wie der LeB4-, DC3-, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), Baeumlein et al. (1992) Plant J. 2 (2): 233-239 (LeB4-Promotor aus einer Leguminose) beschrieben sind, wobei sich diese Promotoren für Dikotyledonen eignen. Die folgenden Promotoren eignen sich beispielsweise für Monokotyledonen: lpt-2- oder lpt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden bzw. Promotoren, die im Embryo und/oder im Endosperm aktiv sind. Samen-spezifische Promotoren können prinzipiell sowohl aus dikotyledonen als auch aus monokotyledonen Pflanzen isoliert werden. Im Folgenden sind vorteilhafte bevorzugte Promotoren aufgeführt: USP (= unknown seed protein) und Vicilin (*Vicia faba*) (Bäumlein et al. (1991) Mol. Gen Genet. 225(3)), Napin (Raps) (US 5,608,152), Acyl-Carrier Protein (Raps) (US 5,315,001 und WO 92/18634), Oleosin (*Arabidopsis thaliana*) (WO 98/45461 und WO 93/20216), Phaseolin (*Phaseolus vulgaris*) (US 5,504,200), Bce4 (WO 91/13980), Leguminosen B4 (LegB4-Promotor) (Bäumlein et al. (1992) Plant J. 2 (2): 233-9), Lpt2 und lpt1(Gerste) (WO 95/15389 und WO95/23230), Samen-spezifische Promotoren aus Reis, Mais u. Weizen (WO 99/16890), Amy32b, Amy 6-6 und Aleurain (US 5,677,474), Bce4 (Raps) (US 5,530,149), Glycinin (Soja) (EP 571 741), Phosphoenol-Pyruvatcarboxylase (Soja) (JP 06/62870), ADR12-2 (Soja) (WO 98/08962), Isocitratlyase (Raps) (US 5,689,040) oder α-Amylase (Gerste) (EP 781 849).

Die Pflanzengenexpression lässt sich auch über einen chemisch induzierbaren Promotor erreichen (siehe eine Übersicht in Gatz (1997) Annu. Rev. Plant Physiol. Plant Mol. Biol. 48: 89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2: 397-404) und ein Ethanol-induzierbarer Promotor.

Um eine stabile Integration der Biosynthesegene in die transgene Pflanze über mehrere Generationen sicherzustellen, sollte jede der im Verfahren verwendeten Nukleinsäuren, die für die Acyl-CoA:Lysophospholipid-Acyltransferase, die vorteilhafte Δ-4-Desaturase, die Δ-5-Desaturase, die Δ-6-Desaturase, die Δ-8-Desaturase und/oder die Δ-5-Elongase, die Δ-6-Elongase und/oder die Δ-9-Elongase kodieren, unter der Kontrolle eines eigenen, bevorzugt eines unterschiedlichen Promotors exprimiert werden, da sich wiederholende Sequenzmotive zur Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt, die vorteilhaft in einem Polylinker liegt und der gegebenenfalls ein Terminator folgt. Diese Abfolge wiederholt sich mehrfach, bevorzugt drei-, vier- oder fünfmal, so dass bis zu fünf Gene in einem Konstrukt zusammengeführt und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Es ist aber auch möglich, mehrere Nukleinsäuresequenzen hinter einen Promotor und ggf. vor einen Terminator zu inserieren. Dabei ist die Insertionsstelle bzw. die Abfolge der inserierten Nukleinsäuren in der Expressionskassette nicht von entscheidender Bedeutung, das heißt, eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4- oder DC3-Promotor und unterschiedliche Terminatoren verwendet werden. Es ist aber auch möglich, nur einen Promotortyp in der Kassette zu verwenden. Dies kann jedoch zu unerwünschten Rekombinationsereignissen führen.

Wie oben beschrieben sollte die Transkription der eingebrachten Gene vorteilhaft durch geeignete Terminatoren am 3'-Ende der eingebrachten Biosynthesegene (hinter dem Stopcodon) abgebrochen werden. Verwendet werden kann hier z.B. der OCS1-Terminator. Wie auch für die Promotoren, so sollten für jedes Gen auch unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsels enthalten sein oder aber diese Gene können auf einem weiteren oder mehreren weiteren Nukleinsäurekonstrukten liegen. Vorteilhaft wird als Biosynthesegen des Fettsäure- oder Lipidstoffwechsels ein Gen ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-Acyl-Transferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) oder eine Kombination dieser Gene verwendet. Besonders vorteilhafte Nukleinsäuresequenzen sind Biosynthesegene des Fettsäure- oder Lipidstoffwechsels ausgewählt aus der Gruppe der Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongasegene.

Dabei können die vorgenannten Desaturasen in Kombination mit anderen Elongasen und Desaturasen in erfindungsgemäße Expressionskassetten kloniert und zur Transformation von Pflanzen mit Hilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vörteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektor eingebracht werden.

Diese vorteilhaften Vektoren, vorzugsweise Expressionsvektoren, enthalten die im Verfahren verwendeten Nukleinsäuren, die für Acyl-CoA:Lysophospholipid-Acyltransferasen kodieren, oder ein Nukleinsäurekonstrukt, das die verwendete Nukleinsäure allein oder in Kombination mit weiteren Biosynthesegenen des Fettsäure- oder Lipidstoffwechsels wie Δ-4-Desaturase-, Δ-5-Desaturase-, Δ-6-Desaturase-, Δ-8-Desaturase-, Δ-9-Desaturase-, Δ-12-Desaturase-, Δ-5-Elongase-, Δ-6-Elongase- und/oder Δ-9-Elongasegene enthält. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch auch andere Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die unten beschriebenen Nukleinsäuren oder das oben beschriebene Genkonstrukt in einer Form, die sich zur Expression der verwendeten Nukleinsäuren in einer Wirtszelle eignet, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfassen. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie so aneinander gebunden sind, dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins, usw. abhängen kann.

Die verwendeten rekombinanten Expressionsvektoren können zur Expression von Acyl-CoA:Lysophospholipid-Acyltransferasen, Desaturasen und Elongasen in prokaryotischen oder eukaryotischen Zellen gestaltet sein. Dies ist vorteilhaft, da häufig Zwischenschritte der Vektorkonstruktion der Einfachheit halber in Mikroorganismen durchgeführt werden. Beispielsweise können Acyl-CoA:Lysophospholipid-Acyltransferase-, Desaturase- und/oder Elongase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: *Holotrichia, Peritrichia*, *Spirotrichia, Suctoria, Tetrahymena, Paramecium, Colpidium*, *Glaucoma, Platyophrya, Potomacus, Cohnilembus, Euplotes, Engelmaniella* und *Stylonychia,* insbesondere der Art *Stylonychia lemnae,* mit Vektoren nach einem Transformationsverfahren, wie beschrieben in WO 98/01572, sowie bevorzugt in Zellen vielzelliger Pflanzen (siehe Schmidt, R. und Willmitzer, L. (1988) Plant Cell Rep. 583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus (1991) Annu. Rev. Plant Physiol. Plant Molec. Biol. 42: 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-*E*. *coli*-Expressionsvektoren sind u.a. pTrc (Amann et al. (1988) Gene 69: 301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer koexprimierten viralen RNA-Polymerase (T7 gn1) vermittelt wird. Diese virale Polymerase wird in den Wirtsstämmen BL21 (DE3) oder HMS 174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in *E. coli* pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCI, in *Streptomyces* pIJ101, pIJ364, pIJ702 oder pIJ361, in *Bacillus* pUB110, pC194 oder pBD214, in *Corynebacterium* pSA77 oder pAJ667.

Bei einer weiteren Ausführungsform ist der Expressionsvektor ein Hefe-Expressionsvektor. Beispiele für Vektoren zur Expression in der Hefe *S. cerevisiae* umfassen pYeDesaturasec1 (Baldari et al. (1987) Embo J. 6: 229-234), pMFa (Kurjan und Herskowitz (1982) Cell 30: 933-943), pJRY88 (Schultz et al. (1987) Gene 54: 113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi (J.W. Bennet & L.L. Lasure, Hrsgb., S. 396-428: Academic Press: San Diego). Weitere geeignete Hefevektoren sind beispielsweise pAG-1, YEp6, YEp13 oder pEMBLYe23.

Alternativ können die Acyl-CoA:Lysophospholipid-Acyltransferasen, Desaturasen und/oder Elongasen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol. 3: 2156-2165) und die pVL-Reihe (Lucklow und Summers (1989) Virology 170: 31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook und Russell, Molecular Cloning: A Laboratory Manual, 3. Auflage, Cold Spring Harbor Laboratory, Cold Spring Harbor Laboratory Press, Cold Spring Harbor, NY, 2001.

Bei einer weiteren Ausführungsform des Verfahrens können die Acyl-CoA:Lysophospholipid-Acyltransferasen, Desaturasen und/oder Elongasen in einzelligen Pflanzenzellen (wie Algen), siehe Falciatore et al., 1999, Marine Biotechnology 1 (3): 239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) Plant Mol. Biol. 20: 1195-1197; und Bevan, M.W. (1984) Nucl. Acids Res. 12: 8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus *Agrobacterium tumefaciens-T-*DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebene beschränkt ist, enthält eine Pflanzen-Expressionskassette vorzugsweise andere funktionsfähig verbundene Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al. (1987) Nucl. Acids Research 15: 8693-8711).

Das zu exprimierende Gen muss wie oben beschrieben funktionsfähig mit einem geeigneten Promotor verbunden sein, der die Genexpression auf rechtzeitige, zell-oder gewebespezifische Weise durchführt. Nutzbare Promotoren sind konstitutive Promotoren (Benfey et al. (1989) EMBO J. 8: 2195-2202), wie diejenigen, die von Pflanzenviren stammen, wie 35S CAMV (Franck et al. (1980) Cell 21: 285-294), 19S CaMV (siehe auch US 5,352,605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment, beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen, notwendig sind (siehe eine Übersicht in Kermode (1996) Crit. Rev. Plant Sci. 15, 4: 285-423 und darin zitierte Literaturstellen).

Die Pflanzengenexpression lässt sich auch wie oben beschrieben über einen chemisch induzierbaren Promotor erleichtern (siehe eine Übersicht in Gatz (1997) Annu. Rev. Plant Physiol. Plant Mol. Biol. 48: 89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2: 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al. (1993) Plant. Mol. Biol. 22: 361-366), der hitzeinduzierbare hsp80-Promotor aus Tomate (US 5,187,267), der kälteinduzierbare Alphaamylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen wie den Zellen des Endosperms und des sich entwickelnden Embryos. Geeignete Promotoren sind der Napingen-Promotor aus Raps (US 5,608,152), der USP-Promotor aus *Vicia faba* (Baeumlein et al. (1991) Mol Gen Genet. 225 (3):459-67), der Oleosin-Promotor aus *Arabidopsis* (WO 98/45461), der Phaseolin-Promotor aus *Phaseolus vulgaris* (US 5,504,200), der Bce4-Promotor aus Brassica (WO 91/13980) oder der Legumin-B4-Promotor (LeB4; Baeumlein et al. (1992) Plant Journal 2 (2): 233-9) sowie Promotoren, welche die samenspezifische Expression in monokotyledonen Pflanzen wie Mais, Gerste, Weizen, Roggen, Reis, usw. herbeiführen. Geeignete Promotoren sind der lpt2- oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, dem Weizen-Glutelin-Gen, dem Mais-Zein-Gen, dem Hafer-Glutelin-Gen, dem Sorghum-Kasirin-Gen, dem Roggen-Secalin-Gen).

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren sind der virale RNA-Polymerase-Promotor, beschrieben in WO 95/16783 und WO 97/06250, und der clpP-Promotor aus Arabidopsis, der in WO 99/46394 beschrieben ist.

Insbesondere kann die multiparallele Expression der im Verfahren verwendeten Acyl-CoA:Lysophospholipid-Acyltransferasen allein oder in Kombination mit Desaturasen und/oder Elongasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren, die jeweils mehrere Expressionskassetten enthalten, transformiert und auf die Wirtszelle übertragen werden.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäuren (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook und Russell (Molecular Cloning: A Laboratory Manual., 3. Aufl., Cold Spring Harbor Laboratory, Cold Spring Harbor Laboratory Press, Cold Spring Harbor, NY, 2001) und anderen Labor-Handbüchern, wie Methods in Molecular Biology, 1995, Bd. 44, Agrobacterium protocols, Hrsgb: Gartland und Davey, Humana Press, Totowa, New Jersey.

Wirtszellen, die im Prinzip zum Aufnehmen der erfindungsgemäßen Nukleinsäure, des erfindungsgemäßen Genproduktes oder des erfindungsgemäßen Vektors geeignet sind, sind alle prokaryotischen oder eukaryotischen Organismen. Die vorteilhafterweise verwendeten Wirtsorganismen sind Mikroorganismen, wie Pilze oder Hefen oder Pflanzenzellen, vorzugsweise Pflanzen oder Teile davon. Pilze, Hefen oder Pflanzen werden vorzugsweise verwendet, besonders bevorzugt Pflanzen, ganz besonders bevorzugt Pflanzen, wie Ölfruchtpflanzen, die große Mengen an Lipidverbindungen enthalten, wie Raps, Nachtkerze, Hanf, Distel, Erdnuss, Canola, Lein, Soja, Safflor, Sonnenblume, Borretsch, oder Pflanzen wie Mais, Weizen, Roggen, Hafer, Triticale, Reis, Gerste, Baumwolle, Maniok, Pfeffer, Tagetes, Solanaceen-Pflanzen wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Besonders bevorzugte erfindungsgemäße Pflanzen sind Ölfruchtpflanzen, wie Soja, Erdnuß, Raps, Canola, Lein, Hanf, Nachtkerze, Sonnenblume, Safflor, Bäume (Ölpalme, Kokosnuss).

Ein weiterer erfindungsgemäßer Gegenstand sind wie oben beschrieben isolierte Nukleinsäuresequenzen, die für Polypeptide mit Acyl-CoA:Lysophospholipid-Acyltransferaseaktivität kodieren, wobei die durch die Nukleinsäuresequenzen kodierten Acyl-CoA:Lysophospholipid-Acyltransferasen spezifisch C₁₆-, C₁₈-, C₂₀-oder C₂₂-Fettsäuren mit mindestens einer Doppelbindungen im Fettsäuremolekül umsetzen.

Isolierte Nukleinsäuresequenzen sind Sequenzen ausgewählt aus der Gruppe bestehend aus:
d) einer Nukleinsäuresequenz mit der in SEQ ID NO: 3 dargestellten Sequenz,
e) Nukleinsäuresequenzen, die sich als Ergebnis des degenerierten genetischen Codes von der in SEQ ID NO: 3 enthaltenen kodierenden Sequenz ableiten lassen
f) Derivaten der in SEQ ID NO: 3 dargestellten Nukleinsäuresequenz, die für Polypeptide mit der in SEQ ID NO: 4 dargestellten Aminosäuresequenz kodieren und mindestens 70% Identität auf Aminosäureebene mit der in SEQ ID NO: 4 dargestellten Aminosäuresequenz zeigen und eine Acyl-CoA:Lysophospholipid-Acyltransferaseaktivität aufweisen.

Die oben genannten erfindungsgemäßen Nukleinsäuresequenzen stammen aus Organismen, wie Tieren, Ciliaten, Pilzen, Pflanzen wie Algen oder Dinoflagellaten, die PUFAs synthetisieren können. Bevorzugt stammen die erfindungsgemäßen Nukleinsäuresequenzen aus *Ostreococcus tauri* oder *Mantonella squamata.*

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Acyl-CoA:Lysophospholipid-Acyltransferasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt, flankieren.

Die im Verfahren verwendeten Nukleinsäuremoleküle, z.B. ein Nukleinsäuremolekül mit einer Nukleotidsequenz der SEQ ID NO: 3 oder eines Teils davon, kann unter Verwendung molekularbiologischer Standardtechniken und der hier bereitgestellten Sequenzinformation isoliert werden. Auch kann mit Hilfe von Vergleichsalgorithmen beispielsweise eine homologe Sequenz oder homologe, konservierte Sequenzbereiche auf DNA- oder Aminosäureebene identifiziert werden. Diese können als Hybridisierungssonde in Standard-Hybridisierungstechniken (wie z.B. beschrieben in Sambrook und Russell, Molecular Cloning: A Laboratory Manual. 3. Aufl., Cold Spring Harbor Laboratory, Cold Spring Harbor Laboratory Press, Cold Spring Harbor, NY, 2001) zur Isolierung weiterer im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 3 oder SEQ ID NO: 5 oder einen Teil davon, durch Polymerasekettenreaktion isolieren, wobei Oligonukleotidprimer auf der Basis dieser Sequenz oder von Teilen davon verwendet werden (z.B. kann ein Nukleinsäuremolekül, umfassend die vollständige Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18: 5294-5299) und cDNA mittels Reverser Transkriptase (z.B. Moloney-MLV-Reverse-Transkriptase, erhältlich von GibcoBRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3 oder SEQ ID NO: 5 gezeigten Nukleinsäuresequenzen oder mit Hilfe der in SEQ ID NO: 2, SEQ ID NO: 4 oder SEQ ID NO: 6 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden.

Homologe der verwendeten Acyl-CoA:Lysophospholipid-Acyltransferase-Nukleinsäuresequenzen mit der Sequenz SEQ ID NO: 1, SEQ ID NO: 3 oder SEQ ID NO: 5 bedeutet beispielsweise allelische Varianten mit mindestens etwa 40 bis 60 %, vorzugsweise mindestens etwa 60 bis 70 %, stärker bevorzugt mindestens etwa 70 bis 80 %, 80 bis 90 % oder 90 bis 95 % und am meisten bevorzugt mindestens etwa 95 %, 96 %, 97 %, 98 %, 99 % oder mehr Homologie zu einer der in SEQ ID NO: 1, SEQ ID NO: 3 oder SEQ ID NO: 5 gezeigten Nukleotidsequenzen oder ihren Homologen, Derivaten oder Analoga oder Teilen davon. Weiterhin beschrieben sind isolierte Nukleinsäuremoleküle einer Nukleotidsequenz, die an eine der in SEQ ID NO: 1, SEQ ID NO: 3 oder SEQ ID NO: 5 gezeigten Nukleotidsequenzen oder einen Teil davon hybridisiert, z.B. unter stringenten Bedingungen. Allelische Varianten umfassen insbesondere funktionelle Varianten, die sich durch Deletion, Insertion oder Substitution von Nukleotiden aus/in der in SEQ ID NO: 1, SEQ ID NO: 3 oder SEQ ID NO: 5 dargestellten Sequenz erhalten lassen, wobei aber die Absicht ist, dass die Enzymaktivität der dadurch entstehenden Proteine im Wesentlichen beibehalten wird.

Homologe der SEQ ID NO: 1, SEQ ID NO: 3 oder SEQ ID NO: 5 bedeuten beispielsweise auch bakterielle, Pilz- und Pflanzenhomologe, verkürzte Sequenzen, einzelsträngige DNA oder RNA der kodierenden und nicht-kodierenden DNA-Sequenz.

Homologe der SEQ ID NO: 1, SEQ ID NO: 3 oder SEQ ID NO: 5 bedeuten auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuremoleküle, die für Proteine mit Acyl-CoA:Lysophospholipid-Acyltransferaseaktivität kodieren, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen vorteilhaft in Pflanzen wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekte Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat, da mehrfach ungesättigte Fettsäuren (= PUFAs) nicht nur in Triacylglycerin, sondern auch in Membranlipide eingebaut werden.

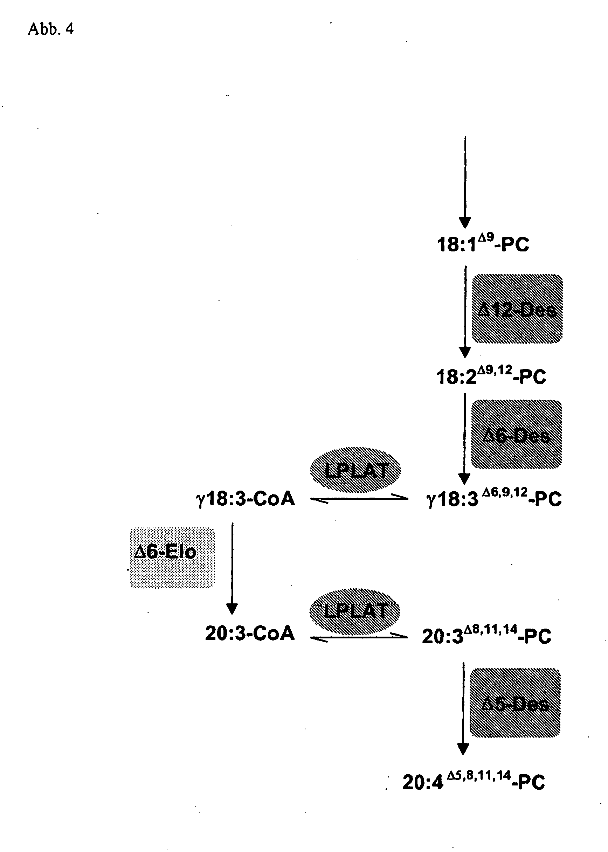

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glykolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten, an Phospholipide gebundenen Fettsäuren müssen anschließend wieder für weitere Elongationen aus den Phospholipiden in den FettsäureCoA-Ester-Pool überführt werden. Dies ermöglichen die erfindungsgemäßen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden (siehe Abb. 4).

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mit Hilfe der im Verfahren verwendeten Acyl-CoA:Lysophospholipid-Acyltransferasen, vorteilhaft in Kombination mit Desaturasen wie der Δ-4-, Δ-5-, Δ-6- und Δ-8-Desaturasen und/oder der Δ-5-, Δ-6-, Δ-9-Elongasen können Arachidonsäure, Eicosapentaensäure, Docosapentaensäure oder Docosahexaensäure sowie verschiedene andere langkettige PUFAs erhalten, extrahiert und für verschiedene Zwecke bei Nahrungsmittel-, Futter-, Kosmetik- oder pharmazeutischen Anwendungen verwendet werden. Mit den genannten Enzymen können vorzugsweise C₁₈-, C₂₀-, und/oder C₂₂-Fettsäuren mit mindestens zwei, vorteilhaft mindestens drei, vier, fünf oder sechs Doppelbindungen im Fettsäuremolekül, vorzugsweise zu C₂₀-, und/oder C₂₂-Fettsäuren mit vorteilhaft drei, vier oder fünf Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von C₂₀ zu C₂₂-Fettsäuren, zu Fettsäuren wie γ-Linolensäure, Dihomo-γ-linolensäure, Arachidonsäure, Stearidonsäure, Eicosatetraensäure oder Eicosapentaensäure. Substrate der Acyl-CoA:Lysophospholipid-Acyltransferasen im erfindungsgemäßen Verfahren sind C₁₆-, C₁₈-, C₂₀-oder C₂₂-Fettsäuren wie zum Beispiel Palmitinsäure, Palmitoleinsäure, Linolsäure, γ-Linolensäure, α-Linolensäure, Dihomo-γ-linolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die C₁₈-, C₂₀- oder C₂₂-Fettsäuren mit mindestens zwei Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin (Mono-, Di- oder Triglycerid) verstanden. Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglykolipide. Beispielhaft seien hier die Glycerophospholipide Lecithin (Phosphatidylcholin), Cardiolipin, Phosphatidylglycerin, Phosphatidylserin und Alkylacylglycerophospholipide genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen (1998) Lipid 100(4-5): 161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney (1997) Genetic Engeneering, Hrsgb.: JK Setlow, 19: 149-166; Ohlrogge und Browse (1995) Plant Cell 7: 957-970; Shanklin und Cahoon (1998) Annu. Rev. Plant Physiol. Plant Mol. Biol. 49: 611-641; Voelker (1996) Genetic Engeneering, Hrsgb.: JK Setlow, 18: 111-13; Gerhardt, (1992) Prog. Lipid R. 31: 397-417; Gühnemann-Schäfer & Kindl (1995) Biochim. Biophys Acta 1256: 181-186; Kunau et al. (1995) Prog. Lipid Res. 34: 267-342; Stymne et al. (1993) in: Biochemistry and Molecular Biology of Membrane and Storage Lipids of Plants, Hrsgb.: Murata und Somerville, Rockville, American Society of Plant Physiologists; 150-158, Murphy & Ross (1998) Plant Journal 13(1): 1-16.

Die im Verfahren hergestellten PUFAs umfassen eine Gruppe von Molekülen, die höhere Tiere nicht mehr (ausreichend) synthetisieren können und somit (zusätzlich) aufnehmen müssen, obwohl sie leicht von anderen Organismen wie Bakterien synthetisiert werden.

Der Begriff "Acyl-CoA:Lysophospholipid-Acyltransferasen" im Sinne der Erfindung umfasst Proteine, die am Transfer der an Phospholipide gebundenen Fettsäuren in den CoA-Ester-Pool und vice versa teilnehmen, sowie ihre Homologe, Derivate oder Analoga. Unter Phospholipiden im Sinne der Erfindung sind zu verstehen Phosphatidylcholin, Phosphatidylethanolamin, Phosphatidylserin, Phosphatidylglycerin, und/oder Phosphatidylinositol, vorteilhafterweise Phosphatidylcholin. Der Begriff Acyl-CoA:Lysophospholipid-Acyltransferase-Nukleinsäuresequenz(en) im Sinne der Erfindung umfasst Nukleinsäuresequenzen, die eine Acyl-CoA:Lysophospholipid-Acyltransferase, insbesondere eine Acyl-CoA:Lysophospatidylcholin-Acyltransferase, kodieren und eine kodierende Region und ggf. entsprechende 5'- und 3'-untranslatierte Sequenzbereiche umfassen können.

Bei einer weiteren Ausführungsform kodieren Derivate des erfindungsgemäßen Nukleinsäuremoleküls, das wiedergegeben ist in SEQ ID NO: 3 Proteine mit mindestens 70 bis 80 %, 80 bis 90 %, 90 bis 95 % und am meisten bevorzugt mindestens etwa 96 %, 97 %, 98 %, 99 % oder mehr Homologie (= Identität) zu einer vollständigen Aminosäuresequenz der SEQ ID NO: 4. Die Homologie wird über den gesamten Aminosäure- bzw. Nukleinsäuresequenzbereich berechnet. Für die Sequenzvergleiche kann das Programm PileUp verwendet werden (J. Mol. Evolution., 25, 351-360, 1987, Higgins et al., CABIOS, 5 1989: 151-153) oder die Programme Gap und BestFit (Needleman and Wunsch (1970) J. Mol. Biol. 48: 443-453 und Smith and Waterman (1981) Adv. Appl. Math. 2: 482-489), die im GCG Software-Packet (Genetics Computer Group, 575 Science Drive, Madison, Wisconsin, USA 53711 (1991)) enthalten sind. Die oben in Prozent angegebenen Sequenzhomologiewerte wurden mit dem Programm BestFit über den gesamten Sequenzbereich mit folgenden Einstellungen ermittelt: Gap Weight: 8, Length Weight: 2.

Die Erfindung umfasst zudem Nukleinsäuremoleküle, die sich von einer der in SEQ SEQ ID NO: 3 gezeigten Nukleotidsequenzen (und Teilen davon) aufgrund des degenerierten genetischen Codes unterscheiden und somit die gleiche Acyl-CoA:Lysophospholipid-Acyltransferase kodieren wie diejenige, die von den in SEQ ID NO: 3 gezeigten Nukleotidsequenzen kodiert wird.

Zusätzlich zu den in SEQ ID NO: 3 gezeigten Acyl-CoA:Lysophospholipid-Acyltransferase-Nukleotidsequenzen erkennt der Fachmann, dass innerhalb einer Population DNA-Sequenzpolymorphismen existieren können, die zu Änderungen in den Aminosäuresequenzen der Acyl-CoA:Lysophospholipid-Acyltransferasen führen können. Diese genetischen Polymorphismen im Acyl-CoA:Lysophospholipid-Acyltransferase-Gen können aufgrund von natürlicher Variation zwischen Individuen innerhalb einer Population existieren. Diese natürlichen Varianten bewirken üblicherweise eine Varianz von 1 bis 5 % in der Nukleotidsequenz des Acyl-CoA:Lysophospholipid-Acyltransferase-Gens. Sämtliche und alle dieser Nukleotidvariationen und daraus resultierende Aminosäurepolymorphismen in der Acyl-CoA:Lysophospholipid-Acyltransferase, die das Ergebnis natürlicher Variation sind und die funktionelle Aktivität der Acyl-CoA:Lysophospholipid-Acyltransferasen nicht verändern, sollen im Umfang der Erfindung enthalten sein.

Für das hier beschriebene Verfahren vorteilhafte Nukleinsäuremoleküle können auf der Grundlage ihrer Homologie zu den hier offenbarten Acyl-CoA:Lysophospholipid-Acyltransferase-Nukleinsäuren unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und die unter stringenten Bedingungen mit den Nukleinsäuremolekülen, die eine Nukleotidsequenz der SEQ ID NO: 1, SEQ ID NO: 3 oder SEQ ID NO: 5 umfassen, hybridisieren. Es können auch Nukleinsäuren mit mindestens 25, 50, 100, 250 oder mehr Nukleotiden verwendet werden. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, bevorzugt mindestens etwa 70 % und besonders bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodium citrate = SSC) bei etwa 45°C, gefolgt von einem oder mehreren Waschschritten in 0,2 x SSC, 0,1 % SDS bei 50 bis 65°C. Dem Fachmann ist bekannt, dass sich diese Hybridisierungsbedingungen je nach dem Typ der Nukleinsäure und je nachdem, ob beispielsweise organische Lösungsmittel vorliegen, hinsichtlich der Temperatur und der Konzentration des Puffers unterscheiden können. Die Temperatur liegt beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im oben genannten Puffer vorliegt, zum Beispiel 50 % Formamid, beträgt die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook und Russell, "Molecular Cloning", Cold Spring Harbor Laboratory, 2001; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Zur Bestimmung der prozentualen Homologie (= Identität) von zwei Aminosäuresequenzen (z.B. einer der Sequenzen der SEQ ID NO: 2, SEQ ID NO: 4 oder SEQ ID NO: 6) oder von zwei Nukleinsäuresequenzen (z.B. SEQ ID NO: 1, SEQ ID NO: 3 oder SEQ ID NO: 5) werden die Sequenzen zum optimalen Vergleich untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen. Die verwendeten Programme bzw. Algorithmen sind oben angegeben.

Ein isoliertes Nukleinsäuremolekül, das für eine Acyl-CoA:Lysophospholipid-Acyl-transferase kodiert, die zu einer Proteinsequenz der SEQ ID NO: 2, SEQ ID NO: 4 oder SEQ ID NO: 6 homolog ist, kann durch Einbringen einer oder mehrerer Nukleotidsubstitutionen, -additionen oder -deletionen in eine Nukleotidsequenz der SEQ ID NO: 1, SEQ ID NO: 3 oder SEQ ID NO: 5 erzeugt werden, so dass eine oder mehrere Aminosäuresubstitutionen, -additionen oder -deletionen in das kodierte Protein eingebracht werden. Mutationen können in eine der Sequenzen der SEQ ID NO: 1, SEQ ID NO: 3 oder SEQ ID NO: 5 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäurereste hergestellt. Bei einer "konservativen Aminosäuresubstitution" wird ein Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer Acyl-CoA:Lysophospholipid-Acyltransferase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der Acyl-CoA:Lysophospholipid-Acyltransferase-kodierenden Sequenz eingebracht werden, z.B. durch Sättigungsmutagenese, und die resultierenden Mutanten können nach der hier beschriebenen Acyl-CoA:Lysophospholipid-Acyltransferase-Aktivität durchmustert werden, um Mutanten zu identifizieren, die die Acyl-CoA:Lysophospholipid-Acyltransferase-Aktivität beibehalten haben. Nach der Mutagenese einer der Sequenzen der SEQ ID NO: 1, SEQ ID NO: 3 oder SEQ ID NO: 5 kann das kodierte Protein rekombinant exprimiert und die Aktivität des Proteins kann z.B. unter Verwendung der hier beschriebenen Tests bestimmt werden.

Diese Erfindung wird durch die nachstehenden Beispiele weiter veranschaulicht, die nicht als beschränkend aufgefasst werden sollten. Der Inhalt sämtlicher in dieser Patentanmeldung zitierten Literaturstellen, Patentanmeldungen, Patente und veröffentlichten Patentanmeldungen ist hier durch Bezugnahme aufgenommen.

### Beispiele

### Beispiel 1: Allgemeine Verfahren

### a) Allgemeine Klonierungsverfahren:

Klonierungsverfahren wie beispielsweise Restriktionsspaltungen, Agarose-Gelelektrophorese, Reinigung von DNA-Fragmenten, Transfer von Nukleinsäuren auf Nitrocellulose- und Nylonmembranen, Verbindung von DNA-Fragmenten, Transformation von *Escherichia coli*- und Hefe-Zellen, Anzucht von Bakterien und Sequenzanalyse rekombinanter DNA, wurden durchgeführt wie beschrieben in Sambrook und Russell (2001) (Cold Spring Harbor Laboratory Press: ISBN 0-87969-309-6) oder Kaiser, Michaelis und Mitchell (1994) "Methods in Yeast Genetics" (Cold Spring Harbor Laboratory Press: ISBN 0-87969-451-3).

### b) Chemikalien

Die verwendeten Chemikalien wurden, wenn im Text nicht anders angegeben, in p. A.-Qualität von den Firmen Fluka (Neu-Ulm), Merck (Darmstadt), Roth (Karlsruhe), Serva (Heidelberg) und Sigma (Deisenhofen) bezogen. Lösungen wurden unter Verwendung von reinem pyrogenfreiem Wasser, im nachstehenden Text als H₂O bezeichnet, aus einer Milli-Q-Wassersystem-Wasserreinigungsanlage (Millipore, Eschborn) hergestellt. Restriktionsendonukleasen, DNA-modifizierende Enzyme und molekularbiologische Kits wurden bezogen von den Firmen AGS (Heidelberg), Amersham (Braunschweig), Biometra (Göttingen), Roche (Mannheim), Genomed (Bad Oeynhausen), New England Biolabs (Schwalbach/Taunus), Novagen (Madison, Wisconsin, USA), Perkin-Elmer (Weiterstadt), Pharmacia (Freiburg), Qiagen (Hilden) und Stratagene (Amsterdam, Niederlande). Wenn nicht anders angegeben, wurden sie nach den Anweisungen des Herstellers verwendet.

### Beispiel 2: Klonierung eines Acyltransferase-Gens aus Ostreococcus tauri

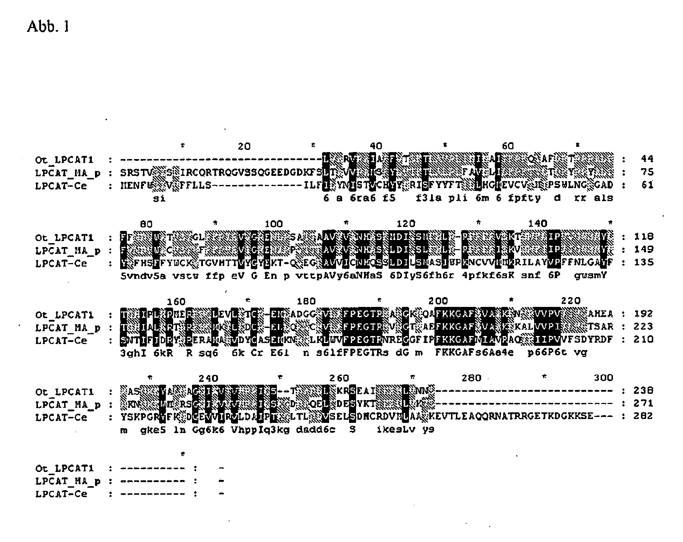

Durch Suche nach konservierten Bereichen in den Proteinsequenzen der bereits vorher isolierten LPCAT aus *C. elegans* (WO 2004/76617) konnte eine Sequenz mit entsprechenden Motiven in einer *Ostreococcus*-Sequenzdatenbank (genomische Sequenzen) identifiziert werden (siehe Abb. 1). Es handelt sich dabei um die folgende Sequenz:

| Gen-Name | SEQ ID Nr.: | Aminosäuren |
|---|---|---|
| OtLPCAT | 2 | 239 |

Die Klonierung des für dieses Protein kodierenden Gens wurde wie folgt durchgeführt:
40 ml einer *Ostreococcus tauri*-Kultur in der stationären Phase wurden abzentrifugiert, in 100 µl Aqua bidest resuspendiert und bei -20°C gelagert. Mit Hilfe des PCR-Verfahrens wurden die zugehörigen genomischen DNAs amplifiziert. Die entsprechenden Primerpaare wurden so ausgewählt, dass sie die ersten 20 Nukleotide am 5'-Ende bzw. die letzten 20 Nukleotide am 3'-Ende (inklusive Stopcodon) sowie am 5'-Ende zusätzlich die Hefe-Konsensus-Sequenz für hocheffiziente Translation (Kozak (1986) Cell 44: 283-292) trugen.

Folgende Primer wurden verwendet:

| | |
|---|---|
| 5'-119-Ot-LPCAT: | ATG CTG GTC GCG CGC GTC CGA GC |
| 3'-120-Ot-LPCAT-XhoI: | ACT CGA GTC ACG AGT TGT TCA CGA GGC |

Die Positionen der verwendeten Primer sind in Abb. 1 angegeben.

Die Amplifizierung der OtLPCAT-DNA wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

### Beispiel 3: Klonierung von Expressionsplasmiden zur heterologen Expression der OtLPCAT in Hefen

Die entsprechenden Primerpaare wurden so ausgewählt, dass sie die Hefe-Konsensus-Sequenz für hocheffiziente Translation (Kozak (1986) Cell 44: 283-292) neben dem Startcodon trugen. Die Amplifizierung der OtLPCAT wurde jeweils mit 1 µL cDNA, 200 µM dNTPs, 2,5 U *Advantage*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide in der PCR-Reaktion verwendet:

| Gen-Name | Primersequenz |
|---|---|
| OtLPCAT (SEQ ID No. 1) | F:5'-accatgctggtcgcgcgcgtccg |
| | R:5'- tcacgagttgttcacgaggc |

| | |
|---|---|
| *F=forward primer, R=reverse primer | |

Die PCR-Produkte wurden für 30 min bei 21 °C mit dem Hefe-Expressionsvektor pYES2.1-TOPO (Invitrogen) gemäß Herstellerangaben inkubiert. Das PCR-Produkt wird dabei durch einen T-Überhang und Aktivität einer Topoisomerase (Invitrogen) in den Vektor ligiert. Nach der Inkubation erfolgte dann die Transformation von *E. coli* DH5α-Zellen. Entsprechende Klone wurden durch PCR identifiziert, die Plasmid-DNA mittels Qiagen DNAeasy-Kit isoliert und durch Sequenzierung verifiziert. Die Sequenz des resultierenden Plasmids pYES2.1-OtLPCAT ist in SEQ ID No. 7 angegeben. Die korrekte Sequenz wurde dann in den *Saccharomyces-*Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthielten die entsprechenden Plasmide pYES2.1 oder pYES2.1-OtLPCAT. Nach der Selektion wurden je zwei Transformanten zur weiteren funktionellen Expression ausgewählt.

### Beispiel 4: Klonierung von OtLPCAT-Expressionsplasmiden zur Samenspezifischen Expression in Pflanzen

Für die Transformation von Pflanzen wurde ein Vektor auf der Basis von pSUN-USP erzeugt.

pSUN300 ist ein Derivat des Plasmides pPZP (Hajdukiewicz, P. et al.(1994) Plant Mol. Biol. 25: 989-994). pSUN-USP entstand aus pSUN300, indem in pSUN300 ein USP-Promotor als *Eco*RI- Fragment inseriert wurde. Der USP-Promotor entspricht den Nukleotiden 1-684 (Genbank Accession X56240), wobei ein Teil der nichtkodierenden Region des USP-Gens im Promotor enthalten ist. Das 684 Basenpaare große Promotorfragment wurde mittels eines käuflichen T7-Standardprimers (Stratagene) und eines synthetisierten Primers über eine PCR-Reaktion nach Standardmethoden amplifiziert.

Das PCR-Fragment wurde mit EcoRI/SalI nachgeschnitten und in den Vektor pSUN300 mit OCS Terminator eingesetzt. Das Polyadenylierungssignal ist das des Octopinsynthase-Gens aus dem *A. tumefaciens* Ti-Plasmid (ocs-Terminator, Genbank Accession V00088) (De Greve,H. et al. (1982) J. Mol. Appl. Genet. 1 (6): 499-511). Es entstand das Plasmid mit der Bezeichnung pSUN-USP. Das Konstrukt wurde zur Transformation von *Arabidopsis thaliana*, Raps, Tabak und Leinsamen verwendet.

Für die Klonierung der Ot-LPCAT in pSUN-USP wurden mit folgendem Primerpaar *Not*I-Schnittstellen am 5'- und 3'-Ende der kodierenden Sequenz eingefügt:
pSUN-OtLPCAT
Forward: 5'-GCGGCCGCACCATGCTGGTCGCGCGCGTCCG
Reverse: 3'-GCGGCCGCTCACGAGTTGTTCACGAGGC

Zusammensetzung des PCR-Ansatzes (50 µl):
5,00 µl Template cDNA
5,00 µl 10x Puffer (Advantage-Polymerase)+ 25 mM MgCl₂
5,00 µl 2mM dNTP
1,25 µl je Primer (10 pmoL/µL)
0,50 µl Advantage-Polymerase (Clontech)

Reaktionsbedingungen der PCR:
Anlagerungstemperatur: 1 min 55°C
Denaturierungstemperatur: 1 min 94°C
Elongationstemperatur: 2 min 72°C
Anzahl der Zyklen: 35

Die PCR-Produkte wurden für 16 h bei 37 °C mit dem Restriktionsenzym NotI inkubiert. Der Pflanzen-Expressionsvektor pSUN300-USP wurde in gleicher Weise inkubiert. Anschließend wurden die PCR-Produkte sowie der 7624 bp große Vektor durch Agarose-Gelelektrophorese aufgetrennt und die entsprechenden DNA-Fragmente ausgeschnitten. Die Aufreinigung der DNA-Fragmente erfolgte mittels Qiagen Gel Purification Kit gemäß Herstellerangaben. Anschließend wurden Vektor und PCR-Produkte ligiert. Dazu wurde das Rapid Ligation Kit von Roche verwendet. Das entstandene Plasmid pSUN-OtLPCAT wurde durch Sequenzierung verifiziert.

### Beispiel 5: Funktionelle Charakterisierung der OtLPCAT

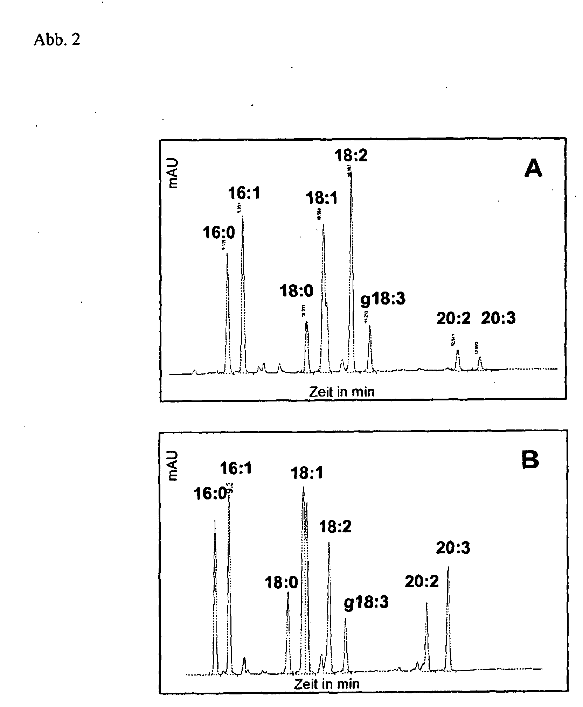

Die Aktivität der OtLPCAT konnte nach Expression der OtLPCAT in Hefen und Fütterung verschiedener Fettsäuren ermittelt werden (Abb. 2).

Da die Expression der OtLPCAT zu einem effizienten Austausch der Acyl-Substrate führen sollte, wurde weiterhin das Doppelkonstrukt pESCLeu-PpD6-PSE 1 hergestellt, das die offenen Leserahmen einer Δ6-Desaturase (PpD6) und einer Δ6-Elongase (PSE1) aus *Physcomitrella patens* (siehe DE 102 19 203) beinhaltet, und zusammen mit entweder dem Leervektor pYES2.1 oder dem Vektor pYES2.1-OtLPCAT transformiert. Die Klonierung des Konstrukts pESCLeu-PpD6-PSE1 kann der WO 2004/076617 entnommen werden, auf deren Inhalt hiermit ausdrücklich Bezug genommen wird.

Analysetechniken zur Bestimmung der Fettsäurezusammensetzung von Organismen sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman (1985) Encyclopedia of Industrial Chemistry, Bd. A2, S. 89-90 und S. 443-613, VCH Weinheim; Fallon, A. et al. (1987) "Applications of HPLC in Biochemistry" in: Laboratory Techniques in Biochemistry and Molecular Biology, Bd. 17; Rehm et al. (1993) "Product recovery and purification" in: Biotechnology, Bd. 3, Kapitel III, S. 469-714, VCH Weinheim; Belter, P.A. et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Für die Expression der OtLPCAT wurden zunächst Vorkulturen aus jeweils 2 ml CMdum-Flüssigmedium mit 2% (w/v) Raffinose, aber ohne Uracil und Leucin mit den ausgewählten Transformanten angeimpft und 2 Tage bei 30°C, 200rpm inkubiert. 5 ml CMdum-Flüssigmedium (ohne Uracil und Leucin) mit 2% Raffinose, 1% (v/v) Tergitol NP-40 und 250 µM Linolsäure (18:2^{Δ9,12}) oder Linolensäure (18:3^{Δ9,12,15}) wurden dann mit den Vorkulturen auf eine OD₆₀₀ von 0,08 angeimpft. Die Expression wurde bei einer OD₆₀₀ von 0,2-0,4 durch die Zugabe von 2% (w/v) Galaktose induziert. Die Kulturen wurden für weitere 48 h bei 20°C inkubiert.

Die Hefezellen aus den Hauptkulturen wurden durch Zentrifugation (100 x g, 10 min, 20°C) geerntet und mit 100 mM NaHCO₃, pH 8,0 gewaschen, um restliches Medium und Fettsäuren zu entfernen. Aus den Hefe-Zellsedimenten wurden durch saure Methanolyse Fettsäuremethylester (FAMEs) hergestellt. Hierzu wurden die Zellsedimente mit 2 ml 1N methanolischer Schwefelsäure und 2% (v/v) Dimethoxypropan für 1 h bei 80°C inkubiert. Die FAMEs wurden zweimal mit Petrolether (PE) extrahiert. Zur Entfernung nicht-derivatisierter Fettsäuren wurden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate 5°C/min und schließlich 10 min bei 250°C (halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson (2001) Lipids 36(8): 761-766; Sayanova et al. (2001) Journal of Experimental Botany 52(360): 1581-1585; Sperling et al.(2001) Arch. Biochem. Biophys. 388(2): 293-298 und Michaelson et al. (1998) FEBS Letters 439(3): 215-218.

Abb. 2A zeigt die Umsetzung der gefütterten Fettsäure 18:2^{Δ9,12} zu 20:3^{Δ8,11,14} durch Hefen, die mit den Plasmiden pESCLeu-PpD6-PSEl und pYES2.1 transformiert worden waren. Im Vergleich dazu zeigt Abb. 2B die Umsetzung in Hefen, die neben dem Plasmid pESCLeu-PpD6-PSE1 zusätzlich das Plasmid pYES2.1-OtLPCAT enthalten. Das gefütterte Substrat war in großen Mengen in allen transgenen Hefen nachzuweisen. Beide transgenen Hefen zeigten eine Synthese von 18:3^{Δ6,9,12} und 20:3^{Δ8,11,14}, den Produkten der Δ-6-Desaturase- und Δ-6-Elongase-Reaktionen. Dies bedeutet, dass die Gene PpD6 und PSE1 funktionell exprimiert werden konnten.

In den Kontroll-Hefen, die mit den Vektoren pESCLeu-PpD6-PSE1 und pYes2.1 transformiert wurden, war der Anteil von 20:3^{Δ8,11,14}, zu dem 18:3^{Δ6,9,12} durch PSE1 elongiert wird, wesentlich niedriger als in den Hefen, die zusätzlich die OtLPCAT exprimieren. Tatsächlich konnte die Elongation von 18:3^{Δ6,9,12} durch die zusätzliche Expression von OtLPCAT um 150% verbessert werden (Abb. 2B). Diese signifikante Erhöhung des LCPUFA-Gehalts ist nur wie folgt zu erklären: die exogen gefütterte Fettsäure (18:2^{Δ9,12}) wird zunächst in Phospholipide eingebaut und dort von der Δ-6-Desaturase zu 18:3^{Δ6,9,12} desaturiert. Erst nach Reäquilibrierung mit dem Acyl-CoA-Pool kann 18:3^{Δ6,9,12} durch die Elongase zu 20:3^{ΔA,11,14}-CoA elongiert und dann wieder in die Lipide eingebaut werden. Die OtLPCAT ist in der Lage, die in Phospholipide eingebauten Δ6-desaturierten Acylgruppen sehr effizient in CoA-Thioester zurückzuverwandeln. Dadurch wird auch die Elongation der gefütterten Fettsäure 18:2^{Δ9,12} verbessert (Abb. 2B).

### Beispiel 6: Klonierung von Acyltransferase-Genen aus Mantoniella squamata

Durch Suche nach konservierten Bereichen in den Proteinsequenzen der bereits vorher isolierten LPCAT aus *C. elegans* (WO 2004/76617) konnten Sequenzen mit entsprechenden Motiven in einer *Mantoniella*-Sequenzdatenbank identifiziert werden (siehe Abb. 1). Es handelt sich dabei um die folgenden Sequenzen:

| Gen-Name | SEQ ID No. | Aminosäuren |
|---|---|---|
| MsLPCAT112 | 4 | 233 |
| MsLPCAT18 | 6 | 272 |

Die Klonierung der für diese Proteine kodierenden Gene wurde wie folgt durchgeführt:
Eine 21-Kultur von *Mantoniella squamata* wurde in f/2 Medium (Guillard, R.R.L. (1975) Culture of phytoplankton for feeding marine invertebrates. In Culture of Marine Invertebrate Animals (Eds. Smith, W.L. and Chanley, M.H.), Plenum Press, New York, pp 29-60) für 14 Tage bei einer Lichtstärke von 80 E/cm² angezogen. Nach Zentrifugation der Zellen wurde mit Hilfe des RNAeasy Kits der Firma Qiagen (Valencia, CA, US) RNA nach Herstellerangaben isoliert. Die mRNA wurde mit dem Marathon cDNA Amplification-Kit (BD Biosciences) revers transkribiert und entsprechend den Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

Die zugehörigen genomischen DNAs wurden durch PCR amplifiziert. Die entsprechenden Primerpaare wurden so ausgewählt, dass sie die ersten 20 Nukleotide am 5'-Ende bzw. die letzten 20 Nukleotide am 3'-Ende (inklusive Stopcodon) sowie am 5'-Ende zusätzlich die Hefe-Konsensus-Sequenz für hocheffiziente Translation (Kozak (1986) Cell 44: 283-292) trugen.

Folgende Primer wurden verwendet:
5'-112-MA-LPCAT-BamHI: AGG ATC CAT GTC TTT TTA CCT CGT CAC CTT CAC C
3'-113-MA-LPCAT_XhoI: ACT CGA GTC ACG AGT ACT TGA CAA GGC
   für die kürzere Form der LPCAT aus *Mantoniella squamata* und
5'-118-MA-LPCAT: ATG TCG AGG TCG ACG GTA TCG AT
3'-113-MA-LPCAT_XhoI: ACT CGA GTC ACG AGT ACT TGA CAA GGC
   für die längere Form der LPCAT aus *Mantoniella squamata*.

Die Positionen der verwendeten Primer sind in Abb. 1 angegeben.

Die Amplifizierung der MsLPCAT-DNAs wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: erste Denaturierung bei 95°C für 5 Minuten, gefolgt von 30 Zyklen bei 94°C für 30 Sekunden, 55°C für 1 Minute und 72°C für 2 Minuten sowie einem letzten Verlängerungsschritt bei 72°C für 10 Minuten.

### Beispiel 7: Klonierung von Expressionsplasmiden zur heterologen Expression von MsLPCAT in Hefen

Die entsprechenden Primerpaare wurden so ausgewählt, dass sie die Hefe-Konsensus-Sequenz für hocheffiziente Translation (Kozak (1986) Cell 44: 283-292) neben dem Startcodon trugen. Die Amplifizierung der MsLPCATs wurde jeweils mit 1 µL cDNA, 200 µM dNTPs, 2,5 U *Advantage*-Polymerase und 100 pmol eines jeden Primers in einem Gesamtvolumen von 50 µl durchgeführt. Die Bedingungen für die PCR waren wie folgt: erste Denaturierung bei 95°C für 5 Minuten, gefolgt von 30 Zyklen bei 94°C für 30 Sekunden, 55°C für 1 Minute und 72°C für 2 Minuten sowie einem letzten Verlängerungsschritt bei 72°C für 10 Minuten.

Für die Klonierung der Sequenz zur heterologen Expression in Hefen wurden folgende Oligonukleotide für die PCR-Reaktion verwendet:

| Gen-Name: | Primersequenz |
|---|---|
| MsLPCAT112 (SEQ ID No. 3) | F:5'-accatgtctttttacctcgtcac |
| | R:5'- tcacgagtacttgacaaggc |
| MsLPCAT118 (SEQ ID No. 5) | F:5'-accatgtcgaggtcgacggtatc |
| | R:5'- tcacgagtacttgacaaggc |

| | |
|---|---|
| *F=forward primer, R=reverse primer | |

Die PCR-Produkte wurden für 30 min bei 21 °C mit dem Hefe-Expressionsvektor pYES2.1-TOPO (Invitrogen) gemäß Herstellerangaben inkubiert. Das PCR-Produkt wird dabei durch einen T-Überhang und Aktivität einer Topoisomerase (Invitrogen) in den Vektor ligiert. Nach der Inkubation erfolgte dann die Transformation von *E. coli* DH5α-Zellen. Entsprechende Klone wurden durch PCR identifiziert, die Plasmid-DNA mittels Qiagen DNAeasy-Kit isoliert und durch Sequenzierung verifiziert. Die Sequenzen der resultierenden Plasmide pYES2.1-MsLPCAT112 bzw. pYES2.1-MsLPCAT118 sind in den SEQ ID Nos. 8 bzw. 9 angegeben. Die korrekte Sequenz wurde dann in den *Saccharomyces*-Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthielten die entsprechenden Plasmide pYES2.1, pYES2.1-MsLPCAT112 oder pYES2.1-MsLPCAT118. Nach der Selektion wurden je zwei Transformanten zur weiteren funktionellen Expression ausgewählt.

### Beispiel 8: Klonierung von Expressionsplasmiden zur Samen-spezifischen Expression in Pflanzen

Für die Transformation von Pflanzen wurde ein weiterer Vektor auf der Basis von pSUN-USP (siehe Beispiel 4) erzeugt. Dazu wurden mit folgenden Primerpaaren *Not*I-Schnittstellen am 5' und 3'-Ende der kodierenden Sequenzen eingefügt:
pSUN-MsLPCAT112
   Forward: 5'-GCGGCCGCACCATGTCTTTTTACCTCGTCAC
   Reverse: 3'-GCGGCCGCTCACGAGTACTTGACAAGGC
pSUN-MsLPCAT118
   Forward: 5'-GCGGCCGCACCATGTCGAGGTCGACGGTATC
   Reverse: 3'-GCGGCCGCTCACGAGTACTTGACAAGGC

Zusammensetzung des PCR-Ansatzes (50 µl):
5,00 µl Template cDNA
5,00 µl 10x Puffer (Advantage-Polymerase)+ 25 mM MgCl₂
5,00µl 2mM dNTP
1,25 µl je Primer (10 pmol/µL)
0,50 µl Advantage-Polymerase (Clontech)

Reaktionsbedingungen der PCR:
Anlagerungstemperatur: 1 min 55°C
Denaturierungstemperatur: 1 min 94°C
Elongationstemperatur: 2 min 72°C
Anzahl der Zyklen: 35

Die PCR-Produkte wurden für 16 h bei 37 °C mit dem Restriktionsenzym *Not*I inkubiert. Der Pflanzen-Expressionsvektor pSUN300-USP wurde in gleicher Weise inkubiert. Anschließend wurden die PCR-Produkte sowie der 7624 bp große Vektor durch Agarose-Gelelektrophorese aufgetrennt und die entsprechenden DNA-Fragmente ausgeschnitten. Die Aufreinigung der DNA-Fragmente erfolgte mittels Qiagen Gel Purification Kit gemäß Herstellerangaben. Anschließend wurden Vektor und PCR-Produkte ligiert. Dazu wurde das Rapid Ligation Kit von Roche verwendet. Die entstandenen Plasmide pSUN-MsLPCAT112 und pSLTN-MsLPCAT118 wurden durch Sequenzierung verifiziert.

### Beispiel 9: Funktionelle Charakterisierung der MsLPCATs

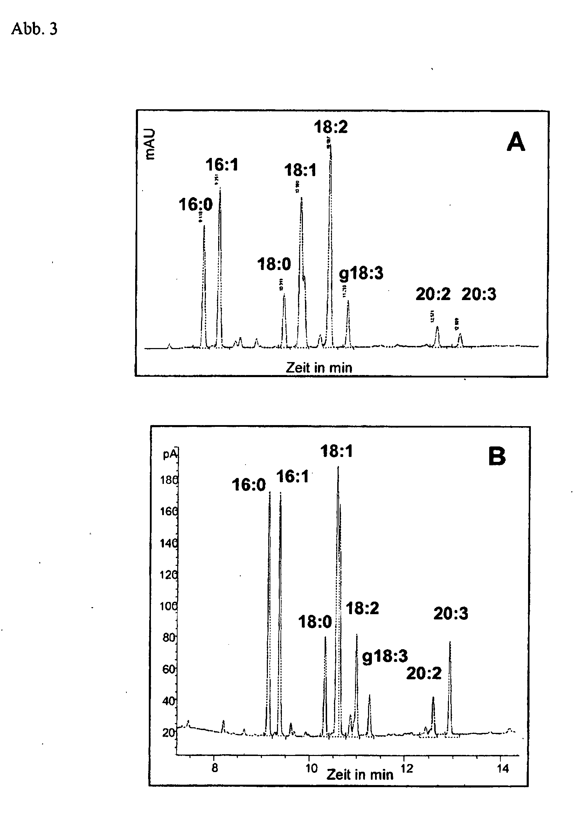

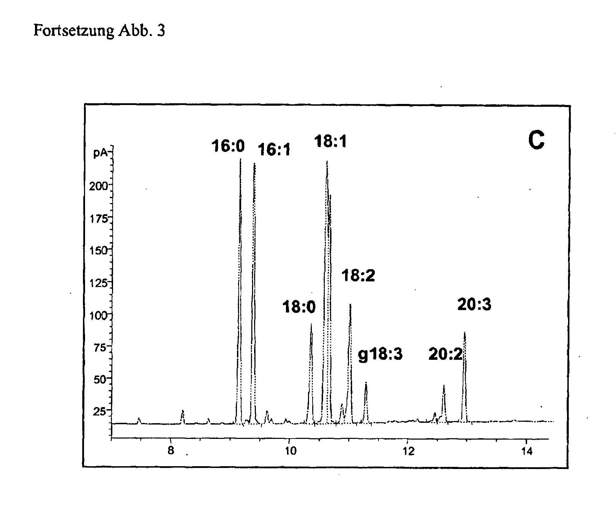

Die Aktivität der MsLPCATs konnte nach Expression der MsLPCATs in Hefen und Fütterung verschiedener Fettsäuren ermittelt werden (Fig. 3A, B und C). Wie in Beispiel 5 wurde auch hier das Konstrukt pESCLeu-PpD6-PSE1 zusammen mit entweder dem Leervektor pYES2.1 oder dem Plasmid pYES2.1-MsLPCAT112 bzw. pYES2.1-MsLPCAT118 in die Hefen eingebracht.

Die Expression der MsLPCATs erfolgte wie in Beispiel 5 für die OtLPCAT beschrieben.

Figur 3A zeigt die Umsetzung der gefütterten Fettsäure 18:2^{Δ9,12} zu 20:3^{Δ8,11,14} durch Hefen, die mit dem Plasmid pESCLeu-PpD6-PSE1 und pYES2.1 transformiert worden waren. Im Vergleich dazu zeigt Figur 3B die Umsetzung in Hefen, die neben dem Plasmid pESCLeu-PpD6-PSE1 zusätzlich das Plasmid pYES2.1-MsLPCAT112 enthalten. Figur 3C beschreibt das Fettsäurespektrum von Hefen, die mit den Plasmiden pESCLeu-PpD6-PSE1 und pYES2.1-MsLPCAT 118 transformiert worden waren. Das gefütterte Substrat Linolsäure (18:2) war in großen Mengen in allen transgenen Hefen nachzuweisen. Alle transgenen Hefen zeigten eine Synthese von 18:3^{Δ6,9,12} und 20:3^{Δ8,11,14}, den Produkten der Δ-6- Desaturase- und Δ-6-Elongase-Reaktionen. Dies bedeutet, dass die Gene PpD6 und Pse1 funktional exprimiert werden konnten.

In den Kontroll-Hefen, die mit den Vektoren pESCLeu-PpD6-PSE1/pYES2.1 transformiert wurden, war der Anteil von 20:3^{Δ8,11,14}, zu dem 18:3^{Δ6,9,12} durch PSE1 elongiert wird, wesentlich niedriger als in den Hefen, die zusätzlich eine der beiden MsLPCATs exprimieren. Tatsächlich konnte die Elongation von 18:3^{Δ6,9,12} durch die zusätzliche Expression einer MsLPCAT um 70% (MsLPCAT112) bzw. 160 % (MsLPCAT118) verbessert werden (Abb. 3B, C). Diese signifikante Erhöhung des LCPUFA-Gehalts ist nur wie folgt zu erklären: die exogen gefütterte Fettsäure (18_{:}2^{Δ9,12}) wird zunächst in Phospholipide eingebaut und dort von der Δ-6-Desaturase zu 18:3^{Δ6,9,12} desaturiert. Erst nach Reäquilibrierung mit dem Acyl-CoA-Pool kann 18:3^{Δ6,9,12} durch die Elongase zu 20:3^{Δ8,11,14}-CoA elongiert und dann wieder in die Lipide eingebaut werden. Die MsLPCATs sind in der Lage, die Δ6-desaturierten Acylgruppen sehr effizient in CoA-Thioester zurückzuverwandeln. Dabei wird auch die Elongation der gefütterten Fettsäuren 18:2^{Δ9,12} verbessert (Abb. 3B, C).

### Beispiel 10: Pflanzentransformation und Expression von PUFA-spezifischen Acyltransferasen in Pflanzen

Die Expression von LCPUFA-spezifischen Acyltransferasen in transgenen Pflanzen ist vorteilhaft, um den LCPUFA-Gehalt in diesen Pflanzen zu erhöhen. Dazu wurden die erfindungsgemäßen Acyltransferase-cDNAs in binäre Vektoren kloniert (siehe Beispiele 4 und 8) und über Agrobacterium-vermittelten DNA-Transfer in *Arabidopsis thaliana, Brassica napus* und *Linum usitatissimum* übertragen. Die Expression der Acyltransferase-cDNA stand dabei unter der Kontrolle des samenspezifischen USP-Promotors (Konstruktion des binären Plasmids pSUN300-USP siehe Beispiel 4).

Besonders bevorzugt sind hierbei transgene Pflanzen, die bereits die für die Synthese von LCPUFAs notwendigen Desaturasen und Elongasen exprimieren und geringe Mengen dieser LCPUFAs herstellen. Solche Pflanzen sind beispielsweise die in DE 102 19 203 beschriebenen, die funktionelle Gene für die Δ-6-Desaturase, die Δ-6-Elongase und die Δ-5-Desaturase enthalten und geringe Mengen an ARA und EPA produzieren.

Die entstandenen binären Vektoren mit Acyltransferasegenen wurden in *Agrobacterium tumefaciens* transformiert (Höfgen und Willmitzer (1988) Nucl. Acids Res. 16: 9877). Die Transformation von *A. thaliana* erfolgte mittels "floral dip" (Clough und Bent (1998) Plant Journal 16: 735 - 743), die von *N. tabacum* über Cokultivierung von Tabakblattstückchen mit transformierten *A. tumefaciens*-Zellen, die von Lein und Raps durch Kokultivierung von Hypokotylstücken mit transformierten *A. tumefaciens-Zellen.* Entsprechende Methoden sind dem Fachmann bekannt.

Die Expression der Acyltransferase-Gene in transgenen Arabidopsis-, Tabak-, Raps- und Leinpflanzen wurde durch Northern-Blot Analyse untersucht. Ausgewählte Pflanzen wurden auf ihren Gehalt an PUFAs im Samenöl untersucht.

Die Agrobacterium-vermittelte Pflanzentransformation kann unter Verwendung von Standard-Transformations- und Regenerationstechniken durchgeführt werden (Gelvin, Stanton B., Schilperoort, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molecular Biology and Biotechnology, B. Raton: CRC Press, 1993, 360 S., ISBN 0-8493-5164-2).

Beispielsweise kann Raps mittels Kotyledonen- oder Hypokotyltransformation transformiert werden (Moloney et al. (1989) Plant Cell Report 8: 238-242; De Block et al. (1989) Plant Physiol. 91: 694-701). Die Verwendung von Antibiotika für die Agrobacterium- und Pflanzenselektion hängt von dem für die Transformation verwendeten binären Vektor und Agrobacteriumstamm ab. Die Rapsselektion wird gewöhnlich unter Verwendung von Kanamycin als selektierbarem Pflanzenmarker durchgeführt. Der Agrobacterium-vermittelte Gentransfer in Lein (*Linum usitatissimum*) lässt sich beispielsweise unter Verwendung einer von Mlynarova et al. (1994) Plant Cell Report 13: 282-285 beschriebenen Technik durchführen.

Die Transformation von Soja kann unter Verwendung beispielsweise einer in EP-A-O 0424047 (Pioneer Hi-Bred International) oder in EP-A-O 0397687, US 5,376,543, US 5,169,770 (University Toledo) beschriebenen Technik durchgeführt werden. Die Pflanzentransformation unter Verwendung von Teilchenbeschuss, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York.

Für den Nachweis von Fettsäuren in Pflanzen werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

So kann die Analyse von Fettsäuren oder Triacylglycerin (= TAG, Abkürzungen in Klammern angegeben) z.B. mittels Fettsäuremethylester (= FAME), Gas-Flüssigkeitschromatographie-Massenspektrometrie (= GC-MS) oder Dünnschichtchromatographie (TLC) erfolgen.

Der unzweideutige Nachweis für das Vorliegen von Fettsäureprodukten kann mittels Analyse rekombinanter Organismen nach Standard-Analyseverfahren wie GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353) erbracht werden.

Das zu analysierende Pflanzenmaterial kann dazu entweder durch Ultraschallbehandlung, Mahlen in der Glasmühle, flüssigen Stickstoff und Mahlen oder über andere anwendbare Verfahren aufgebrochen werden. Das Material wird dann anschließend nach dem Aufbrechen zentrifugiert. Das Sediment wird danach in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester können anschließend in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen werden. Die Identität der erhaltenen Fettsäuremethylester lassen sich unter Verwendung von Standards, die aus kommerziellen Quellen erhältlich sind (d.h. Sigma), definieren.

Bei Fettsäuren, für die keine Standards verfügbar sind, kann die Identität über Derivatisierung und anschließende GC-MS-Analyse gezeigt werden. Beispielsweise erfolgt die Lokalisierung von Fettsäuren mit Dreifachbindung über GC-MS nach Derivatisierung mit 4,4-Dimethoxyoxazolin-Derivaten (Christie, 1998, siehe oben).

### Fettsäureanalyse in Pflanzen

Die Gesamt-Fettsäuren wurden aus Pflanzensamen extrahiert und mittels Gaschromatographie analysiert.

Die Samen wurden mit 1 % Natriummethanolat in Methanol aufgenommen und 20 min bei RT (ca. 22 °C) inkubiert. Anschließend wurde mit NaCl-Lösung gewaschen und die FAME in 0,3 ml Heptan aufgenommen.

Die Proben wurden auf einer ZEBRON-ZB-Wax-Kapillarsäule (30 m, 0,32 mm, 0,25 mikro m; Phenomenex) in einem Hewlett Packard-6850-Gaschromatograph mit einem Flammenionisationsdetektor aufgetrennt. Die Ofentemperatur wurde von 70°C (1 min halten) bis 200°C mit einer Rate von 20°C/min, dann auf 250°C (5 min halten) mit einer Rate von 5°C/min und schließlich auf 260°C mit einer Rate von 5°C/min programmiert. Als Trägergas wurde Stickstoff verwendet (4,5 ml/min bei 70°C). Die Fettsäuren wurden durch Vergleich mit Retentionszeiten von FAME-Standards (SIGMA) identifiziert.

Aus den hier vorliegenden Arbeiten lässt sich die Funktion der Acyl-CoA:Lysophopholipid-Acyltranserase wie in Abb. 4 dargestellt ableiten. Der Biosynthese-Weg der LCPUFAS stellt sich damit wie folgt dar:
Desaturasen katalysieren die Einführung von Doppelbindungen in lipidgekoppelte Fettsäuren (*sn2*-Acyl-Phosphatidylcholin), während die Elongasen exklusiv die Elongation Coenzym A-veresterter Fettsäuren (Acyl-CoAs) katalysieren. Nach diesem Mechanismus erfordert die alternierende Wirkung von Desaturasen und Elongasen einen ständigen Austausch von Acyl-Substraten zwischen Phospholipiden und dem Acyl-CoA-Pool und somit die Existenz einer zusätzlichen Aktivität, die die Acyl-Substrate in die jeweils notwendige Substratform, d.h. Lipide (für Desaturasen) oder CoA-Thioester (für Elongasen), überführt. Dieser Austausch zwischen Acyl-CoA Pool und Phospholipiden wird durch LCPUFA-spezifische Acyl-CoA:Lyso-phopholipid-Acyltranserasen ermöglicht.

### Beschreibung der Abbildungen

Abb. 1: Aminosäure-Sequenzvergleich von OtLPCAT, MsLPCAT und CeLPCAT
Abb. 2: Fettsäureanalyse von Hefen, die mit den Plasmiden pESCLeu-PpD6-PSE1 und pYES2.1 (A) bzw. pLEU-PSE1(Pp)_d6Des(Pp) und pYES2.1-OtLPCAT (B) transformiert worden waren, nach Fütterung mit 18:2^{Δ9,12}.
Abb. 3: Fettsäureanalyse von Hefen, die mit dne Plasmiden pESCLeu-PpD6-PSE1 und pYES2.1 (A), pESCLeu-PpD6-PSE1 und pYES2.1-MsLPCAT112 (B) bzw. pESCLeu-PpD6-PSE1 und pYES2.1-MsLPCAT112 (C) transformiert worden waren, nach Fütterung mit 18:2^{Δ9,12}.
Abb. 4: Biosyntheseweg von LCPUFAs

## Patentansprüche

1. Isolierte Nukleinsäuresequenz, die für Polypeptid mit Acyl-CoA:Lyso-phospholipid-Acyltransferaseaktivität kodiert, wobei die durch die Nukleinsäuresequenz kodierte Acyl-CoA:Lysophospholipid-Acyltransferase spezifisch C₁₆-, C18-, C₂₀- oder C₂₂-Fettsäuren mit mindestens einer Doppelbindung im Fettsäuremolekül umsetzt und wobei die Nukleinsäuresequenz ausgewählt ist aus der Gruppe bestehend aus:
a) einer Nukleinsäuresequenz mit der in SEQ ID NO: 3 dargestellten Sequenz,
b) Nukleinsäuresequenzen, die sich als Ergebnis des degenerierten genetischen Codes von der in SEQ ID NO: 3 enthaltenen kodierenden Sequenz ableiten lassen, und
c) Derivaten der in SEQ ID NO: 3 dargestellten Nukleinsäuresequenz, die für ein Polypeptid mit der in SEQ ID NO: 4 dargestellten Aminosäuresequenz kodiert und mindestens 70% Identität auf Aminosäureebene mit SEQ ID NO: 4 zeigen und eine Acyl-CoA:Lysophospholipid-Acyltransferaseaktivität aufweisen.

2. Aminosäuresequenz, die von einer isolierten Nukleinsäuresequenz nach Anspruch 1 kodiert wird.

3. Genkonstrukt, enthaltend eine isolierte Nukleinsäure nach Anspruch 1, wobei die Nukleinsäure funktionsfähig mit einem oder mehreren Regulationssignalen verbunden ist.

4. Genkonstrukt nach Anspruch 3,
**dadurch gekennzeichnet, dass** das Nukleinsäurekonstrukt zusätzliche Biosynthesegene des Fettsäure- oder Lipidstoffwechsels enthält, ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-Acyl-Transferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n).

5. Genkonstrukt nach Anspruch 3 oder 4,
**dadurch gekennzeichnet, dass** das Nukleinsäurekonstrukt zusätzliche Biosynthesegene des Fettsäure- oder Lipidstoffwechsels enthält, ausgewählt aus der Gruppe der Δ-4-Desaturase-, Δ-5-Desaturase-, Δ-6-Desaturase-, Δ-8-Desaturase-, Δ-9-Desaturase-, Δ-12-Desaturase-, Δ-5-Elongase-, Δ-6-Elongase- oder Δ-9-Elongasegene.

6. Vektor, enthaltend eine Nukleinsäure nach Anspruch 1 oder ein Genkonstrukt nach einem der Ansprüche 3 bis 5.

7. Transgene Pflanze,wobei die Pflanze transgen für die Nukleinsäure nach Anspruch 1, das Genkonstrukt nach einem der Ansprüche 3 bis 5 oder einen Vektor nach Anspruch 6 ist.

8. Verfahren zur Herstellung mehrfach ungesättigter Fettsäuren in einem Organismus,
**dadurch gekennzeichnet, dass** das Verfahren folgende Schritte umfasst:
a) Einbringen mindestens einer Nukleinsäuresequenz in den Organismus ausgewählt aus der Gruppe bestehend aus
i) Nukleinsäuresequenz mit der in SEQ ID NO: 3 dargestellten Sequenz, die für ein Polypeptid mit einer Acyl-CoA:Lysophospholipid-Acyltransferaseaktivität kodiert;
ii) Nukleinsäuresequenzen, die sich als Ergebnis des degenerierten genetischen Codes von der in SEQ ID NO: 3 enthaltenen kodierenden Sequenz ableiten lassen; und
iii) Derivaten der in SEQ ID NO: 3 dargestellten Nukleinsäuresequenz, die für ein Polypeptid mit der in SEQ ID NO: 4 dargestellten Aminosäuresequenz kodiert und mindestens 70 % Identität auf Aminosäureebene mit SEQ ID NO: 4 zeigen und eine Acyl-CoA:Lysophospholipid-Acyltransferaseaktivität aufweisen; und
b) Kultivieren und Ernten des Organismus.

9. Verfahren nach Anspruch 8,
**dadurch gekennzeichnet, dass** zusätzlich zu den unter Schritt a) genannten Nukleinsäuresequenzen weitere Nukleinsäuresequenzen in den Organismus eingebracht werden, die für Polypeptide des Fettsäure- oder Lipidstoffwechsels ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-Acyl-Transferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) kodieren.

10. Verfahren nach einem der Ansprüche 8 oder 9,
**dadurch gekennzeichnet, dass** zusätzlich zu den unter Schritt a) von Anspruch 8 genannten Nukleinsäuresequenzen und ggf. den in Anspruch 9 genannten Nukleinsäuresequenzen weitere Nukleinsäuresequenzen in den Organismus eingebracht werden, die für Polypeptide ausgewählt aus der Gruppe bestehend aus Δ-4-Desaturasen, Δ-5-Desaturasen, Δ-6-Desaturasen, Δ-8-Desatwasen, Δ-9-Desaturasen, Δ-12-Desaturasen, Δ-3-Elongasen, Δ-6-Elongasen oder Δ-9-Elongasen kodieren.

11. Verfahren nach einem der Ansprüche 8 bis 10,
**dadurch gekennzeichnet, dass** als Substrat der Acyl-CoA:Lysophospholipid-Acyltransferasen C₁₆-, C₁₈-, C₂₀- oder C₂₂-Fettsäuren verwendet werden.

12. Verfahren nach einem der Ansprüche 8 bis 11,
**dadurch gekennzeichnet, dass** die mehrfach ungesättigten Fettsäuren aus dem Organismus in Form eines Öls, Lipids oder einer freien Fettsäure isoliert werden.

13. Verfahren nach einem der Ansprüche 8 bis 12,
**dadurch gekennzeichnet, dass** die im Verfahren hergestellten mehrfach ungesättigten Fettsäuren C₁₈-, C₂₀- oder C₂₂-Fettsäuren mit mindestens zwei Doppelbindungen im Molekül sind.

14. Verfahren nach einem der Ansprüche 8 bis 13,
**dadurch gekennzeichnet, dass** der Organismus eine transgene Pflanze ist.

15. Verwendung einer Nukleinsäuresequenz ausgewählt aus der Gruppe bestehend aus
i) Nukleinsäuresequenz mit der in SEQ ID NO: 3 dargestellten Sequenz, die für ein Polypeptid mit einer Acyl-CoA:Lysophospholipid-Acyltransferaseaktivität kodiert;
ii) Nukleinsäuresequenzen, die sich als Ergebnis des degenerierten genetischen Codes von der in SEQ ID NO: 3 enthaltenen kodierenden Sequenz ableiten lassen; und
iii) Derivaten der in SEQ ID NO: 3 dargestellten Nukleinsäuresequenz, die für ein Polypeptid mit der in SEQ ID NO: 4 dargestellten Aminosäuresequenz kodiert und mindestens 70% Identität auf Aminosäureebene mit SEQ ID NO: 4 zeigen und eine Acyl-CoA:Lysophospholipid-Acyltransferaseaktivität aufweisen,
zur Herstellung eines Öls, Lipids oder einer Fettsäurezusammensetzung, wobei das Öl, Lipid oder die Fettsäurezusammensetzung mehrfach ungesättigte Fettsäuren umfasst und aus einer mit dieser Nukleinsäuresequenz transgenen Pflanze isoliert wurde.

## Claims

1. An isolated nucleic acid sequence which codes for a polypeptide with acyl-CoA lysophospholipid acyltransferase activity, wherein the acyl-CoA lysophospholipid acyltransferase encoded by the nucleic acid sequence specifically converts C₁₆, C₁₈, C₂₀ or C₂₂ fatty acids with at least one double bond in the fatty acid molecule and wherein the nucleic acid sequence is selected from the group consisting of:
a) a nucleic acid sequence with the sequence shown in SEQ ID No.3,
b) nucleic acid sequences which as a result of the degenerate genetic code can be derived from the coding sequence contained in SEQ ID No.3, and
c) derivatives of the nucleic acid sequence shown in SEQ ID No.3 which codes for a polypeptide with the amino acid sequence shown in SEQ ID No.4 and show at least 70% identity with SEQ ID No.4 at the amino acid level and have an acyl-CoA lysophospholipid acyltransferase activity.

2. An amino acid sequence which is encoded by an isolated nucleic acid sequence as claimed in claim 1.

3. A gene construct containing an isolated nucleic acid as claimed in claim 1, wherein the nucleic acid is functionally linked with one or more regulation signals.

4. The gene construct as claimed in claim 4,
**wherein** the nucleic acid construct contains additional biosynthesis genes of the fatty acid or lipid metabolism, selected from the group consisting of acyl-CoA dehydrogenase(s), acyl-ACP [= acyl carrier protein] desaturase(s), acyl-ACP thioesterase(s), fatty acid acyl transferase(s), fatty acid synthase(s), fatty acid hydroxylase (s) , acetyl-coenzyme A carboxylase (s), acyl-coenzyme A oxidase(s), fatty acid desaturase(s), fatty acid acetylenases, lipoxygenases, triacylglycerol lipases, allene oxide synthases, hydroperoxide lyases or fatty acid elongase(s).

5. The gene construct as claimed in claim 3 or 4,
**wherein** the nucleic acid construct contains additional biosynthesis genes of the fatty acid or lipid metabolism, selected from the group of the Δ-4 desaturase, Δ-5 desaturase, Δ-6 desaturase, Δ-8 desaturase, Δ-9 desaturase, Δ-12 desaturase, Δ-5 elongase, Δ-6 elongase or Δ-9 elongase genes.

6. A vector containing a nucleic acid as claimed in claim 1 or a gene construct as claimed in one of claims 3 to 5.

7. A transgenic plant, wherein the plant is transgenic for the nucleic acid as claimed in claim 1, the gene construct as claimed in one of claims 3 to 5 or a vector as claimed in claim 6.

8. A method for the production of multiply unsaturated fatty acids in an organism,
**wherein** the process comprises the following steps:
a) introduction of at least one nucleic acid sequence into the organism selected from the group consisting of
i) a nucleic acid sequence with the sequence shown in SEQ ID No.3, which codes for a polypeptide with an acyl-CoA lysophospholipid acyltransferase activity,
ii) nucleic acid sequences which as a result of the degenerate genetic code can be derived from the coding sequence contained in SEQ ID No.3, and
iii) derivatives of the nucleic acid sequence shown in SEQ ID No.3 which codes for a polypeptide with the amino acid sequence shown in SEQ ID No.4 and show at least 70% identity with SEQ ID No.4 at the amino acid level and have an acyl-CoA lysophospholipid acyltransferase activity, and
b) cultivating and harvesting the organism.

9. A method as claimed in claim 8,
**wherein,** in addition to the nucleic acid sequences mentioned under step a), further nucleic acid sequences are introduced into the organism, which code for polypeptides of the fatty acid or lipid metabolism, selected from the group consisting of acyl-CoA dehydrogenase(s), acyl-ACP [= acyl carrier protein] desaturase(s), acyl-ACP thioesterase(s), fatty acid acyl transferase(s), fatty acid synthase(s), fatty acid hydroxylase (s) , acetyl-coenzyme A carboxylase (s) , acyl-coenzyme A oxidase(s), fatty acid desaturase(s), fatty acid acetylenases, lipoxygenases, triacylglycerol lipases, allene oxide synthases, hydroperoxide lyases or fatty acid elongase(s).

10. The method as claimed in one of claims 8 or 9,
**wherein** in addition to the nucleic acid sequences mentioned under step a) of claim 8, and if appropriate the nucleic acid sequences mentioned in claim 9, further nucleic acid sequences are introduced into the organism, which code for polypeptides of the fatty acid or lipid metabolism, selected from the group consisting of the Δ-4 desaturases, Δ-5 desaturases, Δ-6 desaturases, Δ-8 desaturases, Δ-9 desaturases, Δ-12 desaturases, Δ-5 elongases, Δ-6 elongases or Δ-9 elongases.

11. The method as claimed in one of claims 8 to 10,
**wherein** as substrate of the acyl-CoA lysophospholipid acyltransferases, C₁₆, C₁₈, C₂₀ or C₂₂ fatty acids are used.

12. The method as claimed in one of claims 8 to 11,
**wherein** the multiply saturated fatty acids are isolated from the organism in the form of an oil, lipid or a free fatty acid.

13. The method as claimed in one of claims 8 to 12,
**wherein** the multiply unsaturated fatty acids produced in the process are C₁₈, C₂₀ or C₂₂ fatty acids with at least two double bonds in the molecule.

14. The method as claimed in one of claims 8 to 13,
**wherein** the organism is a transgenic plant.

15. The use of a nucleic acid sequence selected from the group consisting of
i) the nucleic acid sequence with the sequence shown in SEQ ID No.3, which codes for a polypeptide with an acyl-CoA lysophospholipid acyltransferase activity,
ii) nucleic acid sequences which as a result of the degenerate genetic code can be derived from the coding sequence contained in SEQ ID No.3, and
iii) derivatives of the nucleic acid sequence shown in SEQ ID No.3 which codes for a polypeptide with the amino acid sequence shown in SEQ ID No.4 and show at least 70% identity with SEQ ID No.4 at the amino acid level and have an acyl-CoA lysophospholipid acyltransferase activity,
for the production of an oil, lipid or a fatty acid composition, wherein the oil, lipid or fatty acid composition comprises multiply unsaturated fatty acids and was isolated from a transgenic plant with this nucleic acid sequence.

## Revendications

1. Séquence d'acide nucléique isolée, qui code pour un polypeptide ayant une activité d'acyl-CoA:lysophospholipide-acyltransférase, l'acyl-CoA:lysophospholipide-acyltransférase codée par la séquence d'acide nucléique convertissant d'une manière spécifique des acides gras en C₁₆, C₁₈, C₂₀ ou C₂₂ ayant au moins une double liaison dans la molécule d'acide gras, et la séquence d'acide nucléique étant choisie dans le groupe consistant en :
a) une séquence d'acide nucléique ayant la séquence représentée dans SEQ ID NO:3,
b) les séquences d'acides nucléiques qui, en tant que résultat du code génétique dégénéré, peuvent dériver de la séquence codante contenue dans SEQ ID NO:3, et
c) les dérivés de la séquence d'acide nucléique représentée dans SEQ ID NO:3, qui code pour un polypeptide ayant la séquence d'acides aminés représentée dans SEQ ID NO: 4, et qui présentent une identité d'au moins 70 % au niveau des acides aminés avec SEQ ID NO:4 et présentent une activité d'acyl-CoA:lysophospholipide-acyltransférase.

2. Séquence d'acides aminés, qui est codée par une séquence d'acide nucléique isolée selon la revendication 1.

3. Construction génique contenant un acide nucléique isolé selon la revendication 1, l'acide nucléique étant fonctionnellement lié à un ou plusieurs signaux de régulation.

4. Construction génique selon la revendication 3, **caractérisée en ce que** la construction d'acide nucléique contient des gènes de biosynthèse supplémentaires du métabolisme des acides gras ou des lipides, choisis dans le groupe consistant en la ou les acyl-CoA-déhydrogénase(s), la ou les acyl-ACP[= protéine porteuse d'acyle]désaturase(s), la ou les acyl-ACP-thioestérase(s), la ou les acide gras-acyl-transférase(s), la ou les acide gras-synthase(s), la ou les acide gras-hydroxylase(s), la ou les acétyl-coenzyme A-carboxylase(s), les acyl-coenzyme A-oxydase(s), la ou les acide gras-désaturase(s), les acide gras-acétylénases, les lipoxygénases, les triacylglycérol-lipases, les allénoxyde-synthases, les hydroperoxydes-lyases ou la ou les acide gras-élongase(s).

5. Construction génique selon la revendication 3 ou 4, **caractérisée en ce que** la construction d'acide nucléique contient des gènes de biosynthèse supplémentaires du métabolisme des acides gras ou des lipides, choisis dans le groupe des gènes de la Δ-4-désaturase, de la Δ-5-désaturase, de la Δ-6-désaturase, de la Δ-8-désaturase, de la Δ-9-désaturase, de la Δ-12-désaturase, de la Δ-5-élongase, de la Δ-6-élongase ou de la Δ-9-élongase.

6. Vecteur contenant un acide nucléique selon la revendication 1 ou une construction génique selon l'une des revendications 3 à 5.

7. Plante transgénique, la plante étant transgénique pour l'acide nucléique selon la revendication 1, la construction génique selon l'une des revendications 3 à 5 ou un vecteur selon la revendication 6.

8. Procédé de fabrication d'acides gras polyinsaturés dans un organisme, **caractérisé en ce que** le procédé comprend les étapes suivantes :
a) insertion d'au moins une séquence d'acide nucléique dans l'organisme, choisi dans le groupe consistant en
i) la séquence d'acide nucléique ayant la séquence représentée dans SEQ ID NO:3, qui code pour un polypeptide ayant une activité d'acyl-CoA-lysophospholipide-acyltransférase ;
ii) les séquences d'acides nucléiques qui, en tant que résultat du code génétique dégénéré, peuvent dériver de la séquence codante contenue dans SEQ ID NO:3, et
iii) les dérivés de la séquence d'acide nucléique représentée dans SEQ ID NO:3, qui code pour un polypeptide ayant la séquence d'acides aminés représentée dans SEQ ID NO: 4, et qui présentent une identité d'au moins 70 % au niveau des acides aminés avec SEQ ID NO: 4et présentent une activité d'acyl-CoA:lysophospholipide-acyltransférase ; et
b) culture et récolte de l'organisme.

9. Procédé selon la revendication 8, **caractérisé en ce qu'**en plus des séquences d'acides nucléiques mentionnées dans l'étape a), des séquences d'acides nucléiques supplémentaires sont insérées dans l'organisme, qui codent pour des polypeptides du métabolisme des acides gras ou des lipides, choisis dans le groupe consistant en la ou les acyl-CoA-déhydrogénase(s), la ou les acyl-ACP[= protéine porteuse d'acyle]désaturase(s), la ou les acyl-ACP-thioestérase(s), la ou les acide gras-acyl-transférase(s), la ou les acide gras-synthase(s), la ou les acide gras-hydroxylase(s), la ou les acétyl-coenzyme A-carboxylase(s), les acyl-coenzyme A-oxydase(s), la ou les acide gras-désaturase(s), les acide gras-acétylénases, les lipoxygénases, les triacylglycérol-lipases, les allénoxyde-synthases, les hydroperoxydes-lyases ou la ou les acide gras-élongase(s).

10. Procédé selon l'une des revendications 8 ou 9, **caractérisé en ce que**, en plus des séquences d'acides nucléiques mentionnées dans l'étape a) de la revendication 8, et éventuellement des séquences d'acides nucléiques mentionnées dans la revendication 9, des séquences d'acides nucléiques supplémentaires sont introduites dans l'organisme, qui codent pour des polypeptides choisis dans le groupe consistant en les Δ-4-désaturases, les Δ-5-désaturases, les Δ-6-désaturases, les Δ-8-désaturases, les Δ-9-désaturases, les Δ-12-désaturases, les Δ-5-élongases, les Δ-6-élongases ou les Δ-9-élongases.

11. Procédé selon l'une des revendications 8 à 10, **caractérisé en ce qu'**on utilise en tant que substrat des acyl-CoA-lysophospholipide-acyltransférases des acides gras en C₁₆, en C₁₈, en C₂₀ ou en C₂₂.

12. Procédé selon l'une des revendications 8 à 11, **caractérisé en ce que** les acides gras insaturés sont isolés de l'organisme sous forme d'une huile, d'un lipide ou d'un acide gras libre.

13. Procédé selon l'une des revendications 8 à 12, **caractérisé en ce que** les acides gras polyinsaturés fabriqués par le procédé sont des acides gras en C₁₈, en C₂₀ ou en C₂₂ ayant au moins deux doubles liaisons dans leur molécule.

14. Procédé selon l'une des revendications 8 à 13, **caractérisé en ce que** l'organisme est une plante transgénique.

15. Utilisation d'une séquence d'acide nucléique choisie dans le groupe consistant en :
i) la séquence d'acide nucléique ayant la séquence représentée dans SEQ ID NO:3, qui code pour un polypeptide ayant une activité d'acyl-CoA-lysophospholipide-acyltransférase ;
ii) les séquences d'acides nucléiques qui, en tant que résultat du code génétique dégénéré, peuvent dériver de la séquence codante contenue dans SEQ ID NO:3, et
iii) les dérivés de la séquence d'acide nucléique représentée dans SEQ ID NO:3, qui code pour un polypeptide ayant la séquence d'acides aminés représentée dans SEQ ID NO:4, et qui présentent une identité d'au moins 70 % au niveau des acides aminés avec SEQ ID NO: 4 et présentent une activité d'acyl-CoA:lysophospholipide-acyltransférase,
pour la fabrication d'une huile, d'un lipide ou d'une composition d'acides gras, l'huile, le lipide ou la composition d'acides gras comprenant des acides gras polyinsaturés et ayant été isolée d'une plante transgénique ayant cette séquence d'acide nucléique.
